(19) Europäisches Patentamt
European Patent Office
Office européen des brevets

(11) EP 3 473 680 A1

(12) **EUROPEAN PATENT APPLICATION**
published in accordance with Art. 153(4) EPC

(43) Date of publication:
**24.04.2019 Bulletin 2019/17**

(21) Application number: **17813421.9**

(22) Date of filing: **16.06.2017**

(51) Int Cl.:
***C09B 61/00*** (2006.01) ***A23K 10/30*** (2016.01)
***A23K 20/121*** (2016.01) ***A23K 20/179*** (2016.01)
***A23L 5/41*** (2016.01) ***A61K 8/49*** (2006.01)
***A61K 8/97*** (2017.01) ***A61Q 1/02*** (2006.01)

(86) International application number:
**PCT/JP2017/022274**

(87) International publication number:
**WO 2017/217527 (21.12.2017 Gazette 2017/51)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **17.06.2016 JP 2016121254**
**27.04.2017 JP 2017088924**

(71) Applicant: **San-Ei Gen F.F.I., INC.**
**Toyonaka-shi, Osaka 561-8588 (JP)**

(72) Inventors:
- **IZUMIDA Keiko**
  **Toyonaka-shi**
  **Osaka 561-8588 (JP)**
- **HAMASAKI Koji**
  **Toyonaka-shi**
  **Osaka 561-8588 (JP)**
- **YOKOYAMA Takamasa**
  **Toyonaka-shi**
  **Osaka 561-8588 (JP)**

(74) Representative: **Vossius & Partner**
**Patentanwälte Rechtsanwälte mbB**
**Siebertstrasse 3**
**81675 München (DE)**

# (54) RED DYE COMPOSITION DERIVED FROM IRIDOID COMPOUND, AND METHOD FOR PRODUCING SAME

(57) An object of the present invention is to provide a technique related to the production of a red colorant composition derived from iridoid compounds, and more particularly, a technique for producing an acid-resistant red colorant composition derived from iridoid compounds that is preferable from the viewpoint of safety of the colorant composition produced and its manufacturing cost.

The present invention provides a method for producing a red colorant composition derived from iridoid compounds, including a step of performing red color development that involves reaction of iridoid aglycones having a carboxyl group at position 4 of an iridoid skeleton with amino group-containing compounds, said red color development being performed in a solution in which ascorbic acid, similar compounds thereof, or two or more compounds selected from the group consisting of said ascorbic acid and said similar compounds are contained at a molar ratio of 5 or more with respect to all iridoid compounds in the solution, whereby red colorant compounds having acid resistance are generated.

*FIG. 1*

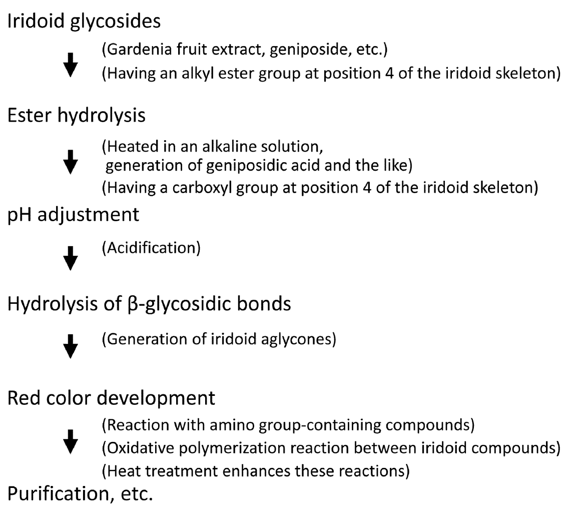


EP 3 473 680 A1

## Description

Technical Field

The present invention relates to a red colorant composition derived from iridoid compounds and the method for producing same.

Background Art

Gardenia red color is a pigment exhibiting red color made from gardenia fruit extract and is a natural colorant having relatively high stability against light and heat. Gardenia red color is a colorant composition obtained by reacting the aglycones of iridoid compounds contained in gardenia fruit extract with amino group-containing compounds. In the development of gardenia red color, a part of the processes in the technique for producing blue colorant compounds having structures derived from iridoid compounds has been improved to obtain colorants exhibiting red color (Patent Document 1).

Reflecting consumer awareness of safety in recent years, the demand for gardenia red color, which is derived from plants, is expanding in many fields, including foods, beverages, cosmetic products, quasi-drugs, and medicines, as a substitute for insect-derived cochineal dye, other synthetic colorings, and the like. Especially, gardenia red color is utilized as an excellent colorant material that can be used even in the field of food and beverage.

However, gardenia red color not only has advantages, but also inherent disadvantages as follows that impose limitations on the purpose of use of the colorant and the fields in which the colorant is put into practical use. Specifically, since gardenia red color forms aggregates under acidic conditions of pH 3.5 or less, there is a fundamental problem in that gardenia red color cannot be used for coloring acidic compositions such as acidic beverages. Therefore, it is expected to overcome the problems related to the dissolution stability against aggregation and precipitation under acidic conditions and develop a technique for producing gardenia red color having acid resistance, so that gardenia red color can be used for broader purposes of use and can be put into practical use in wider fields.

There are prior art documents related to the acid resistance of gardenia red color developed under such circumstances. For example, in the prior art technique for obtaining acid resistance in the production process of gardenia red color disclosed in Patent Document 2, sulfite ions are generated at the time of red color development to confer acid resistance to gardenia red colorant. However, sulfite ions in sodium metabisulfite, potassium metabisulfite and the like, which are the technical feature of this technique, are pointed out to have various impacts when used in foods and beverages. Therefore, even if the sulfite ions are removed in the subsequent processes, this technique poses concerns for today's consumers, who are highly safety conscious.

In the prior art technique for achieving acid resistance of gardenia red color disclosed in Patent Document 3, substances containing taurine are added at the time of red color development. However, the price per unit of the taurine-containing substances, which are used to be added in the technique disclosed in this document, is incomparably higher than that of ordinary organic acids, and thus, this technique has problems from the viewpoint of the production cost of the colorant. Moreover, the acid resistance of the gardenia red colorant composition produced by the method disclosed in this document is not sufficient. For example, in the gardenia red colorant composition produced by a method disclosed in this document, aggregation and precipitation occur at a pH of less than 3 (*see* Examples 7 and 8 in the Examples section below). That is, it is admitted that the function of taurine to impart acid resistance as described in this prior art document is insufficient as a technique to be applied to acidic foods, beverages, and the like.

Related prior art techniques in this field are found in the following documents. For example, prior art techniques aiming at improving the color of gardenia red color include a technique in which red color development is performed under the presence of organic acids having a molar ratio of 2 or more and specific amino acids, such as arginine, having a molar ratio of 0.7 or more with respect to the iridoid compounds (Patent Document 4), and a technique in which red color development is performed under the presence of protein hydrolysates fulfilling specific standards (Patent Document 5). However, the documents disclosing these techniques neither refer to technical problems related to aggregation and the like under acidic conditions nor have any disclosure or suggestion that may lead to the development of a method for conferring acid resistance to gardenia red color.

Citation List

Patent Documents

Patent Document 1: Japanese Examined Patent Publication No. S55-5778

Patent Document 2: Japanese Patent No. 5753373
Patent Document 3: Japanese Patent No. 4605824
Patent Document 4: Japanese Patent No. 2802451
Patent Document 5: Japanese Unexamined Patent Application Publication No. 2011-217728

## Summary of Invention

### Technical Problem

The present invention has been achieved in light of the aforementioned circumstances of the conventional techniques, and it is an object thereof to provide a technique related to the production of a red colorant composition derived from iridoid compounds, and more particularly, a technique for producing an acid-resistant red colorant composition derived from iridoid compounds that is preferable from the viewpoint of safety of the colorant composition produced and its manufacturing cost.

### Solution to Problem

As a result of intensive study conducted to solve the aforementioned problems, the present inventors have developed a technique of adding an increased amount of ascorbic acid and the like during the red color development process which involves the reaction between the iridoid aglycones having a carboxyl group at position 4 of the iridoid skeleton and the amino group-containing compounds. Performing red color development by a method based on this finding, the inventors have found that a red colorant composition derived from iridoid compounds excellent in acid resistance can be obtained. The inventors have found that this red colorant composition has outstanding acid resistance, and especially, has remarkably improved dissolution stability under acidic conditions of less than pH 3, which has been difficult to achieve in prior art techniques.
Ascorbic acid used in the production process mentioned above is a substance widely used as vitamin C and antioxidants for foods, beverages, and the like, and it is a compound whose safety for human body is ensured. In this regard, the technique according to the present invention is considered to have overcome the concerns over the safety of the conventional colorant composition obtained by generating sulfite ions such as sodium metabisulfite and potassium metabisulfite as disclosed in Patent Document 2.
Moreover, since ascorbic acid is an inexpensive material widely used in commercially produced foods, beverages, and the like, the technique according to the present disclosure has an advantage from the viewpoint of the production cost, compared with the prior art technique utilizing compounds containing taurine, which are expensive material, disclosed in

Patent Document 3.

Moreover, as the inventors performed comparative experiments to assess the acid resistance of the red colorant composition obtained by the aforementioned process and the red colorant composition disclosed in Patent Document 3 obtained by adding a composition containing taurine, a significant difference was observed in the function of imparting acid resistance and dissolution stability under the acidic conditions of less than pH 3. It has been confirmed that the red colorant composition according to the present invention has excellent acid resistance compared with the red colorant composition of Patent Document 3 (*see* Examples 7, 8 and 15 in the Examples section below).
Section 0036 of Patent Document 2 shows the examples of organic acids that can be optionally added in the production process of the iridoid compounds, and ascorbic acid is referred to as one such example. Also, several organic acids are referred to as the substance to be added in claim 1 of

Patent Document 4.

However, these parts of the documents also refer to "citric acid", with which it is impossible to achieve the effects of the present invention, and therefore teach away from the present invention. Moreover, in the Examples section of these documents, there is no single example in which ascorbic acid is added, and it is impossible to conclude that these documents disclose methods that enable to generate the colorant composition having acid resistance according to the present invention.
Moreover, Patent Documents 2 and 4 merely disclose the invention developed from technical ideas different from those of the present invention, and they neither teach nor suggest the addition of increased amount of ascorbic acid and the like and its effects on the improvement of the acid resistance of iridoid compounds.
Based on these findings, the present inventors have accomplished the present invention. The present invention

specifically relates to aspects of the invention described below.

[1] A method for producing a red colorant composition derived from iridoid compounds, including the following step:

(1) performing red color development that involves reaction of iridoid aglycones having a carboxyl group at position 4 of an iridoid skeleton with amino group-containing compounds,
said red color development being performed in a solution in which ascorbic acid, similar compounds thereof, or two or more compounds selected from the group consisting of said ascorbic acid and said similar compounds are contained at a molar ratio of 5 or more with respect to all iridoid compounds in the solution,
whereby red colorant compounds having acid resistance are generated.

[2] The method according to aspect 1, wherein the red color development in step (1) is performed in a solution in which ascorbic acid is contained at a molar ratio of more than 6 with respect to all iridoid compounds in the solution.

[3] The method according to aspect 1 or 2, wherein said red colorant composition derived from iridoid compounds is a gardenia red colorant composition.

[4] The method according to any one of aspects 1 to 3, wherein, in step (1), production of iridoid aglycones is carried out by hydrolysis of β-glycosidic bonds in iridoid glycosides prior to and/or concurrently with the red color development.

[5] A method for producing a red colorant composition derived from iridoid compounds, including the following step:
(1') performing red color development that involves reaction of aglycones of geniposide ester hydrolysate and/or aglycones of geniposidic acid having a carboxyl group at position 4 of an iridoid skeleton with amino group-containing compounds,
said red color development being performed under an acidic condition in a solution in which ascorbic acid is contained at a molar ratio of more than 6 with respect to all iridoid compounds in the solution,
whereby red colorant compounds having acid resistance are generated,
wherein, in step (1'), production of iridoid aglycones is carried out by hydrolysis of β-glycosidic bonds in iridoid glycosides prior to and/or concurrently with the red color development, and
said red colorant composition derived from iridoid compounds is a gardenia red colorant composition.

[6] A method for imparting acid resistance to a produced red colorant composition derived from iridoid compounds, wherein step (1) recited in any one of aspects 1 to 4 or step (1') recited in aspect 5 is performed in a process for producing the red colorant composition derived from iridoid compounds.

[7] A red colorant composition derived from iridoid compounds, obtained by the method according to any one of aspects 1 to 5.

[8] A red colorant composition derived from iridoid compounds having a feature as follows:
an $A_{\lambda max} / A_{600\ nm}$ value of an aqueous solution is 4.9 or more, when the aqueous solution contains the red colorant composition derived from iridoid compounds at a color value ($E^{10\%}_{1\ cm}$) of 0.05 and is adjusted to have a pH being 5.0 using McIlvaine buffer.

[9] The red colorant composition derived from iridoid compounds according to aspect 8, having a feature related to acid resistance as follows:
a color residual rate of a filtrate of an aqueous solution is 35% or more, when the aqueous solution contains the red colorant composition derived from iridoid compounds at a content of 0.05 mass% when calculated in terms of color value ($E^{10\%}_{1\ cm}$) 50.5, is adjusted to have a pH being 2.2 using McIlvaine buffer, and is filtered through a 0.45-μm filter to obtain said filtrate.

[10] A pigment preparation containing the red colorant composition derived from iridoid compounds according to any one of aspects 7 to 9.

[11] A method for producing a pigment preparation, a food, a beverage, a cosmetic product, a medicine, a quasi-drug, a personal hygiene product, or feed, the method including a step in which the red colorant composition derived from iridoid compounds obtained by the method according to any one of aspects 1 to 5 is contained.

[12] A method for producing a food, a beverage, a cosmetic product, a medicine, a quasi-drug, a personal hygiene

product, or feed, the method including a coloring step in which the red colorant composition derived from iridoid compounds according to any one of aspects 7 to 9 or the pigment preparation according to aspect 10 is used.

[13] The method for producing the food, the beverage, the cosmetic product, the medicine, the quasi-drug, the personal hygiene product, or the feed according to aspect 12, at least a part or the entire part of said method including a step/steps performed at a pH of less than 3.

[14] A food, a beverage, a cosmetic product, a medicine, a quasi-drug, a personal hygiene product, or feed, containing the red colorant composition derived from iridoid compounds according to any one of aspects 7 to 9 or the pigment preparation according to aspect 10.

[15] The food, the beverage, the cosmetic product, the medicine, the quasi-drug, the personal hygiene product, or the feed according to aspect 14, wherein at least a part or the entire part of the food, the beverage, the cosmetic product, the medicine, the quasi-drug, the personal hygiene product, or the feed has a pH of less than 3.

Advantageous Effects of the Invention

The present invention provides a technique related to the production of a red colorant composition derived from iridoid compounds, and more particularly, a technique for producing an acid-resistant red colorant composition derived from iridoid compounds that is preferable from the viewpoint of safety of the colorant composition produced and its manufacturing cost.

The present invention enables, for example, to provide gardenia red color that is excellent in dissolution stability under acidic conditions and can be utilized for a wide range of purposes and products for which conventional gardenia red color could hardly be used.

Brief Description of Drawings

FIG. 1 is a flowchart showing the outline of the main steps in the production of the red colorant composition derived from iridoid compounds in the examples in this specification.
FIG. 2 shows the chroma values calculated in the evaluation of color characteristics of the gardenia red colorant compositions produced in Example 1.
FIG. 3 shows the plots on the orthogonal coordinate system consisting of the *a* axis and the *b* axis of the Hunter Lab colorimetric system in the evaluation of color characteristics of the gardenia red colorant compositions produced in Example 1.
FIG. 4 is a photographic image of the test bottles containing the gardenia red colorant compositions produced in Example 1.
FIG. 5 shows the chroma values calculated in the evaluation of color characteristics of the gardenia red colorant compositions produced in Example 2.
FIG. 6 shows the plots on the orthogonal coordinate system consisting of the *a* axis and the *b* axis of the Hunter Lab colorimetric system in the evaluation of color characteristics of the gardenia red colorant compositions produced in Example 2.
FIG. 7 is a photographic image of the test bottles containing the gardenia red colorant compositions produced in Example 2.
FIG. 8 shows the chroma values calculated in the evaluation of color characteristics of the milk beverages produced in Example 4.
FIG. 9 shows the plots on the orthogonal coordinate system consisting of the *a* axis and the *b* axis of the Hunter Lab colorimetric system in the evaluation of color characteristics of the milk beverages produced in Example 4.
FIG. 10 is a photographic image of the test bottles containing the milk beverages produced in Example 4.
FIG. 11 is top-view photographic images of the transparent retort pouches filled with the processed fish meat products produced in Example 5.
FIG. 12 shows the color residual rates of the gardenia red colors immediately after the preparation of test liquids of different pH levels from weak acidity to acidity in the acid resistance test in Example 7.
FIG. 13 shows the color residual rates of the gardenia red colors after the test liquids of different pH levels in the acidic range were stored under low temperature in the acid resistance test in Example 8.
FIG. 14 shows the spectral waveforms obtained in the color measurement of the transmitted light in Example 9.
FIG. 15 shows the chroma values calculated in the evaluation of color characteristics of the puddings produced in

Example 10.

FIG. 16 shows the plots on the orthogonal coordinate system consisting of the *a* axis and the *b* axis of the Hunter Lab colorimetric system in the evaluation of color characteristics of the puddings produced in Example 10.

FIG. 17 shows photographic images of the pudding samples produced in Example 10.

FIG. 18 shows the chroma values calculated in the evaluation of color characteristics of the bread produced in Example 11.

FIG. 19 shows the plots on the orthogonal coordinate system consisting of the *a* axis and the *b* axis of the Hunter Lab colorimetric system in the evaluation of color characteristics of the bread produced in Example 11.

FIG. 20 shows photographic images of the bread samples produced in Example 11.

FIG. 21 shows photographic images of the chewing gum samples produced in Example 12.

FIG. 22 shows photographic images of the pickled vegetable samples of Example 13 immediately after production.

FIG. 23 shows photographic images of the pickled vegetable samples of Example 13 showing the colors before and after light irradiation.

FIG. 24 shows the color residual rates of the gardenia red colors immediately after the preparation of test liquids of pH 2.2 in the acid resistance test in Example 15.

FIG. 25 shows the color residual rates of the gardenia red colors after the test liquids of pH 2.2 were stored under low temperature in the acid resistance test in Example 15.

## Description of Embodiments

The present invention relates to red colorant compositions derived from iridoid compounds having acid resistance, production methods thereof, and application thereof. Hereinafter, embodiments of the present invention will be described in detail.

The present application claims priority to Japanese Patent Application Serial Nos. 2016-121254 and 2017-088924 filed with the Japan Patent Office by the applicant of the present invention, the entire contents of which are incorporated herein by reference.

Embodiments including elements other than those described below are not excluded from the technical scope of the present invention, as long as they do not practically inhibit the effects brought about by the technical features of the present invention. Also, the technical scope of the present invention is not limited to a mode in which all of the following features are included.

### 1. Production of Red Colorant Composition Derived from Iridoid Compounds

The red colorant composition derived from iridoid compounds according to the present invention can be prepared and produced by the steps as described below. Inclusion of steps other than those described below is not excluded from the production method according to the present invention as long as they do not practically inhibit the effects brought about by the technical features of the present invention. Also, the technical scope of the present invention is not limited to a mode including all the steps described below, as long as its essential steps are included.

The method for producing a red colorant composition derived from iridoid compounds according to the present invention relates to the method including the following step: (1) performing red color development that involves reaction of iridoid aglycones having a carboxyl group at position 4 of the iridoid skeleton with amino group-containing compounds; said red color development being performed in a solution in which ascorbic acid, similar compounds thereof, or two or more compounds selected from the group consisting of said ascorbic acid and said similar compounds are contained at a molar ratio of 5 or more with respect to all iridoid compounds in the solution; whereby red colorant compounds having acid resistance are generated.

### [Iridoid Compound]

The term "iridoid compound" as used herein refers to a compound having an iridoid skeleton. The "compound having an iridoid skeleton" refers to a compound having a structure composed of a five-membered ring fused to an oxygen-containing six-membered heterocyclic ring, and it specifically refers to a compound having the basic skeleton structure represented by the Structural Formula I shown below. In Structural Formula I, the atoms constituting the iridoid skeleton are specified with numbers.

The term iridoid compound as used herein not only refers to iridoid glycosides but also iridoid aglycones, which are the aglycones of iridoid glycosides. The iridoid compounds in this specification also include those in a dissolved ionic state.

Here, $R_1$, $R_2$, $R_3$, $R_4$, and $R_5$ in the structural formula can represent any functional groups, and $R_1$ is a carboxyl group in the reactant for red color development according to the present invention. When $R_1$ is a lower alkyl group such

as a methyl ester group, it can be converted to a carboxyl group by ester hydrolysis, so that the compound can be used as the reactant for red color development.

Iridoid compounds contained in plants, fruits, and the like often have a glycoside structure, which is usually inactive and stable. In this case, $R_2$ is a sugar molecule linked via a β-glycosidic bond. The aglycone which serves as the reactant for red color development according to the present invention has a structure in which a hydroxyl group (-OH) is attached to position 1 of the iridoid skeleton. Specifically, this aglycone has a structure expressed by the Structural Formula II shown below.

$R_3$, $R_4$, and $R_5$ are hydrogen atoms (-H) in many compounds. Yet, compounds in which $R_3$, $R_4$, or $R_5$ is a functional group containing a hydroxyl group, an alkyl group, or a phenyl compound are also reported. Here, the structures represented by Structural Formulae III and IV are also encompassed in which a double bond is formed between carbon atoms at positions 6 and 7 of the iridoid skeleton and/or carbon atoms at positions 7 and 8 of the iridoid skeleton. Moreover, the carbon atoms at positions 6 and 7 of the iridoid skeleton and/or the carbon atoms at positions 7 and 8 of the iridoid skeleton can be linked via a common atom or molecular structure. Examples thereof are structures represented by Structural Formulae V and VI, in which these carbon atoms are liked via an oxygen atom.

Furthermore, the iridoid compounds of the present invention may also include various types of functional groups as long as the stability and color characteristics of the red colorant composition according to the present invention are not practically impaired.

Structural Formula I

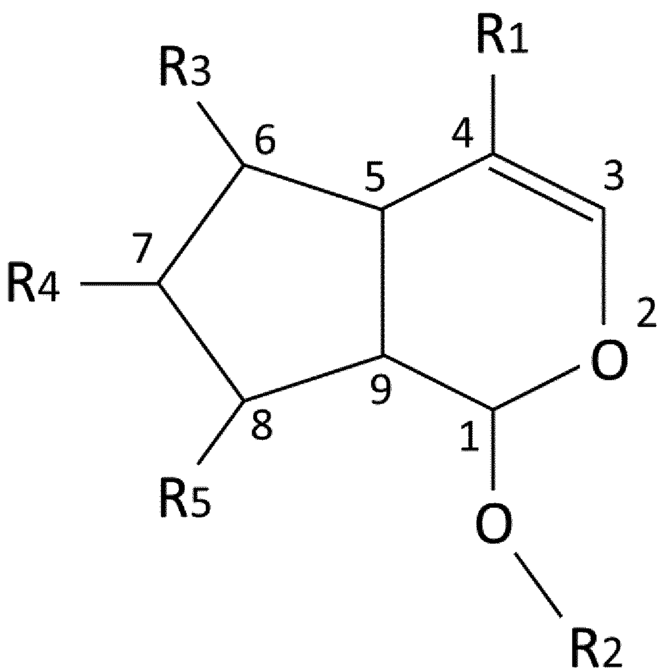

···(I)

Structural Formula II

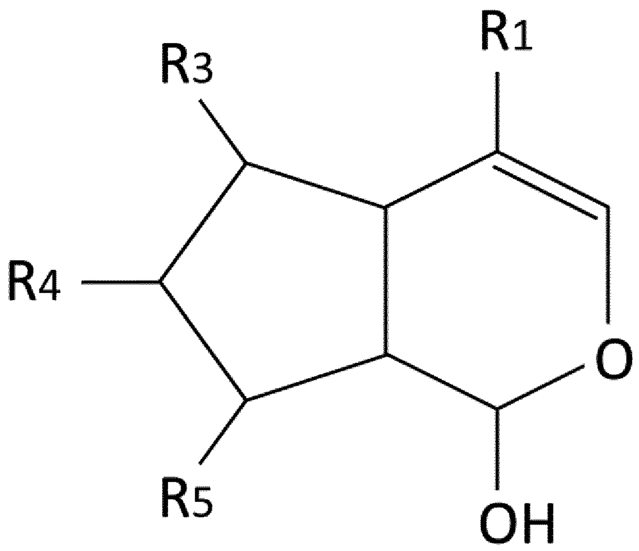

・・・(II)

Structural Formula III

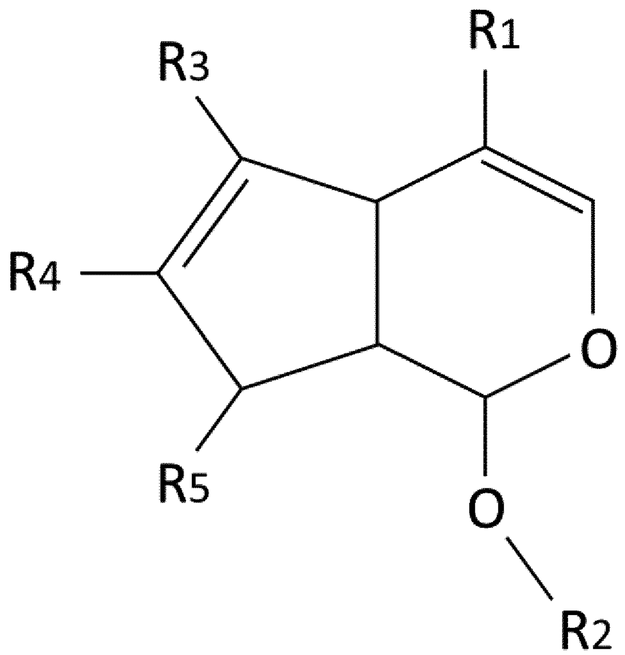

・・・(III)

Structural Formula IV

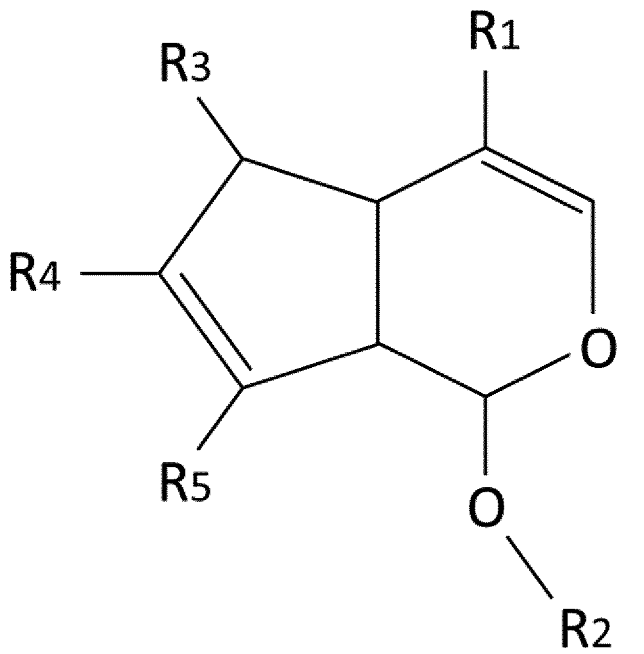

···(IV)

Structural Formula V

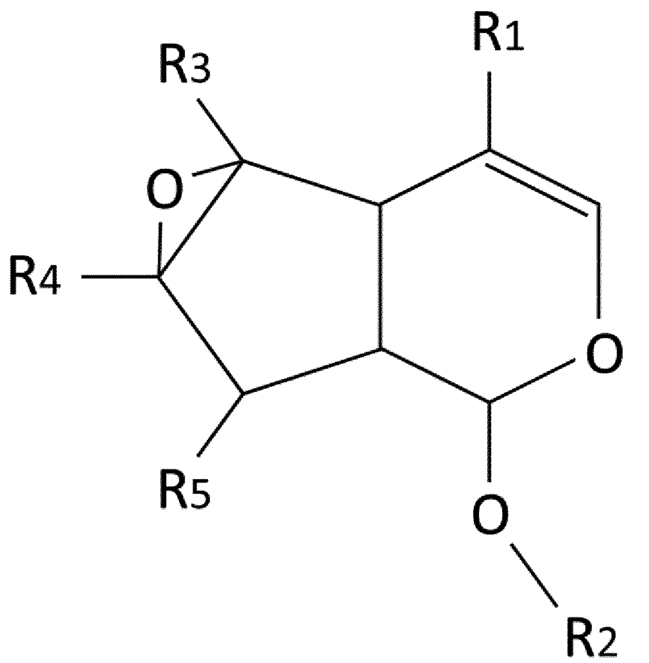

···(V)

Structural Formula VI

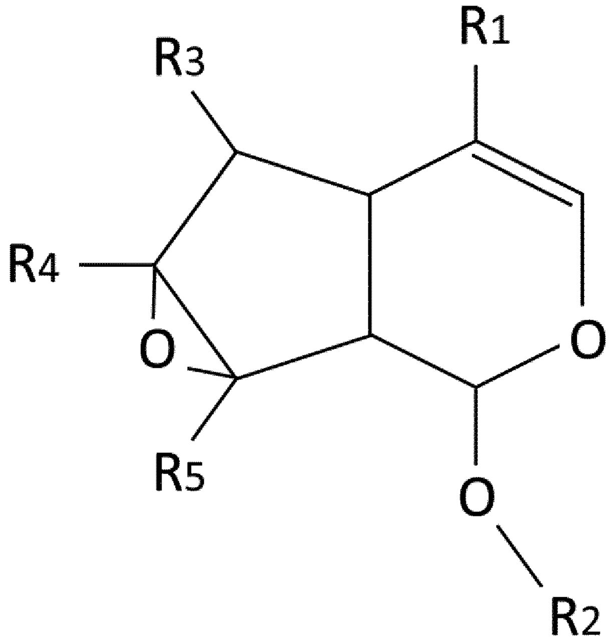

...(VI)

[Reactant]

In the method for producing the red colorant composition according to the present invention, red color development that involves the reaction of iridoid aglycones having a carboxyl group at position 4 of the iridoid skeleton with amino group-containing compounds is performed. An "iridoid aglycone having a carboxyl group at position 4 of the iridoid skeleton" as used herein refers to the aglycone obtained from an iridoid glycoside having a carboxyl group at position 4 of the iridoid skeleton.

An example of the "iridoid aglycone having a carboxyl group at position 4 of an iridoid skeleton" is genipic acid. Genipic acid is an aglycone of geniposidic acid, which is abundantly contained in gardenia fruits. Genipic acid is also a compound generated by hydrolyzing the methyl ester group at position 4 of the iridoid skeleton of genipin to a carboxyl group.

Plant Raw Material

In the production method of the red colorant composition according to the present invention, it is preferable to use as the raw material the plants containing iridoid compounds. Examples of the materials used in the present invention are gardenia, huito (*Genipa americana*), yellow catalpa, and *Eucommia ulmoides.* Although any part of the plant body, including the aboveground and underground parts, can be used as the raw material, fruits are preferable. From the viewpoint of producing a colorant composition having excellent red color characteristics, gardenia fruits are preferable.

The "gardenia fruit" as used herein can refer to the plants belonging to the genus *Gardenia* of the family Rubiaceae, and more specifically, it refers to the fruits of the species belonging to the genus *Gardenia.* Examples of the plants belonging to the genus *Gardenia* are *Gardenia jasminoides* and *Gardenia augusta.* Their closely related species and hybrids with such closely related species are also encompassed. A preferable example of the plants belonging to the genus *Gardenia* is *Gardenia jasminoides.*

From the viewpoint of producing a colorant composition having excellent red color characteristics, the fruit of *Gardenia jasminoides* is preferably used.

When a plant raw material is used in the method for producing red colorant composition according to the present invention, a plant extract containing iridoid compounds can be used. Any known methods for extracting iridoid compounds from plant bodies can be used without limitation, and it is preferable to employ a method by which a higher yield of iridoid compounds can be obtained. In an example of the method for obtaining an extract from plant bodies, the raw material is crushed, pulverized, ground, mashed, powdered, or dried, and is subjected to extraction with water, aqueous alcohol, or alcohol. Then, such treatments as filtration and purification are performed, and the extract obtained can be used as the extract of plant raw material.

When the plant body is directly used without being subjected to the extraction process, it is also possible to

use the plant juice, fruit juice, puree, dried products of said juice or puree, or the like as the material.

Hydrolysis Treatment on Precursors

In the present invention, red color development is performed by using as the reactant the iridoid aglycone having a carboxyl group at position 4 of the iridoid skeleton.

The iridoid aglycone having a carboxyl group at position 4 of the iridoid skeleton can be directly used as the material in the present invention. Also, i) when an iridoid compound having an alkyl ester group at position 4 of the iridoid skeleton is used as the precursor, it can be used by hydrolyzing the alkyl ester group at position 4 of the iridoid skeleton to a carboxyl group by ester hydrolysis. Here, in a preferable mode, the alkyl ester group at position 4 is a methyl ester group. Moreover, ii) when an iridoid glycoside is used as the precursor, it can be used by hydrolyzing the β-glycosidic bond and generating an aglycone.

When iridoid compounds having an alkyl ester group at position 4 of the iridoid skeleton, which are the precursors, are used as the material in the method for producing the red colorant composition according to the present invention, i) it is preferable to perform a reaction in which the alkyl ester group at position 4 of the iridoid skeleton is hydrolyzed to a carboxyl group by ester hydrolysis.

Any procedures involving the hydrolysis of an alkyl ester group at position 4 of the iridoid skeleton to a carboxyl group can be employed for the ester hydrolysis treatment in the present invention. For example, treatment with an alkaline solution, treatment with ion exchange resin, or treatment with enzymes exhibiting esterase activity can be employed. In one example, ester hydrolysis treatment can be performed by preparing an alkaline solution of about pH 10 to 13 containing sodium hydroxide or the like, and heating the iridoid compounds in the solution at around 40 to 70°C.

When iridoid glycosides, which are the precursors, are used as the material in the method for producing the red colorant composition according to the present invention, ii) it is preferable to perform a reaction in which iridoid aglycones are generated by hydrolyzing the β-glycosidic bond at position 1 of the iridoid skeleton.

Any methods involving the hydrolysis of the β-glycosidic bond at position 1 of the iridoid skeleton can be employed for the hydrolysis treatment of the β-glycosidic bond. For example, treatment with enzymes exhibiting β-glucosidase activity, treatment with microorganisms having β-glucosidase activity, or treatment with an acidic solution can be employed. In one example, an enzyme solution of about pH 3 to 6 exhibiting β-glucosidase activity is prepared, and as the iridoid glycosides are treated in this solution, aglycones can be generated. Here, any enzymes having β-glucosidase activity can be used without limitation, and an example thereof is cellulase. Examples of commercially available cellulase preparations are Cellulase T "Amano" and Cellulase A "Amano" manufactured by Amano Enzyme Inc.; Driselase KSM, Multifect A40, and Cellulase GC220 manufactured by Genencor Kyowa Co., Ltd.; GODO-TCL cellulase, GODO TCD-H cellulase, Vesselex, and GODO-ACD cellulase manufactured by Godo Shusei Co., Ltd.; Cellulase manufactured by Toyobo Co., Ltd.; Cellulizer and Cellulase XL-522 manufactured by Nagase ChemteX Corporation; Cellusoft and Denimax manufactured by Novozymes A/S; Cellulosin AC40, Cellulosin AL, and Cellulosin T2 manufactured by HBI Enzymes Inc.; Cellulase "Onozuka" 3S and Cellulase Y-NC manufactured by Yakult Pharmaceutical Industry Co., Ltd.; Sumizyme AC and Sumizyme C manufactured by Shinnihon Chemicals Corporation; and Enzylon CM, Enzylon MCH, and Biohit manufactured by Rakuto Kasei Industrial Co., Ltd.

In the treatment of precursors described in i) and ii) above, it is preferable to select the treatment method suitable for the compound or composition of the material used as the precursor.

For example, when iridoid glycosides having an alkyl ester group at position 4 of the iridoid skeleton are used as the precursors, it is required to perform both of the procedures described in i) and ii) above. Specific examples of such compounds are geniposide and gardenoside, which are iridoid glycosides having a methyl ester group at position 4 of the iridoid skeleton. Geniposide, which is abundantly contained in gardenia fruits, is preferable.

When iridoid glycosides having a carboxyl group at position 4 of the iridoid skeleton are used as the precursors, it is required to perform the procedure described in ii) above. Specific examples of such compounds are geniposidic acid and the like. Geniposidic acid, which is abundantly contained in gardenia fruits, is especially preferable. Geniposidic acid is a compound obtained by hydrolyzing the methyl ester group at position 4 of the iridoid skeleton of geniposide, which is abundantly contained in gardenia fruits, to a carboxyl group.

When iridoid aglycone having an alkyl ester group at position 4 of the iridoid skeleton is used as the precursor, it is required to perform the procedure described in i) above. A specific example of such compound is genipin, which is an iridoid aglycone having a methyl ester group at position 4 of the iridoid skeleton.

When plant bodies or extracts are used in the production method according to the present invention, it is preferable to perform both of the procedures described in i) and ii) above to obtain iridoid aglycones, which are the substrates for red color development, because the plant bodies or extracts have a high content of iridoid glycosides having a methyl ester group at position 4 of the iridoid skeleton, which are the precursors.

The following table shows the relationship between the structure of the iridoid compounds and the treatment procedures, with geniposide, geniposidic acid, and the like, which are abundantly contained in gardenia fruits, taken as

examples.

Table 1

| | Methyl ester group at position 4 | Carboxyl group at position 4 (Ester hydrolysate) |
|---|---|---|
| Iridoid glycoside | Geniposide, etc. (precursor) | Geniposidic acid, etc. (precursor) |
| Iridoid aglycone (obtained by hydrolysis of β-glycosidic bond) | Aglycone of geniposide, Genipin, etc. (precursor) | Aglycone of geniposidic acid, Genipic acid, etc. (reactant) |

[Red Color Development]

In the method for producing the red colorant composition according to the present invention, red color development that involves the reaction of amino group-containing compounds with iridoid aglycones having a carboxyl group at position 4 of the iridoid skeleton is performed.

The term "red color development" as used herein refers to the series of reactions that progress involving the reaction between the iridoid aglycones described above and amino group-containing compounds. Here, the term "reaction between the iridoid aglycones and amino group-containing compounds" refers to a complex reaction including the reaction in which the amino group-containing compounds bond to the iridoid compounds as an oxygen atom at position 2 of the iridoid skeleton is replaced by a nitrogen atom of the amino group-containing compound, and the reaction in which the oxidative polymerization between iridoid compounds occur (Japanese Examined Patent Publication No. S55-5778).

Solvent and the Like

Water is preferable as the solvent for performing the red color development according to the present invention. Purified water, distilled water, ultrapure water, and the like are especially preferable, but any water with purity suitable for manufacturing foods, beverages, colorant compositions, and the like can be used as the solvent. Aqueous solutions containing various salts, organic acids, pH adjusters, pH buffers, lower alcohols, or the like can also be used as long as the red color development is not practically inhibited.

Ascorbic Acid and the Like

The red color development in the present invention is performed in a condition in which ascorbic acid and/or compounds similar to ascorbic acid exist in the reaction solution. As the red color develops under the presence of ascorbic acid and/or similar compounds in the present invention, the structure of the red colorant compounds generated and/or the composition of the red colorant composition generated are changed, and the red colorant composition can exhibit acid resistance due to their compositional features. Especially, the dissolution stability at a pH of less than 3 remarkably improves.

Moreover, as the red color development is performed under the presence of ascorbic acid and/or similar compounds in the present invention, the red colorant composition obtained can exhibit not only acid resistance but also bright color.

The red color development according to the present invention is performed in a solution in which ascorbic acid, similar compounds thereof, or two or more compounds selected from the group consisting of said ascorbic acid and said similar compounds are contained.

The term "ascorbic acid" as used herein refers to L-ascorbic acid. It is regarded as a compound highly safe for the human body, since it is a biologically active substance that functions as vitamin C in the living body and is a compound widely used as an antioxidant for foods and beverages. Moreover, ascorbic acid is inexpensive as a material. It is highly water soluble and can be easily handled in the production processes such as the addition to the product. The ascorbic acid in this specification also include those in a dissolved ionic state.

Though it is most preferable to use ascorbic acid in the present invention, it is also possible to use similar compounds thereof.

The term "similar compounds" as used herein refers to the compounds which are similar to ascorbic acid and can exhibit functions of conferring acid resistance to the same or higher extent than ascorbic acid when used in the production processes according to the present disclosure. Examples of the compounds similar to ascorbic acid are the optical isomers and stereoisomers of L-ascorbic acid. An example of the optical isomers is D-xyloascorbic acid. An example of the stereoisomers is araboascorbic acid, and erythorbic acid, which is D-araboascorbic acid, is also included.

Moreover, compounds similar to ascorbic acid include its derivatives and the derivatives of its isomers. These derivatives include compounds obtained by replacing the functional group/groups or the like of ascorbic acid or its isomers while maintaining their basic skeleton structures, and any such compounds can be used if they can exhibit the function of imparting acid resistance to the same or higher extent than ascorbic acid when used in the production processes according to the present disclosure.

Further, compounds similar to ascorbic acid include compounds such as the salts of ascorbic acid and the salts of the compounds exemplified above. Compounds similar to ascorbic acid also include the compounds exemplified above in a dissolved ionic state.

The reaction solution for red color development is required to contain a large amount of ascorbic acid and/or similar compounds with respect to the content of iridoid compounds in the reaction solution. That is, the red color development according to the present invention needs to be performed in a solution containing ascorbic acid and the like at a concentration far beyond the normal level of usage.

As a specific example of the content of the ascorbic acid and/or similar compounds, the molar ratio of the ascorbic acid and/or similar compounds contained in the reaction solution with respect to all iridoid compounds contained in the reaction solution is preferably 5 or more. Especially, the molar ratio of the ascorbic acid and/or similar compounds contained in the reaction solution with respect to all iridoid compounds contained in the reaction solution is 6 or more, preferably more than 6, more preferably 6.5 or more, more preferably 7 or more, and even more preferably 8 or more, from the viewpoint of imparting acid resistance to the red colorant composition generated. In order to achieve especially excellent acid resistance, the molar ratio of the ascorbic acid and/or similar compounds contained in the reaction solution with respect to all iridoid compounds contained in the reaction solution is 6.5 or more, preferably 7 or more, and more preferably 8 or more. The aforementioned ranges of the molar ratio are preferable also from the viewpoint of developing a bright red color.

The upper limit of the molar ratio of the ascorbic acid and/or similar compounds contained in the reaction solution with respect to all iridoid compounds contained in the reaction solution is not particularly defined, but it can be, for example, 20 or less, preferably 15 or less, and more preferably 12 or less.

Reaction Conditions and the Like

The red color development according to the present invention is performed using as the reactants the iridoid aglycones having a carboxyl group at position 4 of the iridoid skeleton. The aglycones of geniposide ester hydrolysate (e.g. geniposidic acid) mentioned above, gardenoside ester hydrolysate, and the like can be used as the iridoid aglycones. The iridoid aglycones preferably contain the aglycones of geniposide ester hydrolysate and/or the aglycones of geniposidic acid, which have a carboxyl group at position 4 of the iridoid skeleton.

Any amount of iridoid compounds can be contained in the solution for the red color development according to the present invention as long as the red color can be developed. In a preferable embodiment, the total amount of iridoid compounds contained in said solution is, for example, 0.1 to 75 mass%, and preferably 0.5 to 50 mass% with respect to the total mass of the solution. In addition to the iridoid compounds, the solution can also contain other substances derived from the material to an extent that does not practically inhibit the red color development.

The red color development according to the present invention involves the reaction in which the amino group-containing compound binds to the iridoid compound as an oxygen atom at position 2 of the iridoid compound is replaced by a nitrogen atom of an amino group of the amino group-containing compound.

Amino acids, which are compounds having primary amino groups, and compositions containing amino acids can be used as the amino group-containing compounds suitable for this reaction. For example, protein hydrolysates or peptides can be used. Examples of protein hydrolysates are hydrolysates of various proteins such as wheat protein, soybean protein, milk protein, and collagen. Various amino acids such as glutamic acid, serine, arginine, lysine, aspartic acid, and glycine can also be used. In addition, sodium salts of these substances, such as monosodium glutamate, can be suitably used.

The content of amino group-containing compounds in the reaction solution is not particularly limited, and it is desirable that the molar ratio of the amino group-containing compounds added with respect to all iridoid compounds in the reaction solution is 0.7 or more, and preferably 1 or more in order to achieve preferable characteristics in red color development. The upper limit of the molar ratio of the amino group-containing compounds added with respect to all iridoid compounds in the reaction solution is not particularly defined, but examples thereof are 10 or less, preferably 8 or less, and more preferably 6 or less.

The red color development according to the present invention is preferably performed in a reaction solution under an inert gas atmosphere to improve the characteristics of the red color of the colorant composition produced. Any inert gas that does not practically inhibit the progress of the reaction can be used without limitation, and examples thereof are nitrogen gas, argon gas, and helium gas. It is preferable to use nitrogen gas and the like.

The red color development according to the present invention is preferably performed under acidic conditions

to improve the characteristics of the red color of the colorant composition produced. The pH for the red color development is preferably pH 3 to 6, and more preferably about pH 4 to 5. It is not preferable to perform this reaction under neutral or alkaline conditions, because the colorant composition generated will have a bluish color with less redness.

The pH can be adjusted to acidic conditions by using known techniques of adding inorganic and/or organic acids, for example. It is preferable to adjust the reaction solution to acidic conditions by adding ascorbic acid and/or similar compounds discussed above. When the reaction solution becomes too acidic, any ordinary means for raising the pH can be used without limitation. An example of such adjustment means is the addition of sodium hydroxide, sodium carbonate, potassium hydroxide, trisodium phosphate, or the like.

Though the red color development according to the present invention can be performed at room temperature of around 1 to 30°C or at normal temperature of around 15 to 25°C, it is preferable to perform heat treatment because the red color development can be enhanced dramatically. The temperature condition for the heat treatment is 50°C or higher, preferably 60°C or higher, and more preferably 70°C or higher. The upper limit can be the boiling point of the aqueous solution, for example, and it is preferably 100°C or lower for an aqueous solution under a normal pressure and 150°C or lower for an aqueous solution under a pressurized condition. Specifically, the temperature for heat treatment is within a range of 50 to 150°C, and preferably 50 to 100°C.

The reaction time is a factor determined by conditions such as temperature and thus can be determined properly, and if the temperature is set to room or normal temperature, it is desirable to perform the reaction for a long time of several hours to several days. If the reaction is performed under heating conditions, the reaction time is within a range of 1 minute to 96 hours, and preferably around 10 minutes to 72 hours, for example.

It is not preferable if the reaction conditions, which are defined by the combination of the temperature and/or the reaction time, are inadequate, because the amount of the red colorant compounds generated becomes too low. Also, it is not preferable if the reaction conditions, which are defined by the combination of the temperature and/or the reaction time, are too excessive, because unnecessary decomposition or the like may take place.

In some embodiments, the reaction process for the red color development can be performed a plurality of times. In some embodiments, various compounds and substances to be contained in the reaction solution can be added in several portions during the reaction.

[Refinement Process and the Like]

In the method for producing the red colorant composition according to the present invention, the reaction solution obtained by the operations described above can be used as the red colorant composition derived from iridoid compounds. Yet, it is preferable to isolate the red colorant composition derived from iridoid compounds of desired quality and/or form by conducting solid-liquid separation, refinement treatment, concentrating treatment, dilution treatment, pH adjustment, drying treatment, sterilization treatment or the like depending on the purpose of use.

These steps can be carried out not only at the final stage after the red color development but also after any of the steps or treatments described above as needed. Desired steps can also be performed in combination, and it is also possible to conduct a desired step a plurality of times.

In the production method according to the present invention, the solid-liquid separation can be performed by an ordinary method. For example, filtration, suction filtration, coprecipitation, or centrifugation can be employed to remove the solid or aggregated insoluble matter and the like. It is also preferable to use a filter aid (e.g. diatomaceous earth) when filtration is performed. The solid-liquid separation step can also be performed a plurality of times if desired.

In the production method according to the present invention, the refinement treatment can be performed by an ordinary method by which separation and purification of the colorant compounds can be conducted. For example, the refinement can be conducted by adsorption treatment using silica gel, porous ceramics, styrenic or aromatic synthetic resins, or the like. It can also be conducted by ion exchange treatment using cationic or anionic resins. It can also be conducted by membrane separation treatment using membrane filters, ultrafiltration membranes, reverse osmosis membranes, electrodialysis membranes, functional polymer membranes, or the like.

In the production method according to the present invention, it is preferable to perform a refinement treatment for eliminating unreacted low molecular compounds or decomposition products from the red colorant composition produced. For example, it is preferable to perform a refinement treatment with a molecular weight cutoff of 3,000 or less, and preferably 2,000 or less, so as to eliminate the low molecular substances. In the present invention, the ascorbic acid and the like which have been added excessively in the reaction described above are preferably removed after the red color development.

In the production method according to the present invention, operations such as concentration, dilution, and drying can be performed by ordinary methods. The refinement treatment can be performed by a method as described above. The pH adjustment operation can be performed by an ordinary method.

In the production method according to the present invention, the sterilization treatment can also be performed by an ordinary method. Examples of the sterilization method are heat treatment, high pressure treatment, high-pressure

heat treatment, sterile filtration, ultraviolet irradiation, and chemical treatment with disinfectants. Sterilization by heat treatment or high-pressure heat treatment is preferable.

Examples of the temperature condition for heat sterilization are 60°C or higher, preferably 70°C or higher, and more preferably 80°C or higher. The upper limit is not particularly limited as long as the components having functions of imparting stability are not negatively affected, and its examples are 140°C or lower under a pressurized condition, and, under a normal pressure, 100°C or lower, and preferably 95°C or lower.

It is considered that ascorbic acid and the like added at the time of the red color development according to the present invention do not practically affect the equilibrium of the reaction and can provide a product yield relative to the material as high as that obtained by a conventional technique.

[Method for Conferring Acid Resistance]

By performing the red color development described above during the process of producing the red colorant composition derived from iridoid compounds according to the present invention, it becomes possible to confer acid resistance to the produced red colorant composition derived from iridoid compounds. That is, in the present invention, it becomes possible to provide a method for imparting acid resistance to a produced red colorant composition derived from iridoid compounds, wherein step (1) mentioned above is performed in the process for producing the red colorant composition derived from iridoid compounds. The detailed features of the acid resistance imparted to the red colorant composition produced are described in Section 2 below.

Moreover, as the red color development described above is performed during the production process of the red colorant composition derived from iridoid compounds according to the present invention, it becomes possible to provide not only acid resistance but also color brightness to the produced red colorant composition derived from iridoid compounds. The detailed features of the color brightness provided to the red colorant composition produced are described in Section 2 below.

## 2. Red Colorant Composition Derived from Iridoid Compounds

The red colorant composition derived from iridoid compounds according to the present invention has the features as described below. Among these features of the red colorant composition according to the present invention, the excellent acid resistance should be emphasized. Since remarkable acid resistance can be achieved even at a pH of less than 3, the red colorant composition derived from iridoid compounds according to the present invention can be utilized for a wide range of purposes and products for which conventional red colorant compositions derived from iridoid compounds (and gardenia red colorant compositions) could hardly be used.

The red colorant composition according to the present invention can include features other than those described below as long as they do not interfere with the effects realized by the technical features of the present invention. Also, except for its essential technical features, the technical scope of the present invention is not limited to a mode in which all of the following features are included.

The red colorant composition derived from iridoid compounds according to the present invention can be obtained by the production method described in Section 1 above. In the red color development according to the present invention, an assembly of colorant compounds is generated as a result of complex chemical reaction systems. Their reaction mechanisms have not been unveiled completely, and the detailed structures and composition of the compounds that bring about the red color development are yet to be clarified. Under the circumstances at the time of the filing of this application, expensive apparatus such as a mass spectrometer is necessary for analyzing the physical properties of the colorant compounds generated and for specifying the structural features causing the red color development, and the financial burden is heavy. Moreover, it is difficult to specify the chemical structures of these compounds even if such apparatus is used. Furthermore, it takes time to complete such analysis, when speediness required for filing a patent application is taken into consideration.

Therefore, in the invention of the red colorant composition derived from iridoid compounds according to the present disclosure, it is inappropriate to regard the scope of the invention as being ambiguous based on the fact that the wording of the claims includes steps for producing a product.

[Compositional Features]

The red colorant composition according to the present invention is composed of the compounds obtained by the oxidative polymerization of reaction products generated by the reaction between the iridoid aglycones having a carboxyl group at position 4 of the iridoid skeleton and the amino group-containing compounds. In the compound composition of the red colorant composition according to the present invention, ascorbic acid and/or similar compounds thereof are not necessarily included. Even if ascorbic acid and/or similar compounds thereof are removed from the

composition produced, the color characteristics and acid resistance of the red colorant composition according to the present invention are not affected.

The form of the red colorant composition according to the present invention can be a liquid form, a paste form, a gelatinous form, a semi-solid form, a solid form, or a powdery form, for example, but is not particularly limited.

Compound Structure

An example of the iridoid compound before reaction, which serves as the compositional unit of the red colorant composition according to the present invention, is the aglycone of an iridoid compound having a carboxyl group at position 4 of the iridoid skeleton as described in Section 1 above. This iridoid aglycone is preferably the aglycone of geniposide ester hydrolysate and/or the aglycone of geniposidic acid.

[Properties Related to Acid Resistance]

Because of its compositional features, the red colorant composition derived from iridoid compounds according to the present invention has excellent acid resistance compared with those obtained by conventional techniques. More specifically, the red colorant composition derived from iridoid compounds according to the present invention has improved dissolution stability under acidic conditions at a pH of less than 3, which has been difficult to achieve in conventional techniques, and is a colorant composition in which aggregation and precipitation are suppressed.

The pH at which the red colorant composition according to the present invention exhibits acid resistance is less than 7, and it shows improved acid resistance compared with those obtained by conventional techniques especially at a pH of less than 3. The red colorant composition according to the present invention has especially remarkable acid resistance compared with those obtained by conventional techniques at a pH of less than 3, preferably pH 2.5 or less, and more preferably pH 2.2 or less. The lower limit of the pH is not particularly defined, and it can be pH 0.1 or higher, and preferably pH 0.5 or higher, for example.

Color Residual Rate

The red colorant composition derived from iridoid compounds according to the present invention has excellent acid resistance. From this viewpoint, it is preferable that the value indicating the color residual rate of the red colorant composition derived from iridoid compounds according to the present invention is within the following range when measured by the method as described in (A) below.

That is, the red colorant composition derived from iridoid compounds according to the present invention has a feature as follows:

(A) A color residual rate of a filtrate of an aqueous solution is 35% or more, preferably 40% or more, more preferably 50% or more, more preferably 60% or more, and even more preferably 65% or more, when the aqueous solution contains the red colorant composition derived from iridoid compounds at a content of 0.05 mass% when calculated in terms of color value ($E^{10\%}{}_{1\ cm}$) 50.5, is adjusted to have a pH being 2.2 using McIlvaine buffer, and is filtered through a 0.45-μm filter to obtain said filtrate.

The red colorant composition derived from iridoid compounds according to the present invention has excellent dissolution stability under acidic conditions even after low-temperature storage. From this viewpoint, it is preferable that the value indicating the color residual rate of the red colorant composition derived from iridoid compounds according to the present invention is within the following range when measured by the method as described in (A') below.

That is, the red colorant composition derived from iridoid compounds according to the present invention has a feature as follows:

(A') A color residual rate of a filtrate of an aqueous solution is 25% or more, preferably 30% or more, more preferably 35% or more, and even more preferably 40% or more, when the aqueous solution contains the red colorant composition derived from iridoid compounds at a content of 0.05 mass% when calculated in terms of color value ($E^{10\%}{}_{1\ cm}$) 50.5, is adjusted to have a pH being 2.2 using McIlvaine buffer, and, after being stored at 10°C for one week, is filtered through a 0.45-μm filter to obtain said filtrate.

Brief Summary on Acid Resistance

The features related to acid resistance as described above demonstrate the dissolution stability of the red colorant composition derived from iridoid compounds according to the present invention under acidic conditions. The values indicating acid resistance as described above cannot be achieved by conventional techniques such as the red

colorant composition derived from iridoid compounds produced under the presence of organic acids, such as citric acid, and the red colorant composition obtained by the addition of taurine (Patent Document 3: Japanese Patent No. 4605824). For example, in these conventional techniques, a large amount of aggregation and precipitation is generated at a pH of less than 3, and the features as described above cannot be realized.

Specifically, the conventional red colorant composition derived from iridoid compounds produced by using organic acids such as citric acid has severe precipitation and color fading problems under aforementioned acidic conditions and cannot achieve the features related to acid resistance that can be expressed by aforementioned values. Meanwhile, though improvement of acid resistance can be observed in the conventional red colorant composition produced by the technique for conferring acid resistance by the addition of taurine disclosed in Patent Document 3, the acid resistance at a pH of less than 3 is insufficient (*see* Examples 7, 8 and 15 in the Examples section below). Moreover, the technique disclosed in Patent Document 3 has problems from the viewpoint of the production cost and is difficult to be applied to a wide range of applications including foods and beverages.

[Color Characteristics]

With regard to its color characteristics, the red colorant composition derived from iridoid compounds according to the present invention has excellent color brightness because of its compositional features. Specifically, while the conventional red colorant compositions derived from iridoid compounds tend to be bluish and exhibit purple or dark red color, the red colorant composition derived from iridoid compounds according to the present invention has vivid red color.

Chroma Value (Saturation) and a Value (Redness)

The red colorant composition derived from iridoid compounds according to the present invention has excellent chroma and exhibits clear red color. From this viewpoint, it is preferable that the red colorant composition derived from iridoid compounds according to the present invention has the values in the Hunter Lab colorimetric system that fall within the following ranges when measured by the methods as described below.

That is, the red colorant composition derived from iridoid compounds according to the present invention has a feature as follows:

(b-1) An aqueous solution has a chroma value in the Hunter Lab colorimetric system being increased by 5% or more compared with a standard colored solution as described below, when the aqueous solution contains the red colorant composition derived from iridoid compounds at a color value ($E^{10\%}{}_{1\ cm}$) of 0.05 and is adjusted to have a pH being 5.0 using McIlvaine buffer.

Furthermore, the red colorant composition derived from iridoid compounds according to the present invention has a feature as follows:

(b-2) An aqueous solution has an a value in the Hunter Lab colorimetric system being increased by 6% or more compared with a standard colored solution as described below, when the aqueous solution contains the red colorant composition derived from iridoid compounds at a color value ($E^{10\%}{}_{1\ cm}$) of 0.05 and is adjusted to have a pH being 5.0 using McIlvaine buffer.

The standard colored solution as used herein indicates a solution that contains a standard colorant such that the color value ($E^{10\%}{}_{1cm}$) is 0.05 and adjusted to have a pH of 5.0 using McIlvaine buffer. The standard colorant can be the red colorant composition obtained by performing the red color development process by a method for producing iridoid compounds described in Section 1 above, except that, instead of ascorbic acid and/or similar compounds, citric acid having a molar ratio of 4.3 with respect to all the iridoid compounds in the reaction solution is added. Specifically, the red colorant composition prepared by a method for producing Sample 1-1 in Example 1 in the Examples section below can be used as the standard colorant.

Since the values related to the Hunter Lab colorimetric system in this technical field tend to vary depending on the measurement apparatus and the like, it is desirable that these values are obtained by using the same measurement apparatus and evaluated by comparison with the standard colored solution.

The rate of increase in the chroma value (degree of improvement in saturation) is 5% or more, preferably 8% or more, more preferably 10% or more, and even more preferably 15% or more. It is preferable that the rate of increase is higher, because the higher the rate, the higher the saturation and the more vivid the color. The upper limit of the rate of increase in the chroma value is not particularly limited, and its example is 50% or less.

The rate of increase in the a value (degree of the shift toward red) is 6% or more, preferably 8% or more, more preferably 10% or more, and even more preferably 15% or more. It is preferable that the rate of increase is higher, because the higher the rate, the less bluish and the more reddish the color. The upper limit of the rate of increase in the a value is not particularly limited, and its example is 55% or less.

The rate of increase in the chroma value can be calculated as follows. The chroma value of the standard colored solution is subtracted from the chroma value of the analyte solution, and then, the value obtained is divided by the chroma value of the standard colored solution. For example, when the chroma value of the standard colored solution is 25 and that of the analyte solution is 30, the rate of increase in the chroma value is 20%. The rate of increase in the a value can also be calculated in the same way.

The *b* Value (Yellowness and Blueness)

The *b* value of the red colorant composition derived from iridoid compounds according to the present invention can be within a range that does not differ greatly from the conventional red colorant composition derived from iridoid compounds produced under the presence of organic acids such as citric acid. Also, since shift to clear red is favorable in the red colorant composition according to the present invention, the *b* value can shift to the negative direction (shift to yellow) to a great extent compared with the conventional red colorant composition derived from iridoid compounds produced under the presence of organic acids such as citric acid, as long as the color characteristics of the colorant composition obtained can fulfill the requirements for realizing clear redness as described below.

Here, an example of the "conventional red colorant composition derived from iridoid compounds produced under the presence of organic acids such as citric acid" is the standard colorant as described above.

The *L* Value (Lightness)

The red colorant composition derived from iridoid compounds according to the present invention exhibits a light color because, in addition to the color characteristics described above, its tone is entirely bright. It is preferable that the red colorant composition according to the present invention has lightness, which is defined by the *L* value, to the same, similar, or greater extent than the conventional red colorant composition derived from iridoid compounds produced using organic acids such as citric acid.

From this viewpoint, it is preferable that the *L* value in the Hunter Lab colorimetric system of the red colorant composition derived from iridoid compounds according to the present invention is within the following range when measured by the method as described in (b-3) below.

That is, the red colorant composition derived from iridoid compounds according to the present invention has a feature as follows:

(b-3) When an aqueous solution that contains the red colorant composition derived from iridoid compounds at a color value ($E^{10\%}{}_{1\,cm}$) of 0.05 and is adjusted to have a pH being 5.0 using McIlvaine buffer is prepared, following Equation 11 is satisfied.

$$\text{Equation 11:}\quad L_1 - L_0 \geq -0.5$$

Here, $L_1$ indicates the *L* value of the solution containing the red colorant composition derived from iridoid compounds to be analyzed. $L_0$ indicates the *L* value of the solution prepared by a method described above except that the standard colorant is used. The standard colorant as described above can be used here.

The value on the right side of Equation 11 indicates the increase in the *L* value. Its preferable example is 0 or more. It is more preferably 0.5 or more, and even more preferably 1 or more.

Entire Color

The red colorant composition derived from iridoid compounds according to the present invention has a color ranging from reddish purple to yellowish red at pH 5. Specifically, the hue value of the colorant composition in the Munsell color system (or the color names in the Japanese Industrial Standards) ranges from 10P (reddish purple) to 5RP (red-purple), 10RP (purplish red), and 5R (red). The colorant composition preferably has a color ranging from red-purple to red at pH 5, and its hue value in the Munsell color system (or the color names in the Japanese Industrial Standards) is preferably in a range from 5RP (red-purple) to 10RP (purplish red) and 5R (red).

The red colorant composition according to the present invention preferably has a high chroma value and a high a value in the Hunter Lab colorimetric system. The color characteristics expressed by the aforementioned values in the Hunter Lab colorimetric system cannot be achieved by the conventional red color development performed under the presence of organic acids such as citric acid.

The color characteristics of the red colorant composition derived from iridoid compounds according to the present invention can also be expressed by the ratio of the absorbance at the maximum absorption wavelength to the absorbance at a fixed wavelength.

In the red colorant composition according to the present invention, when a solution containing the red colorant composition is adjusted to pH 5 using McIlvaine buffer, the solution has a maximum absorption wavelength of 520 to 545 nm, and preferably 530 to 542 nm.

In this specification, the ratio of the absorbance at the maximum absorption wavelength to the absorbance at a fixed wavelength can be expressed by $A_{\lambda max}$ / $A_{600nm}$, which is the ratio of the absorbance at the maximum absorption wavelength ($\lambda max$) to the absorbance at the wavelength of 600 nm. The $A_{\lambda max}$ / $A_{600nm}$ value indicates the ratio of the absorbance at the maximum absorption wavelength ($\lambda max$) to the absorbance at the wavelength of 600 nm, and the higher the ratio, the lighter and the less dull the color.

From this viewpoint, it is preferable that the $A_{\lambda max}$ / $A_{600nm}$ value of the red colorant composition according to the present invention is within the following range when measured by the method as described in (C) below.

That is, the red colorant composition derived from iridoid compounds according to the present invention has a feature as follows:

(C) An $A_{\lambda max}$ / $A_{600\,nm}$ value of an aqueous solution is 4.6 or more, and preferably 4.8 or more, when the aqueous solution contains the red colorant composition derived from iridoid compounds at a color value ($E^{10\%}{}_{1\,cm}$) of 0.05 and is adjusted to have a pH being 5.0 using McIlvaine buffer. Especially, it is preferable that the $A_{\lambda max}$ / $A_{600\,nm}$ value is 4.9 or more, preferably 5 or more, and more preferably 5.2 or more from the viewpoint of developing a clear red color.

The ratio of the absorbance at the maximum absorption wavelength to the absorbance at a fixed wavelength can also be expressed by $A_{\lambda max}$ / $A_{430nm}$, which is the ratio of the absorbance at the maximum absorption wavelength ($\lambda max$) to the absorbance at the wavelength of 430 nm. The $A_{\lambda max}$ / $A_{430nm}$ value indicates the ratio of the absorbance at the maximum absorption wavelength ($\lambda max$) to the absorbance at the wavelength of 430 nm, and the higher the ratio, the less yellowish and the less dull the color.

From this viewpoint, it is preferable that the $A_{\lambda max}$ / $A_{430nm}$ value of the red colorant composition according to the present invention is within the following range when measured by the method as described in (D) below.

That is, the red colorant composition derived from iridoid compounds according to the present invention has a feature as follows:

(D) An $A_{\lambda max}$ / $A_{430\,nm}$ value of an aqueous solution is 2.5 or more, when the aqueous solution contains the red colorant composition derived from iridoid compounds at a color value ($E^{10\%}{}_{1\,cm}$) of 0.05 and is adjusted to have a pH being 5.0 using McIlvaine buffer.

In the red colorant composition according to the present invention, it is preferable that the value obtained by dividing the absorbance at the maximum absorption wavelength by the absorbance at a fixed wavelength is higher, because the higher the value, the lighter and the less dull the color. The color characteristics expressed by this ratio cannot be achieved by the conventional red color development performed under the presence of organic acids such as citric acid.

The color characteristics indicated by the values that fall within the aforementioned ranges show that the red colorant composition derived from iridoid compounds according to the present invention has a bright, clear red color. Such color characteristics cannot be achieved by the conventional red colorant compositions derived from iridoid compounds produced by using organic acids such as citric acid. For example, the red colorant composition produced under the presence of taurine by the method disclosed in Patent Document 3, which is reported to have acid resistance, has less saturation and has a paler red color compared with the red colorant composition according to the present invention, and its color characteristics greatly differ from those of the red colorant composition according to the present invention.

[Stability against Light and Heat]

The red colorant composition derived from iridoid compounds according to the present invention has high stability against light and heat, and its color characteristics are not easily damaged even under light irradiation or exposure to heat. The red colorant composition according to the present invention has heat resistance and light resistance to the same or higher extent than the conventional red colorant composition derived from iridoid compounds produced by adding citric acid.

Because of this stability, the red colorant composition derived from iridoid compounds according to the present invention can be suitably used to color commercial products that are required to have resistance against such conditions as fluorescent light irradiation and high temperature storage. Also, it can be suitably used for products that are supposed to undergo heat treatment, such as retort sterilization, processing, and cooking.

The red colorant composition derived from iridoid compounds according to the present invention is a light-resistant colorant composition, having stability against light irradiation. Specifically, it is preferable that the value indicating the color residual rate is within the following range when measured by the method as described in (E) below.

That is, the red colorant composition derived from iridoid compounds according to the present invention has a feature as follows:

(E) When an acid-sugar solution that contains the red colorant composition derived from iridoid compounds at a content

of 0.05 mass% when calculated in terms of color value ($E^{10\%}{}_{1\ cm}$) 50.5 and that is adjusted to have 10° Brix by high fructose corn syrup and to have a pH being 5.0 is stored under white fluorescent light of 10,000 lux at 10°C for 5 days, a color residual rate of the acid-sugar solution is 50% or more, preferably 60% or more, and more preferably 70% or more compared with before storage.

The red colorant composition derived from iridoid compounds according to the present invention has stability against heat. Specifically, it is preferable that the value indicating the color residual rate is within the following range when measured by the method as described in (F) below.

That is, the red colorant composition derived from iridoid compounds according to the present invention has a feature as follows:

(F) When an acid-sugar solution that contains the red colorant composition derived from iridoid compounds at a content of 0.05 mass% when calculated in terms of color value ($E^{10\%}{}_{1\ cm}$) 50.5 and that is adjusted to have 10° Brix by high fructose corn syrup and to have a pH being 5.0 is stored in a dark place at 50°C for 5 days, a color residual rate of the acid-sugar solution is 50% or more, preferably 60% or more, and more preferably 70% or more compared with before storage.

The features described above indicate that the red colorant composition according to the present invention has excellent light and heat resistance to the same or higher extent than the conventional gardenia red color or the like. The red colorant composition according to the present invention has the same level of stability as the conventional red colorant composition produced under the presence of organic acids such as citric acid, and it is indicated that the red colorant composition according to the present invention can be utilized as a colorant composition excellent in preservability and processing properties.

[Gardenia Red Colorant Composition]

A specific example of the red colorant composition derived from iridoid compounds according to the present invention is a gardenia red colorant composition produced by using gardenia fruits or their extract as the raw material for obtaining iridoid compounds.

The term "gardenia red colorant composition" as used herein indicates the colorant composition containing red colorant compounds generated by the reaction between the iridoid aglycones, which are derived from gardenia fruits described in Section 1 above and have a carboxyl group at position 4 of the iridoid skeleton, and the amino group-containing compositions.

The Eighth Edition of the Specifications and Standards for Food Additives (published by the Ministry of Health, Labour and Welfare of Japan) defines the gardenia red color as "a substance obtained by adding β-glucosidase to the mixture of ester-hydrolysates of iridoid glycosides obtained from gardenia fruits and protein-decomposed substances", but the production process in this definition merely describes one limited example of the production processes in the present disclosure. The gardenia red colorant composition according to the present invention includes a wider range of colorant compositions than those defined in general.

Moreover, the gardenia red colorant composition according to the present invention includes not only the red colorant compositions produced by directly using gardenia fruits or their extract as the raw material but also those produced by using geniposide and/or geniposidic acid obtained from gardenia fruits by separation or refinement. The red colorant compositions produced from the derivatives of these substances, that is, genipin and/or genipic acid, are also encompassed.

[Terminology Related to Colorants]

Some important terms related to colorants used in this specification are explained below.

The term "Hunter Lab colorimetric system (Lab system)" as used herein refers to a colorimetric system represented by a color solid composed of an orthogonal coordinate system consisting of the *a* and *b* axes indicating chromaticity and the *L* axis vertical to these axes.

Here, the "*L* value" is a numerical value expressing brightness. The color is white when *L* = 100 and is black when *L* = 0. The "*a* value" is a numerical value indicating redness and greenness. The redness becomes stronger as the *a* value increases in the positive direction, and the greenness becomes stronger as the *a* value increases in the negative direction. The "*b* value" is a numerical value indicating yellowness and blueness. The yellowness becomes stronger as the *b* value increases in the positive direction, and the blueness becomes stronger as the *b* value increases in the negative direction.

The "chroma value" as used herein is the distance from the origin in the Hunter Lab colorimetric system expressed by Equation 1 below. It is used as a value indicating the saturation. The higher the value, the more vivid the color.
[Equation 1]

$$\text{Chroma} = \sqrt{a^2 + b^2} \quad \cdots \text{ Equation 1}$$

The term "color difference ($\Delta E$)" as used herein is the distance between the plots for two colors, ($a_1$, $b_1$, $L_1$) and ($a_2$, $b_2$, $L_2$), which is calculated by Equation 2 below.
[Equation 2]

$$\Delta E = \sqrt{(L_1 - L_2)^2 + (a_1 - a_2)^2 + (b_1 - b_2)^2} \quad \cdots \text{ Equation 2}$$

The "hue value" as used herein is a value indicating hue obtained by converting the angle formed by the straight line between the origin and the plot (*a, b*) in the orthogonal coordinate system of the *a* and *b* axes of the Hunter Lab colorimetric system to the hue in the Munsell hue circle. It is expressed by the combination of a letter and a number.
The term "maximum absorption wavelength" ($\lambda max$) as used herein refers to the light wavelength (nm) in the visible light region at which a colorant or a colorant composition has the maximum absorption. The term "absorbance" as used herein refers to a value indicating the degree of light absorption by a substance. For example, the absorbance ($A_\lambda$) at the maximum absorption wavelength ($\lambda max$) can be calculated by Equation 3 below. In this equation, $A$ represents the absorbance, $\lambda$ represents the maximum absorption wavelength, $A_\lambda$ represents the absorbance at the maximum absorption wavelength, $I$ represents the incident light intensity, and $I_0$ represents the transmitted light intensity.
[Equation 3]

$$A_\lambda = -\log_{10}(I / I_0) \quad \cdots \text{ Equation 3}$$

The term "color residual rate" as used herein refers to the value calculated by Equation 4 below based on the absorbance at the maximum absorption wavelength of the colorant measured before and after a test on stability or the like. In this specification, the ratio of the colorant compounds maintaining the maximum absorption wavelength is evaluated as the color residual rate to assess the ratio of the colorant compounds that stably maintain their color characteristics.
[Equation 4]

$$\text{Color residual rate (\%)} = \frac{\text{Absorbance at the maximum absorption wavelength after the test}}{\text{Absorbance at the maximum absorption wavelength before the test}} \times 100$$

$$\cdots \text{ Equation 4}$$

The term "color value" as used herein refers to the "color value $E^{10\%}{}_{1cm}$", and "color value $E^{10\%}{}_{1cm}$" is a value calculated by measuring the absorbance ($A$) of a solution containing 10 mass% of a colorant composition at the maximum absorption wavelength ($\lambda max$) in the visible light region by using a measurement cell with the optical path length of 1 cm.
The term "in terms of color value" as used herein means to calculate the content of the colorant (colorant composition) as a content for a certain unit of color value. For example, "0.05 mass% when calculated in terms of color value 60" means that the content of the colorant (colorant composition) in a solution is 0.05 mass% when the solution is adjusted to have a color value of 60.
The term "McIlvaine buffer" as used herein refers to a buffer solution prepared by using citric acid and phosphate ($Na_2HPO_4$) and is also known as the citric acid buffer.

3. Application

The red colorant composition derived from iridoid compounds according to the present invention is a colorant composition having features as described in Section 2 above and is a red colorant composition excellent in acid resistance. Especially, remarkable acid resistance can be achieved even at a pH of less than 3. Therefore, the red colorant composition according to the present invention can be utilized for a wide range of purposes and products for which conventional

red colorant compositions derived from iridoid compounds (and conventional gardenia red colorant compositions) could hardly be used.

[Pigment Preparation]

The red colorant composition derived from iridoid compounds according to the present invention can be utilized as a pigment preparation. Especially, when the red colorant composition according to the present invention is a gardenia red colorant composition, it can be suitably used as a gardenia red pigment preparation.

The form of the pigment preparation according the present invention is not particularly limited, and examples thereof are a liquid form, a paste form, a gelatinous form, a semi-solid form, a solid form, and a powdery form. Processed solid forms such as a granular form and a tablet form are also included.

In addition, because of its water-soluble nature, the red colorant composition derived from iridoid compounds according to the present invention can be directly used as a water-soluble pigment preparation. It can also be processed as oil-soluble (W/O) or double-emulsified (W/O/W) pigment preparations.

The ratio of the red colorant composition derived from iridoid compounds contained in the pigment preparation according to the present invention can be adjusted depending on the type and purpose of use of the pigment preparation and is not particularly limited. For example, it is suitable that the red colorant composition is contained such that the color value $E^{10\%}_{1cm}$ is 20 or more, preferably 30 or more, and more preferably 40 or more. The upper limit of the color value of the pigment preparation according to the present invention is not particularly defined, and for example, the upper limit of the color value $E^{10\%}_{1cm}$ can be 800.

While the ratio of the red colorant composition derived from iridoid compounds contained in the pigment preparation can be calculated in terms of color value as described above, it can be 0.1 to 99 mass%, preferably 1 to 90 mass%, and more preferably 5 to 75 mass% when calculated in terms of mass.

The pigment preparation according to the present invention can contain other ingredients as long as the acid resistance or the color characteristics of the red colorant composition according to the present invention are not practically lost. Specifically, the colorant preparation according to the present invention can contain additives having a function of stabilizing or improving the color characteristics and the like. Examples of the additives that can be contained include, but are not limited to, antioxidants, acidity regulators, polysaccharide thickeners, and other materials for food products.

Moreover, the pigment preparation according to the present invention can contain other colorants in addition to the red colorant composition according to the present invention. With other colorants added, the pigment preparation according to the present invention can become a pigment preparation that is adjusted to produce a desired color. Natural colorants having color characteristics as stable as those of the red colorant composition according to the present invention can be suitably used as the colorants to be contained in the pigment preparation. Examples thereof include, but are not limited to, such natural colorants as gardenia yellow, gardenia blue, safflower color, anthocyanin-based color, *Monascus* color, turmeric color, tamarind color, Japanese persimmon color, caramel color, spirulina color, Kaoliang color, cochineal color, and tomato color.

[Products]

The red colorant composition or the pigment preparation according to the present invention can be suitably used not only for purposes as colorants for which conventional red colorant compositions (and conventional gardenia red colorant compositions) derived from iridoid compounds have been used, but also in a wide range of fields, such as for purposes for which the method or timing of adding the red colorant composition (or the gardenia red colorant composition) has been limited in conventional techniques, and moreover, for products for which the usage of the red colorant composition itself has been impossible in conventional techniques.

The red colorant composition or the pigment preparation according to the present invention can be suitably used as a natural colorant for such products as foods, beverages, cosmetic products, medicines, quasi-drugs, personal hygiene products, and feed. That is, the present invention makes it possible to provide foods, beverages, cosmetic products, medicines, quasi-drugs, personal hygiene products, and feed containing the red colorant composition or the pigment preparation according to the present invention.

The products that can be colored by the red colorant composition or the pigment preparation according to the present invention include, but are not limited to, the following examples.

Examples of "foods" and "beverages" are beverages, frozen or chilled desserts, desserts, sugar confectionery (e.g. candies, gummies, and marshmallows), chewing gums, chocolate, confectionery (e.g. cookies), bread, processed agricultural products (e.g. pickled vegetables), processed meat products, processed marine products, dairy products, noodles, seasonings, jellies, syrups, jams, sauces, and alcoholic drinks.

Examples of "cosmetic products" are skin lotions, lipsticks, sunscreen cosmetics, and makeup cosmetics.

Examples of "medicines" are various tablets, capsules, liquid medicines, lozenges, and mouthwashes.

Examples of "quasi-drugs" are nutritional supplements, various supplements, dentifrice, mouth freshener, deodorants, hair growth tonics, anti hair loss tonics, and skin moisturizers.

Examples of "personal hygiene products" are soaps, detergents, shampoos, conditioners, hair treatments, dentifrice, and bath additives.

Examples of "feed" are various pet foods such as cat foods and dog foods, and feed for aquarium and farmed fish.

The colorant composition and the pigment preparation according to the present invention have excellent dissolution stability especially under acidic conditions and thus can be suitably used to color the products which are at least partially or entirely acidic. That is, the colorant composition or the pigment preparation according to the present invention can be preferably used to color products in which at least a part or the entire part of the product is acidic, and moreover, whose acidic portion is required to maintain red color. Here, preferred examples of the acidic conditions are pH 3.5 or less, preferably pH 3 or less, and more preferably less than pH 3.

Specific examples of the products to be colored in the present invention are beverages, frozen or chilled desserts, desserts, and sugar confectionery, many of which have a pH of around 3. While many of these products have high transparency and the occurrence of turbidity and precipitation reduces their commercial value, the red colorant composition and the pigment preparation according to the present invention can suppress the occurrence of turbidity and precipitation under acidic conditions and can suitably color these products.

Moreover, since the colorant composition and the pigment preparation according to the present invention not only have acid resistance but also have excellent color characteristics, exhibiting a vivid bright red color, materials whose basic color is milky white, which tends to become dull, can also be colored with bright red tone. For example, the colorant composition and the pigment preparation according to the present invention can be suitably used to color milk beverages, dairy products, and processed fish meat products.

Furthermore, since the colorant composition and the pigment preparation according to the present invention have light resistance, they can be suitably used to color products which are supposed to be exposed to light during storage, display, and the like. Also, since the colorant composition and the pigment preparation according to the present invention have heat resistance, they can be suitably used for products which are supposed to be stored under high temperature.

Since the colorant composition and the pigment preparation according to the present invention are produced by a method whose technical feature is the addition of ascorbic acid or the like, they are preferable from the viewpoint of safety and can be suitably used to color products to be ingested into the human body. Especially, it can be suitably used to color foods and beverages.

The colorant composition and the pigment preparation according to the present invention can be suitably used for coloring purposes in the production process of the products as described above.

The coloring step can be performed by an ordinary method for coloring these products except that the colorant composition or the pigment preparation according to the present invention is used as the natural colorant. The ratio of the red colorant composition or the pigment preparation according to the present invention contained in these products can be adjusted depending on the type and purpose of use of the product. For example, the content of the colorant composition in the product can be adjusted to 0.001 to 1 mass%, preferably 0.005 to 0.5 mass%, more preferably 0.01 to 0.2 mass%, and even more preferably 0.02 to 0.1 mass% when calculated in terms of color value 80.

The colorant composition or the pigment preparation according to the present invention can be used for a product that is treated under acidic conditions as described above in at least a part of or the entire production process, even if the pH of the final product is weak acidic to alkaline. That is, it can be suitably used for coloring purposes in a production process, at least a part or the entire part of which includes a step/steps performed at a pH of less than 3, even if the final product has a pH of 3.5 or higher.

[Various Production Methods]

The present invention includes methods for producing pigment preparations, various products, and the like. That is, the present invention includes the method for producing pigment preparations, foods, beverages, cosmetic products, medicines, quasi-drugs, personal hygiene products, feed, or the like, the method including a step in which the red colorant composition derived from iridoid compounds obtained by the production method as described above is added to the product.

The paragraphs above on pigment preparations and various products can be applied when the content and the like of the red colorant composition derived from iridoid compounds to be added in the method for producing the pigment preparations and various products are determined. Also, in the method for producing the pigment preparations and various products, processes in their conventional production methods can be employed except that the aforementioned red colorant composition derived from iridoid compounds is used.

[Effects in Various Products]

While the products containing the red colorant composition or the pigment preparation according to the present invention can be colored with clear red excellent in acid resistance, other remarkable effects can be further achieved in some products to be colored. Such effects are various and include the secondary effects of those described above, as well as other effects of different nature.

Prevention of Color Dullness in Heated Products

When the product to be colored requires heat treatment and when the heat treatment is conducted with an ordinary gardenia red color or the like added to the material, the red color tends to become dull and dark. In contrast, when the coloring is performed using the red colorant composition or the pigment preparation according to the present invention, even a product that requires heat treatment can be colored in clear red.

More specifically, in the present disclosure, examples of the products whose color tends to become dull due to heat treatment are those that require sterilization treatment after mixing the materials, especially those that require retort sterilization. In some embodiments, examples of such products are processed fish meat products (e.g. fish sausages, *kamaboko* steamed fish paste, and other fish paste products) and livestock meat products (e.g. ham, sausages, and bacon). Other products that are generally classified in the same category as the aforementioned products subjected to heat sterilization treatment are also included. In the present disclosure, even the food products whose color tends to become dull after heat treatment can be colored with clear red.

Prevention of Uneven Coloration in Livestock Meat Products and Processed Marine Products

When the product to be colored is a livestock meat product or a processed marine product and when the pickling liquid is prepared using an ordinary gardenia red color or the like, the product tends to be colored unevenly by streaks or patches due to the characteristics of the various kinds of tissue or the like constituting the product, and uniform coloration is difficult in some cases. In contrast, when the coloring is performed using the red colorant composition or the pigment preparation according to the present invention, even a livestock meat product or a processed marine product can be colored uniformly in clear red, with the unevenness of the color suppressed.

In some preferable embodiments, for coloring livestock meat products or processed marine products, the material of the livestock meat product is soaked in a pigment solution (e.g. pickling liquid), or alternatively, a pigment solution (e.g. pickling liquid) is injected into the material of the livestock meat product. In prior art techniques, such methods tend to cause uneven coloration, which hardly occurs in fish paste products or the like. However, when such coloring processes are performed using the red colorant composition or the colorant preparation according to the present invention, the product can be colored uniformly in clear red, with the unevenness of the color being suppressed.

Examples of products in which uneven coloration is desirably suppressed in the present disclosure are livestock meat products and processed marine products. Specific examples of processed livestock products are meat products in general, such as ham, sausages, bacon, slices, various parts of meat, minced meat, roasted products, grilled meat, and smoked products. Other products that are generally classified in the same category as the aforementioned meat products are also encompassed. Examples of the meat material are beef, pork, and chicken, and in principle, the present disclosure can be applied to a wide variety of material derived from the tissue or the like of the species of mammals, aves, reptiles, and amphibians.

Examples of processed marine products are meat products

in general, such as fish sausages, fish paste products, slices, various parts of fish meat, roe products, and smoked products. Other products that are generally classified in the same category as the aforementioned processed marine products are also included. An example of the material of the processed marine products is fish meat, and in principle, the present disclosure can be applied to a wide variety of material derived from the tissue or the like of the species of fishes.

Prevention of Uneven Coloration in Processed Agricultural Products

When the product to be colored is a processed agricultural product such as pickled vegetables and when the colorant solution or the like is prepared using an ordinary gardenia red color or the like, the product tends to be colored unevenly due to the characteristics of the plant tissue or the like constituting the material, and uniform coloration is difficult in some cases. In contrast, when the coloring is performed using the red colorant composition or the pigment preparation according to the present invention, even a processed agricultural product such as pickled vegetables can

be colored uniformly in clear red, with the unevenness of the color being suppressed.

Examples of pickled products, which are agricultural products, in which uneven coloration is desirably suppressed in the present disclosure are *asazuke* (quick pickled vegetables), *takuan* (pickled daikon radish), *shibazuke* (pickled cucumbers and eggplants), *fukujinzuke* (chopped mixed pickled vegetables), *umeboshi* (pickled fruits of *Prunus mume*) and *kimchi* (Korean salt-fermented vegetables). Other products that are generally classified in the same category as the aforementioned pickled products are also included. Examples of the plant material of these products are vegetables, leaf vegetables, root vegetables, fruit vegetables, and fruits, and in principle, the present disclosure can be applied to a wide variety of material derived from plant tissue.

Pink Tone

When the material of the product to be colored has a whitish color and when the product is colored using an ordinary gardenia red color or the like, the color tends to become dark bluish due to the tone generated by the mixing of the pigments and the material, and it has been difficult to create a light pink hue. In contrast, when the coloring is performed using the red colorant composition or the colorant preparation according to the present invention, a light pink color can be obtained even if the color of the colorant is mixed with that of the product whose material or the like has a whitish color. It becomes even possible to develop a color reminiscent of strawberries, which has been difficult to obtain with an ordinary gardenia red color.

Examples of the products which are desirably colored with pink in the present disclosure are bread, chewing gums, pudding, Bavarian cream, strawberry milk, various dairy products, candies, fillings, cream, cookies, donuts, cakes, biscuits, syrups, and jams. Other products that are generally classified in the same category as the aforementioned products are also included. In the present disclosure, products whose material or the like has a whitish color can be colored with light pink because the generation of a dark bluish color due to the mixing of the colorants with the whitish material is suppressed.

Examples

Hereinafter, the present invention will be explained with examples, but the scope of the present invention is not limited thereto.

FIG. 1 is a flowchart showing the main steps in the production process of the red colorant composition derived from iridoid compounds according to the present invention. The term "molar ratio with respect to GP" as used herein refers to the molar ratio of the compounds used, added, or contained in the production process of the gardenia red colorant composition with respect to geniposide, which is the substance serving as the raw material. It may also be expressed as "eq" in this specification.

In this Examples section, the asterisk symbol (*) indicates the product manufactured by San-Ei Gen F.F.I., Inc. and the double asterisk symbol (**) indicates a trademark of San-Ei Gen F.F.I., Inc.

Example 1: Production of Gardenia Red Colorant Composition

Ascorbic acid was added during the reaction in the production process of the gardenia red colorant composition, and its effects on the color characteristics of the gardenia red colorant composition were evaluated.

(1) Preparation of Gardenia Red Colorant Composition

A solution of 110 g containing purified geniposide (hereinafter, may be abbreviated as GP) at a content of 32 % was prepared, 22.5 g of 48 % sodium hydroxide solution was added to obtain a solution of pH 12, and the solution was diluted to 300 mL with water. The solution was stirred at 50°C for 2 hours to perform ester hydrolysis, and geniposidic acid was generated.

After the hydrolysis treatment, 66 g of monosodium glutamate (4.3-fold molar ratio with respect to GP) was added. Ascorbic acid, which is an organic acid, was added in an amount (8-fold molar ratio with respect to GP) that can adjust the solution to a pH of about 4.5, the solution was diluted to 500 g with water, and heat treatment was performed at 80°C for 10 minutes. A control was prepared by the same procedure, except that, instead of ascorbic acid, citric acid was added in an amount (4.3-fold molar ratio with respect to GP) that can adjust the solution to a pH of about 4.5.

Then, under nitrogen atmosphere, 6 g of cellulase was added to the acidic solution, and the solution was stirred gently at 50°C for 24 hours to perform the reaction of β-glucosidase. Consequently, aglycone geniposidic acid was generated. The solution was adjusted again to about pH 4.5, and heat treatment was performed at 80°C for 5 hours so that the reaction with amino group-containing compounds and the oxidative polymerization of iridoid compounds would proceed and red color development would be enhanced. While the red color development in this example is initiated,

with the aglycones generated serving as the substrates, during the β-glucosidase reaction, it proceeds rapidly during the subsequent heat treatment.

Then, the solution was cooled to room temperature, and diatomaceous earth, used as a filter aid, was added at a ratio of 1 part by mass of diatomaceous earth with respect to the mass of the solution and mixed. The mixed solution was subjected to suction filtration using a filter (No. 2 Filter, φ150mm, Advantec Toyo Kaisha, Ltd.) on which a layer of the diatomaceous earth was formed in advance, and the filtrate was collected. The filtrate obtained was subjected to reverse osmosis membrane filtration, and the fraction of molecules having molecular weights (Mv)
smaller than 2500 - 3500 was removed to get rid of the low-molecular compounds added such as organic acids. The remaining liquid collected was concentrated under reduced pressure using an evaporator, the solution was diluted with water and ethanol to adjust the color value ($E^{10\%}{}_{1cm}$) to around 110, heat sterilization was performed at 80°C for 10 minutes, the solution was sieved through 150 mesh, and the liquid obtained was poured into sample bottles in small portions. This way, concentrated solutions of the gardenia red colorant composition was prepared.

The color value ($E^{10\%}{}_{1cm}$) was calculated by measuring the absorbance at the maximum absorption wavelength range of 520 - 545 nm, using pH 5.0 McIlvaine buffer as the solvent.

(2) Evaluation of Color Characteristics

The color characteristics of the solution of the colorant composition prepared above were evaluated. The values measured in (1) above were used as the color value. To obtain the values used for the evaluation of color, a test liquid of color value ($E^{10\%}{}_{1cm}$) = 0.05 was prepared using pH 5.0 McIlvaine buffer, the color of the transmitted light at the measurement wavelength of 380 to 780 nm was measured using a spectrophotometer (V-560, JASCO Corporation; the optical path length of the measurement cell: 1 cm), and the tristimulus values of the Hunter Lab colorimetric system (*L, a,* and *b* values) were measured. Then, using the measured values, brightness, chroma, and hue were evaluated. The *L* value measured above was directly used as the brightness. The chroma value was calculated by Equation 1 above. Hue was evaluated by calculating the hue value. The color difference, that is, the distance of the color of Sample 1-2 (the product of the present invention) from that of Sample 1-1 (comparative product) obtained by the conventional technique was evaluated by calculating the $\Delta E$ value by Equation 2 above.

The results of the measurement and calculation are shown in Table 2, FIGS. 2 and 3. FIG. 4 shows the results of the visual observation of the test bottles.

The results showed that the gardenia red colorant composition (Sample 1-2) obtained by performing the reaction under the presence of ascorbic acid had a higher *L* value, indicating brightness, and a higher chroma value, indicating saturation, compared with the conventional gardenia red colorant composition (Sample 1-1) obtained by performing the reaction under the presence of citric acid. The difference between Sample 1-2 and Sample 1-1 was especially evident in the chroma value, indicating that a gardenia red color having a more vivid and brighter color was obtained in Sample 1-2 (FIG. 2). Moreover, the *a* and *b* values, indicating hue, of Sample 1-2 showed a shift to red and yellow compared with Sample 1-1, and the hue value of Sample 1-2 also indicated redness (FIG. 3). Visual observation of the test bottles also indicated that Sample 1-2 had a bright, vivid red color.

In contrast, the conventional gardenia red colorant composition (Sample 1-1) obtained by performing the reaction under the presence of citric acid had a lower *L* value, or brightness, and a lower chroma value, or saturation, compared with the colorant composition of Sample 1-2. Moreover, the *a* and *b* values, indicating hue, showed a shift to blue and green compared with Sample 1-2, and the hue value also indicated purpleness. Visual observation of the test bottles also indicated that Sample 1-1 had a dull, dark purplish color.

Furthermore, the $\Delta E$ value, that is, the color difference between Samples 1-2 and 1-1, was about 8, indicating a great difference in color between these two samples.

These results show that a gardenia red colorant composition exhibiting a lighter red color with improved brightness and chroma can be produced by performing the series of reactions under the presence of ascorbic acid in the production process of the gardenia red colorant composition. Especially, it is shown that the addition of ascorbic acid improves the chroma and enables the production of a gardenia red colorant composition exhibiting a brighter, more vivid red color. Meanwhile, since the pH in the reaction process for both Samples 1-1 and 1-2 was almost the same (around pH 4.5), it was proved that pH was not a factor responsible for the color-brightening effects of ascorbic acid on gardenia red color.

Table 2

| Gardenia red colorant composition | Sample 1-1 (comparative product) | Sample 1-2 (product of the present invention) |
|---|---|---|
| Organic acid added during the reaction | Citric acid | Ascorbic acid |

(continued)

| Gardenia red colorant composition | Sample 1-1 (comparative product) | Sample 1-2 (product of the present invention) |
|---|---|---|
| Color value | 108.5 | 106.6 |
| Brightness (*L*) | 69.87 | 72.90 |
| *a*<br>*b* | 32.30<br>-9.80 | 39.30<br>-7.85 |
| Chroma | 33.75 | 40.08 |
| Hue | 5.3RP | 6.9RP |
| Color difference ($\Delta E$) | - | 7.87 |
| Visual observation | Dull, dark and strongly bluish red color | Bright and vivid red color |

[Example 2] Study of the Amount of Ascorbic Acid to be Added

The inventors studied the amount of ascorbic acid to be added during the reaction in the production process of the gardenia red colorant composition.

(1) Preparation of Gardenia Red Colorant Composition

The series of reactions using geniposide as the raw material was performed by a method described in Example 1 (1), except that ascorbic acid was added at the amounts shown in Table 3 during the reaction in the production process of the gardenia red colorant composition. This example was conducted at a reaction volume of one-tenth of that in Example 1 (1). After the red color development, gardenia red colorant compositions were obtained by simple purification using a method of Example 1 (1) (that is, the purification process was terminated after the suction filtration using a filter paper described in Example 1 (1)).

(2) Evaluation of Color Characteristics

Color values of the solutions containing the colorant compositions as prepared above were measured. Also, the tristimulus values of the Hunter Lab colorimetric system were measured to evaluate brightness, chroma, and hue. The measurement and evaluation of values related to color characteristics were carried out by a method described in Example 1 (2). The results of the measurement and calculation are shown in Table 3, FIGS. 5 and 6. FIG. 7 shows the results of the visual observation of the test bottles.

The results showed that the samples obtained by performing the reaction under the presence of increased amounts of ascorbic acid had higher *L* values, indicating brightness, and higher chroma values, indicating saturation, showing correlation with the amount of ascorbic acid added. The correlation was especially evident between the amount of ascorbic acid added and the chroma value, and it was shown that the higher the amount of ascorbic acid added, the more vivid and brighter color the gardenia red color exhibited (Table 3 and FIG. 5). Moreover, as the amount of ascorbic acid added increased, the *a* and *b* values, indicating hue, showed a shift to red and yellow, and the hue value also indicated increased redness (FIG. 6). Visual observation of the test bottles also indicated that the red color became brighter and more vivid as the amount of ascorbic acid added increased (FIG. 7).

With regard to the correlation between the amount of ascorbic acid added and the increase in the chroma value, a strongly positive correlation was observed until the amount of ascorbic acid reached 5-fold molar ratio with respect to GP (Sample 2-5), and, even beyond this value, a positive correlation was observed until the amount reached 7-fold molar ratio with respect to GP (Sample 2-7) (Table 3).

These results show that a gardenia red colorant composition exhibiting a lighter red color with improved brightness and chroma can be produced by increasing the amount of ascorbic acid added in the production process of the gardenia red colorant composition. Especially, it is shown that increasing the amount of ascorbic acid added enables the production of a gardenia red colorant composition having improved chroma and exhibiting a brighter, more vivid red color.

Moreover, these results indicate that it is desirable to add ascorbic acid at a molar ratio of 5 or more, preferably 6 or more, and more preferably more than 6 with respect to GP in order to obtain a gardenia red colorant composition

exhibiting a lighter red color with improved brightness and chroma. Also, it is shown that, when a gardenia red colorant composition exhibiting an especially vivid color is desired, it is preferable to add ascorbic acid at a molar ratio of 7 or more relative to GP, at which the positive correlation between the amount of ascorbic acid and the chroma value reaches its maximum.

Meanwhile, since the pH of the sample was readjusted to around 4.5 before initiation of heat treatment for red color development in the same way as in Example 1, it was proved that pH was not a factor responsible for the color-brightening effects of ascorbic acid on gardenia red color.

Table 3

| Gardenia red colorant composition | Sample 2-2 | Sample 2-3 | Sample 2-4 | Sample 2-5 | Sample 2-6 | Sample 2-7 | Sample 2-8 |
|---|---|---|---|---|---|---|---|
| Amount of ascorbic acid added (Molar ratio with respect to GP) | 2eq | 3eq | 4eq | 5eq | 6eq | 7eq | 8eq |
| pH at the time of adding ascorbic acid | 5.31 | 4.92 | 4.82 | 4.59 | 4.55 | 4.51 | 4.58 |
| Color value | 26.52 | 29.84 | 32.84 | 34.03 | 34.57 | 36.53 | 36.83 |
| Brightness (*L*) | 66.91 | 70.63 | 72.27 | 72.93 | 73.29 | 73.11 | 73.35 |
| *a* | 24.54 | 31.14 | 34.48 | 36.56 | 37.71 | 38.32 | 38.33 |
| *b* | -8.65 | -4.94 | -4.39 | -4.46 | -4.61 | -4.86 | -4.16 |
| Chroma | 26.02 | 31.53 | 34.76 | 36.83 | 37.99 | 38.63 | 38.56 |
| Hue | 4.6RP | 7.5RP | 8.0RP | 8.1RP | 8.1RP | 8.0RP | 8.3RP |

[Example 3] Study of the Color-Brightening Effects of Erythorbic Acid

The inventors studied the color-brightening effects of erythorbic acid, a stereoisomer of ascorbic acid, on the gardenia red colorant composition.

(1) Preparation of Gardenia Red Colorant Composition

The series of reactions using geniposide as the raw material was performed by a method described in Example 1 (1), except that organic acids shown in Table 4 were added during the reaction in the production process of the gardenia red colorant composition.

(2) Evaluation of Color Characteristics

Color values of the solutions containing the colorant compositions as prepared above were measured. The measurement of color values was conducted by a method described in Example 1 (1). Also, the tristimulus values of the Hunter Lab colorimetric system were measured to evaluate brightness, chroma, and hue. The measurement and evaluation of values related to color characteristics were carried out by a method described in Example 1 (2). The results of the measurement and calculation are shown in Table 4.

These results showed that a gardenia red colorant composition exhibiting a lighter red color with improved brightness and chroma could be produced by performing the series of reactions under the presence of erythorbic acid in the production process of the gardenia red colorant composition, and the color was yellowish red.

Table 4

| Gardenia red colorant composition | Sample 3-1 (comparative product) | Sample 3-2 (product of the present invention) |
|---|---|---|
| Organic acid added during the reaction | Citric acid | Erythorbic acid |
| Brightness (*L*) | 69.87 | 72.74 |
| *a* | 32.30 | 35.71 |

(continued)

| Gardenia red colorant composition | Sample 3-1 (comparative product) | Sample 3-2 (product of the present invention) |
|---|---|---|
| *b* | -9.80 | 2.25 |
| Chroma | 33.75 | 35.78 |
| Hue | 5.3RP | 1.0R |
| Color difference ($\Delta E$) | - | 12.85 |
| Visual observation | Dull, dark and strongly bluish red color | Bright, vivid and yellowish red color |

[Example 4] Coloring of Milk Beverages

The inventors studied the coloring effects of the gardenia red colorant compositions prepared in the example above on milk beverages, which tend to have a dull color when colored.

(1) Preparation of Milk Beverages

Milk was colored by adding a gardenia red colorant composition at a content of 0.1 mass% when calculated in terms of color value ($E^{10\%}_{1cm}$) 50.5, and milk beverages were prepared (Samples 4-1 and 4-2). As the gardenia red colorant composition, those prepared in Example 1 (1) were used.

(2) Evaluation of Color Characteristics

The tristimulus values of the Hunter Lab colorimetric system of the milk beverages prepared as above were measured to evaluate brightness, chroma, and hue. The results of the measurement and calculation are shown in Table 5, FIGS. 8 and 9. FIG. 10 shows the results of the visual observation of the test bottles.

The results showed that the milk beverage (Sample 4-2) colored by adding the gardenia red colorant composition produced under the presence of ascorbic acid had a higher *L* value, indicating brightness, and a higher chroma value, indicating saturation, compared with the milk beverage (Sample 4-1) colored by adding the conventional gardenia red colorant composition produced under the presence of citric acid. The difference was especially evident in the chroma value (FIG. 8). Moreover, the *a* and *b* values, indicating hue, of Sample 4-2 showed a shift to red and yellow compared with Sample 4-1, and the hue value also indicated redness (FIG. 9). Visual observation of the test bottles also indicated that Sample 4-2 exhibited a milky white color tinted with bright red with less dullness (FIG. 10).

In contrast, the milk beverage (Sample 4-1) colored by adding the conventional gardenia red colorant composition produced under the presence of citric acid had a lower *L* value, or brightness, and a lower chroma value, or saturation, compared with the milk beverage of Sample 4-2. Moreover, the *a* and *b* values, indicating hue, showed a shift to blue and green compared with Sample 4-2, and the hue value also indicated purpleness. Visual observation of the test bottle also indicated that Sample 4-1 had a dull, dark, purplish milky white color.

These results show that a milk beverage exhibiting a lighter color with improved chroma can be produced when the beverage is colored by adding the gardenia red colorant composition produced under the presence of ascorbic acid. Especially, it is shown that a milk beverage exhibiting a milky white color tinted with bright red with less dullness can be produced.

Table 5

| Milk beverages | Sample 4-1 (comparative product) | Sample 4-2 (product of the present invention) |
|---|---|---|
| Gardenia red colorant composition | Produced under the presence of citric acid | Produced under the presence of ascorbic acid |
| Brightness (*L*) | 66.89 | 67.81 |
| *a*<br>*b* | 8.07<br>-4.97 | 10.72<br>-4.11 |
| Chroma | 9.48 | 11.48 |

(continued)

| Milk beverages | Sample 4-1 (comparative product) | Sample 4-2 (product of the present invention) |
|---|---|---|
| Hue | 1.2RP | 4.2RP |
| Color difference ($\Delta E$) | - | 2.93 |
| Visual observation | Tinted with dark, dull and bluish red | Tinted with bright red with less dullness |

[Example 5] Coloring of Retort-Sterilized Processed Fish Meat Products

The inventors studied the coloring effects on processed fish meat products, which tend to have a dull color when colored and which undergo retort sterilization, using the gardenia red colorant compositions prepared in the example above.

(1) Preparation of Processed Fish Meat Products

Fish paste was colored by mixing and kneading frozen fish paste (60 g), salt (3 g), vegetable oil (8.3 g), potato starch (10 g), a moisture-retention and elasticity-increasing agent for fish paste products (S-PROGEN** K169*) (1.5 g), and the gardenia red colorant composition prepared in Example 1 (1). The content of the gardenia red colorant composition was adjusted to 0.03 mass% when calculated in terms of color value ($E^{10\%}{}_{1cm}$) 110.

This mixture was sealed into a transparent retort pouch and heated at 121°C for 15 minutes for retort sterilization, and processed fish meat products were prepared (Samples 5-1 and 5-2).

(2) Evaluation of Color Characteristics

The color characteristics of the processed fish meat products prepared as above were evaluated by visual observation. The results are shown in Table 6 and FIG. 11.

The results showed that the retort-sterilized processed fish meat product (Sample 5-2) colored by adding the gardenia red colorant composition produced under the presence of ascorbic acid exhibited a brighter color with less dullness and had a more reddish color compared with the retort-sterilized processed fish meat product (Sample 5-1) colored by adding the conventional gardenia red colorant composition produced under the presence of citric acid (FIG. 11). Here, the improvement of the color brightness is an effect confirmed even after the retort sterilization.

In contrast, it was confirmed that the processed fish meat product (Sample 5-1) colored by adding the conventional gardenia red colorant composition produced under the presence of citric acid had a dull, dark purplish, milky white color.

These results show that a processed fish meat product exhibiting a milky white color tinted with bright red with less dullness can be produced when the milk beverage is colored by adding the gardenia red colorant composition produced under the presence of ascorbic acid. Moreover, it is shown that the color-brightening effects are not diminished even after retort sterilization.

Table 6

| Processed fish meat products | Gardenia red colorant composition | Visual observation after retort sterilization |
|---|---|---|
| Control | Not colored | White |
| Sample 5-1 (comparative product) | Produced under the presence of citric acid | Tinted with dark, dull and bluish red color |
| Sample 5-2 (product of the present invention) | Produced under the presence of ascorbic acid | Tinted with bright red with less dullness |

[Example 6] Evaluation of Resistance against Light and Heat

Evaluation of the stability of the colorant compounds against light and heat was conducted on the gardenia red colorant composition prepared in the example above.

(1) Preparation of the Test Liquids

An acid-sugar solution that contained the gardenia red colorant composition at a content of 0.05 mass% when calculated in terms of color value ($E^{10\%}_{1cm}$) 50.5, was 10° Brix (adjusted with 13.3 mass% of 75° Brix high fructose corn syrup) and had pH 5.0 (adjusted with citric acid anhydrous and trisodium citrate dihydrate) was prepared. As the gardenia red colorant composition, those prepared in Example 1 (1) were used.

The acid-sugar solution obtained was heated up to 93°C for sterilization and hot packed into 200 mL plastic bottles. The plastic bottle beverages obtained were used as the test liquids.

(2) Light and Heat Resistance Tests

The test liquids in plastic bottles were irradiated with white fluorescent light of 10,000 lux at 10°C for 10 days in a fluorescent light irradiation device to conduct a light resistance test. A CLH-301 cultivation chamber (Tomy Seiko Co., Ltd.) was used as the fluorescent light irradiation device. To conduct a heat resistance test, the test liquids in plastic bottles were stored in a dark place under a high temperature of 50°C for 10 days.

(3) Calculation of the Color Residual Rate

Color of the transmitted light at the measurement wavelength of 380 to 780 nm was measured for the beverages before and after the aforementioned stability tests using a spectrophotometer (V-560, JASCO Corporation; the optical path length of the measurement cell: 1 cm), and the color residual rates were calculated by Equation 4 described above. The results of the light resistance test are shown in Table 7. The results of the heat resistance test are shown in Table 8.

The results showed that the test liquids (Sample 6-2) containing the gardenia red colorant composition produced under the presence of ascorbic acid had color residual rates at the same or similar level compared to the test liquids (Sample 6-1) containing the conventional gardenia red colorant composition produced under the presence of citric acid, even when they were exposed to severe light or heat conditions for a long period of time.

Therefore, it is confirmed that the gardenia red colorant compounds produced under the presence of ascorbic acid are colorant compounds having high stability against light and heat. That is, it is confirmed that the gardenia red colorant composition produced under the presence of ascorbic acid can serve as a pigment preparation having light and heat resistance that can be used for commercial products such as foods and beverages.

Table 7

| Gardenia red colorant composition | | Color residual rate (%) after light resistance test | |
|---|---|---|---|
| | | 10,000 lux for 5 days | 10,000 lux for 10 days |
| Sample 6-1 (comparative product) | Produced under the presence of citric acid | 86.2 | 74.9 |
| Sample 6-2 (product of the present invention) | Produced under the presence of ascorbic acid | 78.4 | 69.2 |

Table 8

| Gardenia red colorant composition | | Color residual rate (%) after heat resistance test | |
|---|---|---|---|
| | | 50°C for 5 days | 50°C for 10 days |
| Sample 6-1 (comparative product) | Produced under the presence of citric acid | 81.9 | 77.7 |
| Sample 6-2 (product of the present invention) | Produced under the presence of ascorbic acid | 76.8 | 71.1 |

[Example 7] Evaluation of Acid Resistance

The acid resistance of the gardenia red colorant compositions prepared in the example above was evaluated by measuring their color residual rates under acidic conditions.

(1) Preparation of Test Liquids

Test liquids that contained different gardenia red colorant compositions at a content of 0.05 mass% when calculated in terms of color value ($E^{10\%}{}_{1cm}$) 50.5 were prepared using the McIlvaine buffers of different pH levels shown in Table 9. As the gardenia red colorant composition produced under the presence of citric acid and the gardenia red colorant composition produced under the presence of ascorbic acid, those prepared in Example 1 (1) were used. Also, a conventional gardenia red colorant composition produced under the presence of taurine, which was reported to have improved acid resistance, was prepared by a method described in Japanese Patent No. 4605824.

(2) Acid Resistance Test

The adjustment of pH to acidic conditions described in (1) above served as the acid resistance test.

(3) Calculation of Color Residual Rates

The test liquids of different pH levels were filtered through the membrane filter (0.45 μm) to remove the aggregated or precipitated colorants, the filtrates collected were subjected to spectrophotometric analysis, and the color residual rates were calculated. Test liquids of pH 4.0 were prepared and filtered through the membrane filter (0.45 μm), the filtrates collected were used as the pre-examination samples, and the color residual rates were calculated by a method described in Example 6 (3). The results are shown in Table 9 and FIG. 12.

The results showed that the conventional gardenia red colorant composition (Sample 7-1) produced under the presence of citric acid had high color residual rates of about 94 to 100 % under weak acidic conditions of pH 3 to 4, but the color residual rate rapidly dropped to about 30 % at pH 2.5 and about 25 % at pH 2.2. Accordingly, it is shown that the gardenia red color is a colorant compound having low stability in an acidic solution of less than pH 3.

In contrast, the gardenia red colorant composition (Sample 7-2) produced under the presence of ascorbic acid had high color residual rates of about 95 to 100 % under weak acidic conditions of pH 3 to 4, and moreover, about 80 % of the colorants were retained at pH 2.5, and about 70 % of the colorants were retained at pH 2.2. These values of the color residual rate were significantly higher than the conventional gardenia red colorant composition (Sample 7-3) produced under the presence of taurine, which was reported to have improved acid resistance.

The difference was especially remarkable in strongly acidic solutions. In the strongly acidic solution of pH 2.2, the color residual rate drastically fell to about 30 % for the gardenia red colorant composition (Sample 7-3) produced under the presence of taurine, while, in contrast, about 70 % of the colorants were retained for the gardenia red colorant composition (Sample 7-2) produced under the presence of ascorbic acid.

These results show that a gardenia colorant composition having high stability in an acidic solution can be obtained by performing the reaction under the presence of ascorbic acid in the production process of the gardenia red colorant composition. That is, it is shown that a gardenia colorant composition which maintains water solubility and hardly forms aggregates in an acidic solution can be produced.

The function of ascorbic acid to impart acid resistance is especially excellent for stabilization of the gardenia red color at less than pH 3, which could not have been achieved by conventional techniques.

Table 9

| Gardenia red colorant composition | | Color residual rate (%) in acid resistance test (immediately after production) | | | |
|---|---|---|---|---|---|
| | | pH 2.2 | pH 2.5 | pH 3.0 | pH 4.0 |
| Sample 7-1 (comparative product) | Produced under the presence of citric acid | 24.7 | 33.8 | 94.0 | 100.0 |
| Sample 7-3 (comparative product) | Produced under the presence of taurine | 32.1 | 70.4 | 97.4 | 100.0 |

(continued)

| Gardenia red colorant composition | | Color residual rate (%) in acid resistance test (immediately after production) | | | |
|---|---|---|---|---|---|
| | | pH 2.2 | pH 2.5 | pH 3.0 | pH 4.0 |
| Sample 7-2 (product of the present invention) | Produced under the presence of ascorbic acid | 68.2 | 80.5 | 94.9 | 100.0 |

[Example 8] Evaluation of Acid Resistance: Low Temperature Storage

The stability in color residual rates after low temperature storage under acidic conditions was evaluated for the gardenia red colorant compositions produced in the example above.

(1) Preparation of Test Liquids

Test liquids containing different gardenia red colorant compositions at a content of 0.05 mass% when calculated in terms of color value ($E^{10\%}_{1cm}$) 50.5 were prepared using the McIlvaine buffers of different pH levels shown in Table 10. The gardenia colorant compositions prepared in Example 7 (1) were used.

(2) Acid Resistance Test

The test liquids were stored in a refrigerator set to 10°C for one week.

(3) Calculation of Color Residual Rates

The test liquids after low temperature storage were filtered through the membrane filter (0.45 μm) to remove the aggregated colorants, the filtrates collected were subjected to spectrophotometric analysis, and the color residual rates were calculated. Test liquids of pH 4.0 were prepared and filtered through the membrane filter (0.45 μm), the filtrates collected were used as the pre-examination samples, and the color residual rates were calculated by a method described in Example 6 (3). The results are shown in Table 10 and FIG. 13.

The results showed that, when the conventional gardenia red colorant composition (Sample 8-1) obtained under the presence of citric acid was stored under low temperature, the color residual rate rapidly dropped to about 24 % at pH 2.5 and to as low as about 16 % at pH 2.2. Accordingly, it is shown that the gardenia red color is a colorant compound whose stability tends to be lost due to low temperature storage at a pH of less than 3.

In contrast, in the gardenia red colorant composition (Sample 8-2) produced under the presence of ascorbic acid, about 56 % of the colorants were retained at pH 2.5, and about 43 % of the colorants were retained even at pH 2.2. These values of the color residual rate were significantly higher than the conventional gardenia red colorant composition (Sample 8-3) produced under the presence of taurine, which was reported to have improved acid resistance. The difference was especially remarkable in strongly acidic solutions. In the strongly acidic solution of pH 2.2, the color residual rate drastically fell to about 20 % for the gardenia red colorant composition (Sample 8-3) produced under the presence of taurine, while, in contrast, more than 40 % of the colorants were retained for the gardenia red colorant composition (Sample 8-2) produced under the presence of ascorbic acid.

These results show that, though the stability of the gardenia red color tends to be lost due to low temperature storage, stability under low temperature storage can be maintained by performing the reaction under the presence of ascorbic acid in the production process of the gardenia red colorant composition. That is, it is shown that a gardenia colorant composition which maintains water solubility and hardly forms aggregates in an acidic solution under low temperature conditions can be produced.

The function of ascorbic acid to impart acid resistance is especially excellent for stabilization of the colorant at less than pH 3, which could not have been achieved by conventional techniques.

Table 10

| Gardenia red colorant composition | | Color residual rate (%) in acid resistance test (after storage at 10°C for a week) | |
|---|---|---|---|
| | | pH 2.2 | pH 2.5 |
| Sample 8-1 (comparative product) | Produced under the presence of citric acid | 15.9 | 24.0 |
| Sample 8-3 (comparative product) | Produced under the presence of taurine | 21.3 | 38.4 |
| Sample 8-2 (product of the present invention) | Produced under the presence of ascorbic acid | 42.4 | 55.5 |

[Example 9] Color Measurement of Transmitted Light

The evaluation of color brightness of the colorant composition was conducted by the color measurement of transmitted light for the gardenia red colorant compositions obtained in the example above.

(1) Preparation of Test Liquids

Test liquids containing different gardenia red colorant compositions were adjusted to color value ($E^{10\%}_{1cm}$) 0.05 using a McIlvaine buffer of pH 5. As the gardenia colorant compositions, those prepared in Example 7 (1) were used. The gardenia red colorant composition obtained under the presence of taurine was also subjected to analysis as a comparative sample, as Japanese Patent No. 4605824 reported on its color brightness.

(2) Color Measurement of Transmitted Light

Color of the transmitted light at the measurement wavelength of 380 to 780 nm was measured for the test liquids as prepared above using a spectrophotometer (V-560, JASCO Corporation; the optical path length of the measurement cell: 1 cm). Based on the values in the absorption spectra obtained, $A_{\lambda max}$ / $A_{600nm}$ and $A_{\lambda max}$ / $A_{430nm}$ were calculated. The measured and calculated values are shown in Tables 11 and 12. FIG. 14 shows the absorption spectra.

The results showed that the ratio of the absorbance at the maximum absorption wavelength to the absorbance at each fixed wavelength for the gardenia red colorant composition (Sample 9-2) obtained under the presence of ascorbic acid was higher than those of the gardenia red colorant composition (Sample 9-1) obtained under the presence of citric acid and the gardenia red colorant composition (Sample 9-3) obtained under the presence of taurine. The difference was especially evident in $A_{\lambda max}$ / $A_{600nm}$, which was the ratio relative to the absorbance at the wavelength of 600 nm. The $A_{\lambda max}$ / $A_{600nm}$ value of the gardenia red colorant composition (Sample 9-2) obtained under the presence of ascorbic acid was about two times higher than that of the gardenia red colorant composition (Sample 9-1) obtained under the presence of citric acid.

Accordingly, it is shown that a gardenia red colorant composition having a bright, less yellowish color with less dullness can be obtained by performing the reaction under the presence of ascorbic acid in the production process of the gardenia red colorant composition.

Table 11

| Gardenia red colorant composition | | Absorbance ratio (relative to the absorbance at 600 nm) | | | |
|---|---|---|---|---|---|
| | | $\lambda max$ | $A_{\lambda max}$ | $A_{600nm}$ | $A_{\lambda max}$ / $A_{600nm}$ |
| Sample 9-1 (comparative product) | Produced under the presence of citric acid | 533 | 0.4999 | 0.1768 | 2.8275 |
| Sample 9-3 (comparative product) | Produced under the presence of taurine | 531 | 0.4996 | 0.1178 | 4.2411 |
| Sample 9-2 (product of the present invention) | Produced under the presence of ascorbic acid | 536 | 0.4996 | 0.0899 | 5.5573 |

Table 12

| Gardenia red colorant composition | | Absorbance ratio (relative to the absorbance at 430 nm) | | | |
|---|---|---|---|---|---|
| | | $\lambda max$ | $A_{\lambda max}$ | $A_{430nm}$ | $A_{\lambda max}$ / $A_{430nm}$ |
| Sample 9-1 (comparative product) | Produced under the presence of citric acid | 533 | 0.4999 | 0.1868 | 2.6761 |
| Sample 9-3 (comparative product) | Produced under the presence of taurine | 531 | 0.4996 | 0.2095 | 2.3847 |
| Sample 9-2 (product of the present invention) | Produced under the presence of ascorbic acid | 536 | 0.4996 | 0.1690 | 2.9562 |

[Example 10] Coloring of Pudding

The inventors studied the coloring effects on pudding, which tends to have a dull color when colored, using the gardenia red colorant compositions prepared in the example above.

(1) Preparation of Pudding

Puddings were prepared following the formula shown in Table 13. While the mixture of water, milk, sweetened condensed skim milk, and coconut oil was being stirred, the mixed powder of sugar, skimmed milk powder, gelling agent, and emulsifier was added thereto, and the mixture obtained was stirred at 80°C for 10 minutes. It was then subjected to homogenization treatment (150 kg/$cm^2$). After a gardenia red colorant composition was added, the mixture was filled into a container and refrigerated until set. In this way, the pudding samples were prepared (Samples 10-1 and 10-2). As the gardenia red colorant composition, either the gardenia red colorant composition prepared in Example 1 (1) or the gardenia red colorant composition used in Example 7, which was obtained under the presence of taurine, was added at a content of 0.035 mass % when calculated in terms of color value ($E^{10\%}_{1cm}$) 100.

Table 13

| Formula of pudding | mass % |
|---|---|
| Milk | 10 |
| Sweetened condensed skim milk | 2 |
| Coconut oil | 4 |
| Sugar | 10 |
| Skimmed milk powder | 5 |
| Gelling agent (GELUP** PI-800) | 0.5 |
| Emulsifier (HOMOGEN** DM-S) | 0.1 |
| Gardenia red colorant composition (calculated in terms of color value 100) | 0.035 |
| Total amount adjusted with water | 100 |

(2) Evaluation of Color Characteristics

The tristimulus values of the Hunter Lab colorimetric system of the puddings as prepared above were measured to evaluate brightness, chroma, and hue. The results of the measurement and calculation are shown in Table 14, FIGS. 15 and 16. FIG. 17 shows the results of the visual observation of the puddings.

The results showed that the pudding (Sample 10-2) colored by adding the gardenia red colorant composition produced under the presence of ascorbic acid had a higher chroma value, indicating saturation, than the pudding (Sample 10-1) colored by adding the conventional gardenia red colorant composition produced under the presence of taurine (FIG. 15). Moreover, the a value, indicating hue, of Sample 10-2 showed a shift to red compared with Sample 10-1, and

the hue value also indicated redness (FIG. 16). Visual observation also indicated that Sample 10-2 exhibited a milky white color tinted with bright red with less dullness (FIG. 17).

In contrast, the pudding (Sample 10-1) colored by adding the conventional gardenia red colorant composition produced under the presence of taurine had a lower chroma value, or saturation, compared with the pudding of Sample 10-2. Moreover, the a value, indicating hue, showed a shift to green compared with Sample 10-2, and the hue value also indicated purpleness. Visual observation also indicated that Sample 10-1 had a dull, dark reddish purple, milky white color.

These results show that a pudding exhibiting a lighter color with improved chroma can be produced when the pudding is colored by adding the gardenia red colorant composition produced under the presence of ascorbic acid. Especially, it is shown that a pudding exhibiting a milky white color tinted with bright red with less dullness can be produced.

Table 14

| Pudding | Sample 10-1 (comparative product) | Sample 10-2 (product of the present invention) |
|---|---|---|
| Gardenia red colorant composition | Produced under the presence of taurine | Produced under the presence of ascorbic acid |
| Brightness (*L*) | 70.45 | 68.85 |
| *a*<br>*b* | 10.16<br>-1.78 | 12.97<br>-1.75 |
| Chroma | 10.31 | 13.09 |
| Hue | 7.2RP | 7.9RP |
| Color difference ($\Delta E$) | - | 3.23 |
| Visual observation | Tinted with dull, dark reddish purple | Tinted with bright red wit h less dullness |

[Example 11] Coloring of Bread

The inventors studied the coloring effects on bread using the gardenia red colorant compositions prepared in the example above.

(1) Preparation of Bread

A white bread dough was colored by adding a gardenia red colorant composition at a content of 0.2 mass % when calculated in terms of color value ($E^{10\%}{}_{1cm}$) 100, and the bread samples were prepared (Samples 11-1 and 11-2). As the gardenia red colorant composition, the gardenia red colorant composition prepared in Example 1 (1) and the gardenia red colorant composition used in Example 7 (obtained under the presence of taurine) were used.

(2) Evaluation of Color Characteristics

The tristimulus values of the Hunter Lab colorimetric system of the bread as prepared above were measured to evaluate brightness, chroma, and hue. The results of the measurement and calculation are shown in Table 15, FIGS. 18 and 19. FIG. 20 shows the results of the visual observation of the bread.

The results show that the bread (Sample 11-2) colored by adding the gardenia red colorant composition produced under the presence of ascorbic acid had a higher *L* value, indicating brightness, and a higher chroma value, indicating saturation, than the bread (Sample 11-1) colored by adding the conventional gardenia red colorant composition produced under the presence of taurine, and significant difference between these two samples was observed (FIG. 18). Moreover, the *a* value, indicating hue, of Sample 11-2 showed a shift to red compared with Sample 11-1, and the hue value of Sample 11-2 also indicated redness (FIG. 19). Visual observation also indicated that Sample 11-2 exhibited a color tinted with bright, clear red (FIG. 20).

In contrast, the bread (Sample 11-1) colored by adding the conventional gardenia red colorant composition produced under the presence of taurine had a lower *L* value, or brightness, and a lower chroma value, or saturation, compared with the bread of Sample 11-2. Moreover, the a value, indicating hue, showed a shift to green compared with Sample 11-2, and the hue value also indicated purpleness .

Visual observation also indicated that Sample 11-1 had a dull, dark red color.

These results show that bread exhibiting a color tinted with bright, clear red can be produced when the bread dough is colored by adding the gardenia red colorant composition produced under the presence of ascorbic acid.

Table 15

| Bread | Sample 11-1 (comparative product) | Sample 11-2 (product of the present invention) |
|---|---|---|
| Gardenia red colorant composition | Produced under the presence of taurine | Produced under the presence of ascorbic acid |
| Brightness (*L*) | 49.47 | 53.48 |
| *a*<br>*b* | 10.10<br>2.13 | 13.25<br>2.41 |
| Chroma | 10.32 | 13.47 |
| Hue | 3.3R | 2.9R |
| Color difference ($\Delta E$) | - | 5.11 |
| Visual observation | Tinted with dull, dark red | Tinted with bright, clear red |

[Example 12] Coloring of Chewing Gum

The inventors studied the coloring effects on chewing gums using the gardenia red colorant compositions prepared in the example above.

(1) Preparation of Chewing Gum

A gardenia red colorant composition was added to a gum base for stick gum at a content of 0.2 mass % when calculated in terms of color value ($E^{10\%}_{1cm}$) 100. In this way, the chewing gum samples were prepared (Samples 12-1 and 12-2). As the gardenia red colorant composition, the gardenia red colorant composition prepared in Example 1 (1) and the gardenia red colorant composition used in Example 7 (obtained under the presence of taurine) were used.

(2) Evaluation of Color Characteristics

The color characteristics of the chewing gum samples as prepared above were evaluated by visual observation. The results are shown in Table 16 and FIG. 21.

The results showed that the chewing gum (Sample 12-2) colored by adding the gardenia red colorant composition produced under the presence of ascorbic acid had a brighter, clearer and more reddish color than the chewing gum (Sample 12-1) colored by adding the conventional gardenia red colorant composition produced under the presence of taurine.

These results show that a chewing gum exhibiting a color tinted with bright, clear red can be produced when the chewing gum is colored by adding the gardenia red colorant composition produced under the presence of ascorbic acid.

Table 16

| Chewing gum | Sample 12-1 (comparative product) | Sample 12-2 (product of the present invention) |
|---|---|---|
| Gardenia red colorant composition | Produced under the presence of taurine | Produced under the presence of ascorbic acid |
| Visual observation | Tinted with dull, dark, and slightly bluish red | Tinted with bright, clear red |

[Example 13] Coloring of Pickled Vegetables

The inventors studied the coloring effects on pickled vegetables using the gardenia red colorant compositions prepared in the example above.

(1) Preparation of Pickled Vegetables

The ingredients were mixed following the formula shown in Table 17, and the liquid seasoning was prepared. The liquid seasoning and the material to be pickled (cut cucumbers) were provided at a mass ratio of 1:1, the material to be pickled was soaked in the liquid seasoning for a week, and colored pickles were prepared (Samples 13-1 and 13-2). As the red colorant composition, the gardenia red colorant composition prepared in Example 1 (1), the gardenia red colorant composition used in Example 7 (obtained under the presence of taurine), and the red cabbage color, which is a red anthocyanin pigment composition, were used.

Table 17

| Formula of liquid seasoning | mass % |
|---|---|
| Brewed vinegar | 6.4 |
| Salt | 6.3 |
| Monosodium glutamate | 1.4 |
| Sorbitol | 0.6 |
| Seasoning (SANLIKE** AMINO BASE NA) | 0.4 |
| Shelf-life extender (ARTFRESH** 101) | 0.4 |
| Citric acid (anhydrous) | 0.5 |
| Red colorant composition (calculated in terms of color value 100) | 0.8 |
| Total amount adjusted with water | 100 |

(2) Evaluation of Color Characteristics

The color characteristics of the pickles as prepared above were evaluated by visual observation. The color characteristics of pickles immediately after production and those after being subjected to light irradiation (fluorescent light of 10,000 lux for one day) were evaluated. The results are shown in Table 18, FIGS. 22 and 23.

The results showed that the pickles (Sample 13-2) colored by adding the gardenia red colorant composition produced under the presence of ascorbic acid were tinted with a brighter, clearer and more reddish color than the pickles (Sample 13-1) colored by adding the conventional gardenia red colorant composition produced under the presence of taurine (FIG. 22). Moreover, the inside of the cucumber was colored unevenly in the pickles (Sample 13-1) colored by adding the gardenia red colorant composition produced under the presence of taurine, while, in contrast, the pickles (Sample 13-2) colored by adding the gardenia red colorant composition produced under the presence of ascorbic acid were colored evenly, with no uneven coloration, and even the inside of the cucumber was colored with bright, clear red (FIG. 22).

Here, the inside of the cucumber could also be colored sufficiently in the pickles (Sample 13-3) colored by adding the red cabbage color, which is a red anthocyanin pigment. However, in the pickles of Sample 13-3 colored by anthocyanin pigments, the color faded drastically after light irradiation, and the red color was lost after light irradiation for one day (FIG. 23). In contrast, in the pickles (Sample 13-2) colored by adding the gardenia red colorant composition produced under the presence of ascorbic acid, the red color was maintained well even after light irradiation for one day (FIG. 23).

These results show that, when pickled vegetables are colored by adding the gardenia red colorant composition produced under the presence of ascorbic acid, the pickled vegetables can be colored with bright, clear red, even the inside of the vegetable can be colored uniformly, with no uneven coloration, and the excellent function to suppress color fading due to light irradiation can be achieved.

Table 18

| Pickled vegetables | Sample 13-1 (comparative product) | Sample 13-2 (product of the present invention) | Sample 13-3 (comparative product) |
|---|---|---|---|
| Red colorant composition | Gardenia red color (produced under the presence of taurine) | Gardenia red color (produced under the presence of ascorbic acid) | Red cabbage color |

(continued)

| Pickled vegetables | Sample 13-1 (comparative product) | Sample 13-2 (product of the present invention) | Sample 13-3 (comparative product) |
|---|---|---|---|
| Immediately after production | Tinted with dark, dull red. Colored unevenly. | Tinted with bright, clear red. Even the inside is colored uniformly. | Tinted with bright, clear red. Even the inside is colored uniformly. |
| After light irradiation | Color fades slightly, but the red color is maintained well. | Color fades slightly, but the red color is maintained well. | Color fades drastically, and the red color is lost. |

[Example 14] Coloring of Hams

The inventors studied the coloring effects on hams using the gardenia red colorant compositions prepared in the example above.

(1) Preparation of Hams

The pickling liquid for coloring the hams was prepared following the formula show in Table 19.

Hydrogenated maltose starch syrup, dried egg white, powdered soybean protein, whey protein, sodium caseinate, gelling agent, polyphosphate, L-sodium ascorbate, and sodium nitrite were added to water and stirred by a mixer for 30 minutes. After salt, seasoning, colorant, and the rest of the water were added to the stirred liquid, the liquid was stirred again for 10 minutes and cooled to obtain the pickling liquid.

The pickling liquid was injected into pork butt so that the total meat weight would be 150% of the original meat weight, and after tumbling, the meat was brined overnight. After brining, the meat was stuffed into fibrous casings and heated (dried at 60°C for 50 minutes, smoked at 70°C for 60 minutes, and steamed at 78°C for 120 minutes), and colored hams were obtained (Samples 14-1 and 14-2). The gardenia red colorant compositions used in this example are the gardenia red colorant composition prepared in Example 1 (1) and a gardenia red colorant composition having a color value ($E^{10\%}_{1cm}$) of about 100 produced by a method described in Example 1 (1) except that the ascorbic acid of 8-fold molar ratio with respect to GP was replaced by citric acid of 4-fold molar ratio with respect to GP.

Table 19

| Formula of pickling liquid | mass % |
|---|---|
| Hydrogenated maltose starch syrup | 8 |
| Dried egg white | 4 |
| Powdered soybean protein | 3 |
| Whey protein (MIL PRO** WG-900*) | 1 |
| Sodium caseinate | 1 |
| Gelling agent (HM-150*) | 0.8 |
| Polyphosphate | 0.8 |
| L-sodium ascorbate (crystal) | 0.2 |
| Sodium nitrite | 0.027 |
| Salt | 4.3 |
| Seasoning (SANLIKE** AMINO BASE NAG*) | 0.3 |
| Gardenia red colorant composition | An amount that can adjust the color value ($E^{10\%}_{1cm}$) of the pickling liquid to 0.067 |
| Total amount adjusted with water | 100 |

(2) Evaluation of Color Characteristics

The hams as prepared above were cut, and visual observation of the cross sections was conducted. The results

are shown in Table 20.

As a result, the ham (Sample 14-2) colored by adding the gardenia red colorant composition produced under the presence of ascorbic acid was tinted with a brighter, clearer and more reddish color than the ham (Sample 14-1) colored by adding the conventional gardenia red colorant composition produced under the presence of citric acid. In the ham (Sample 14-1) colored by adding the conventional gardenia red colorant composition produced under the presence of citric acid, pink streaks were observed on the cross section, and the central part of the ham could not be colored uniformly. In contrast, in the ham (Sample 14-2) colored by adding the gardenia red colorant composition produced under the presence of ascorbic acid, streaks or patches due to uneven coloration were not observed, and even the central part of the ham was colored uniformly (Table 20).

These results show that, when livestock meat products are colored by adding the gardenia red colorant composition produced under the presence of ascorbic acid, the livestock meat products can be tinged with bright clear red, and even the inside of the livestock meat products can be colored uniformly, with no uneven coloration.

Table 20

| Ham | Sample 14-1 (comparative product) | Sample 14-2 (product of the present invention) |
|---|---|---|
| Gardenia red colorant composition | Produced under the presence of citric acid | Produced under the presence of ascorbic acid |
| Visual observation | Streaks due to uneven coloration observed. | Colored uniformly with bright, clear red, with no streaks or patches observed. |

[Example 15] Evaluation of Acid Resistance

The inventors evaluated the acid resistance of the gardenia red colorant compositions produced with different amounts of ascorbic acid added during the reaction in the production process of the gardenia red colorant composition.

(1) Preparation of the Gardenia Red Colorant Composition

The series of reactions using geniposide as the raw material was performed by a method described in Example 1 (1), except that ascorbic acid of 6.5-fold molar ratio with respect to GP was added during the reaction in the production process of the gardenia red colorant composition.

(2) Preparation of the Test Liquid

Test liquids that contained the gardenia red colorant composition at a content of 0.05 mass% when calculated in terms of color value ($E^{10\%}{}_{1cm}$) 50.5 were prepared using the McIlvaine buffers of different pH levels shown in the table below.

(3) Acid Resistance Test

The adjustment of pH to acidic conditions described in (1) above served as the acid resistance test. Also, as the acid resistance test under low temperature storage, the test liquids were stored in a refrigerator set to 10°C for one week.

(4) Calculation of Color Residual Rates

The test liquids were filtered through the membrane filter (0.45 μm) to remove the aggregated colorants, the filtrates collected were subjected to spectrophotometric analysis, and the color residual rates were calculated. The test liquid of pH 4.0 was filtered through the membrane filter (0.45 μm), the filtrate collected was used as the pre-examination sample, and the color residual rate was calculated by a method described in Example 6 (3).

The color residual rates obtained were compared with those calculated in the acid resistance tests in Examples 7 and 8. Table 21 and FIG. 24 show the results for the test liquids immediately after production. Table 22 and FIG. 25 show the results for the test liquids after low temperature storage.

As a result, in the gardenia red colorant composition immediately after production (Sample 15-1) which was produced by adding ascorbic acid of 6.5-fold molar ratio with respect to GP, about 41% of the colorants were retained under a strongly acidic condition of pH 2.2 (Table 21 and FIG. 24). Even after this gardenia red colorant composition

was stored under low temperature (Sample 15-2), about 35% of the colorants were retained under a strongly acidic condition of pH 2.2 (Table 22 and FIG. 25).

Though these color residual rates were lower than those of the gardenia red colorant compositions (Samples 7-2 and 8-2) produced by adding ascorbic acid of 8.0-fold molar ratio with respect to GP, they were higher than those of the conventional gardenia red colorant compositions (Samples 7-3 and 8-3) produced under the presence of taurine, which was reported to have functions to improve acid resistance.

These results show that, even when the red color development is performed by adding an increased, but not excessive, amount of ascorbic acid in the production process of the gardenia red colorant composition, a gardenia red colorant composition which sufficiently maintains water solubility and hardly forms aggregates even in an acidic solution can be produced. Especially, the function of ascorbic acid to impart acid resistance brings about stabilization of the colorant at less than pH 3, which could not have been achieved by conventional techniques.

Table 21

| Gardenia red colorant composition | | Color residual rate (%) in acid resistance test (immediately after production) | | |
|---|---|---|---|---|
| | | pH 2.2 | pH 3.0 | pH 4.0 |
| Sample 7-1 (comparative product) | Produced under the presence of citric acid | 24.7 | 94.0 | 100.0 |
| Sample 7-3 (comparative product) | Produced under the presence of taurine | 32.1 | 97.4 | 100.0 |
| Sample 15-1 (product of the present invention) | Produced under the presence of ascorbic acid (6.5-fold molar ratio with respect to GP) | 41.4 | 95.6 | 100.0 |
| Sample 7-2 (product of the present invention) | Produced under the presence of ascorbic acid (8-fold molar ratio with respect to GP) | 68.2 | 94.9 | 100.0 |

Table 22

| Gardenia red colorant composition | | Color residual rate (%) in acid resistance test (after storage at 10°C for a week) | |
|---|---|---|---|
| | | pH 2.2 | pH 2.5 |
| Sample 8-1 (comparative product) | Produced under the presence of citric acid | 15.9 | 24.0 |
| Sample 8-3 (comparative product) | Produced under the presence of taurine | 21.3 | 38.4 |
| Sample 15-2 (product of the present invention) | Produced under the presence of ascorbic acid (6.5-fold molar ratio with respect to GP) | 34.6 | 45.6 |
| Sample 8-2 (product of the present invention) | Produced under the presence of ascorbic acid (8-fold molar ratio wit h respect to GP) | 42.4 | 55.5 |

List of Reference Numerals

1-1: Sample 1-1

1-2: Sample 1-2

2-2: Sample 2-2

2-5: Sample 2-5

4-1: Sample 4-1

4-2: Sample 4-2

91: 430 nm

92: 600nm

10-1: Sample 10-1

10-2: Sample 10-2

11-1: Sample 11-1

11-2: Sample 11-2

## Claims

1. A method for producing a red colorant composition derived from iridoid compounds, comprising the following step:

    (1) performing red color development that involves reaction of iridoid aglycones having a carboxyl group at position 4 of an iridoid skeleton with amino group-containing compounds,
    said red color development being performed in a solution in which ascorbic acid, similar compounds thereof, or two or more compounds selected from the group consisting of said ascorbic acid and said similar compounds are contained at a molar ratio of 5 or more with respect to all iridoid compounds in the solution,
    whereby red colorant compounds having acid resistance are generated.

2. The method according to claim 1, wherein the red color development in step (1) is performed in a solution in which ascorbic acid is contained at a molar ratio of more than 6 with respect to all iridoid compounds in the solution.

3. The method according to claim 1 or 2, wherein said red colorant composition derived from iridoid compounds is a gardenia red colorant composition.

4. The method according to any one of claims 1 to 3, wherein, in step (1), production of iridoid aglycones is carried out by hydrolysis of β-glycosidic bonds in iridoid glycosides prior to and/or concurrently with the red color development.

5. A method for producing a red colorant composition derived from iridoid compounds, comprising the following step:
    (1') performing red color development that involves reaction of aglycones of geniposide ester hydrolysate and/or aglycones of geniposidic acid having a carboxyl group at position 4 of an iridoid skeleton with amino group-containing compounds,
    said red color development being performed under an acidic condition in a solution in which ascorbic acid is contained at a molar ratio of more than 6 with respect to all iridoid compounds in the solution,
    whereby red colorant compounds having acid resistance are generated,
    wherein, in step (1'), production of iridoid aglycones is carried out by hydrolysis of β-glycosidic bonds in iridoid glycosides prior to and/or concurrently with the red color development, and
    said red colorant composition derived from iridoid compounds is a gardenia red colorant composition.

6. A method for imparting acid resistance to a produced red colorant composition derived from iridoid compounds, wherein step (1) recited in any one of claims 1 to 4 or step (1') recited in claim 5 is performed in a process for producing the red colorant composition derived from iridoid compounds.

7. A red colorant composition derived from iridoid compounds, obtained by the method according to any one of claims 1 to 5.

8. A red colorant composition derived from iridoid compounds having a feature as follows:
an $A_{\lambda max} / A_{600nm}$ value of an aqueous solution is 4.9 or more, when the aqueous solution contains the red colorant composition derived from iridoid compounds at a color value ($E^{10\%}_{1\ cm}$) of 0.05 and is adjusted to have a pH being 5.0 using McIlvaine buffer.

9. The red colorant composition derived from iridoid compounds according to claim 8, having a feature related to acid resistance as follows:
a color residual rate of a filtrate of an aqueous solution is 35% or more, when the aqueous solution contains the red colorant composition derived from iridoid compounds at a content of 0.05 mass% when calculated in terms of color value ($E^{10\%}_{1\ cm}$) 50.5, is adjusted to have a pH being 2.2 using McIlvaine buffer, and is filtered through a 0.45-μm filter to obtain said filtrate.

10. A pigment preparation containing the red colorant composition derived from iridoid compounds according to any one of claims 7 to 9.

11. A method for producing a pigment preparation, a food, a beverage, a cosmetic product, a medicine, a quasi-drug, a personal hygiene product, or feed, the method comprising a step in which the red colorant composition derived from iridoid compounds obtained by the method according to any one of claims 1 to 5 is contained.

12. A method for producing a food, a beverage, a cosmetic product, a medicine, a quasi-drug, a personal hygiene product, or feed, the method comprising a coloring step in which the red colorant composition derived from iridoid compounds according to any one of claims 7 to 9 or the pigment preparation according to claim 10 is used.

13. The method for producing the food, the beverage, the cosmetic product, the medicine, the quasi-drug, the personal hygiene product, or the feed according to claim 12, at least a part or the entire part of said method comprising a step/steps performed at a pH of less than 3.

14. A food, a beverage, a cosmetic product, a medicine, a quasi-drug, a personal hygiene product, or feed, containing the red colorant composition derived from iridoid compounds according to any one of claims 7 to 9 or the pigment preparation according to claim 10.

15. The food, the beverage, the cosmetic product, the medicine, the quasi-drug, the personal hygiene product, or the feed according to claim 14, wherein at least a part or the entire part of the food, the beverage, the cosmetic product, the medicine, the quasi-drug, the personal hygiene product, or the feed has a pH of less than 3.

*FIG. 1*

Iridoid glycosides

↓ (Gardenia fruit extract, geniposide, etc.)
(Having an alkyl ester group at position 4 of the iridoid skeleton)

Ester hydrolysis

↓ (Heated in an alkaline solution,
generation of geniposidic acid and the like)
(Having a carboxyl group at position 4 of the iridoid skeleton)

pH adjustment

↓ (Acidification)

Hydrolysis of β-glycosidic bonds

↓ (Generation of iridoid aglycones)

Red color development

↓ (Reaction with amino group-containing compounds)
(Oxidative polymerization reaction between iridoid compounds)
(Heat treatment enhances these reactions)

Purification, etc.

FIG. 2

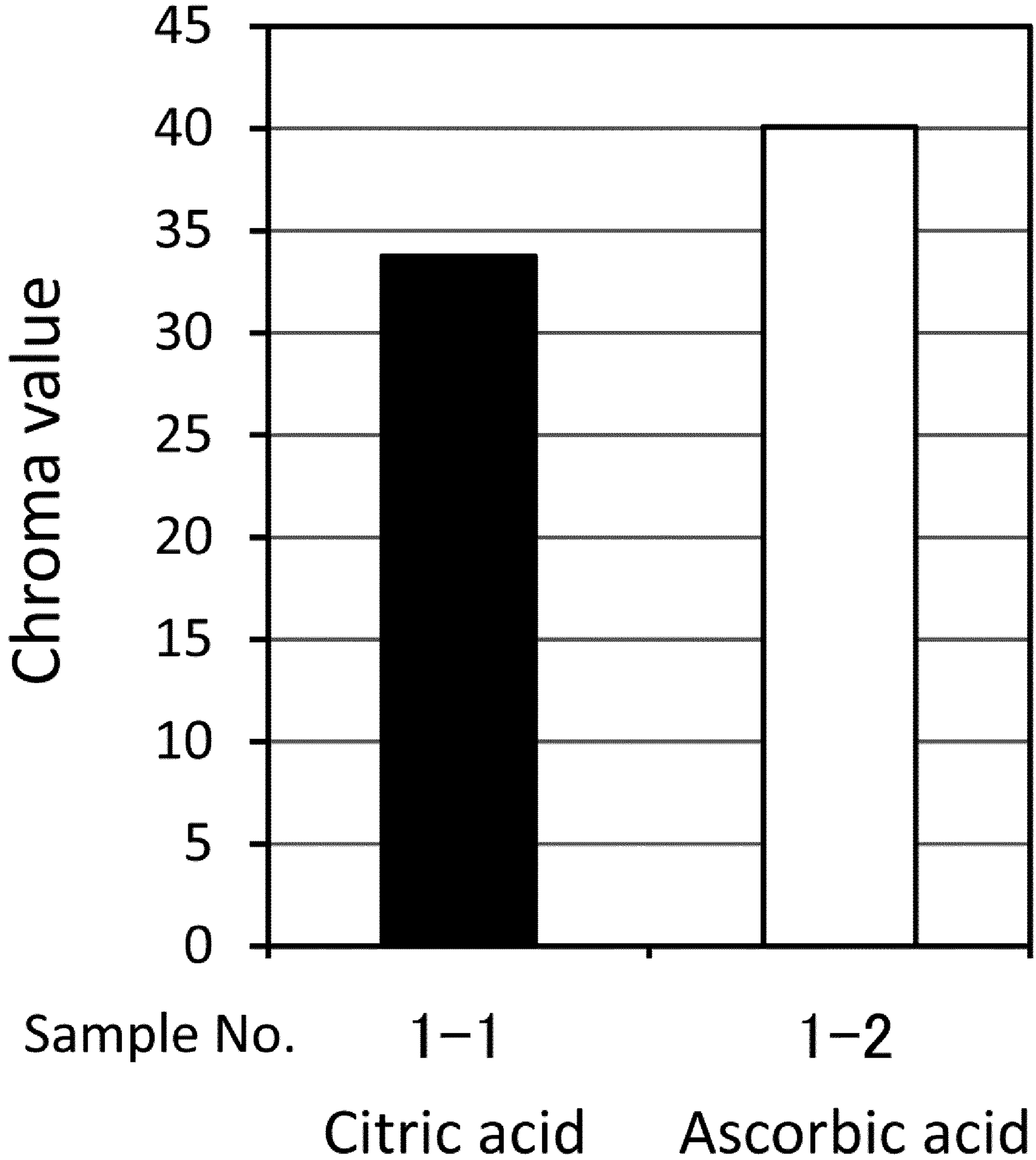
45
40
35
30
25
20
15
10
5
0
Chroma value
Sample No.
1−1
Citric acid
1−2
Ascorbic acid

FIG. 3

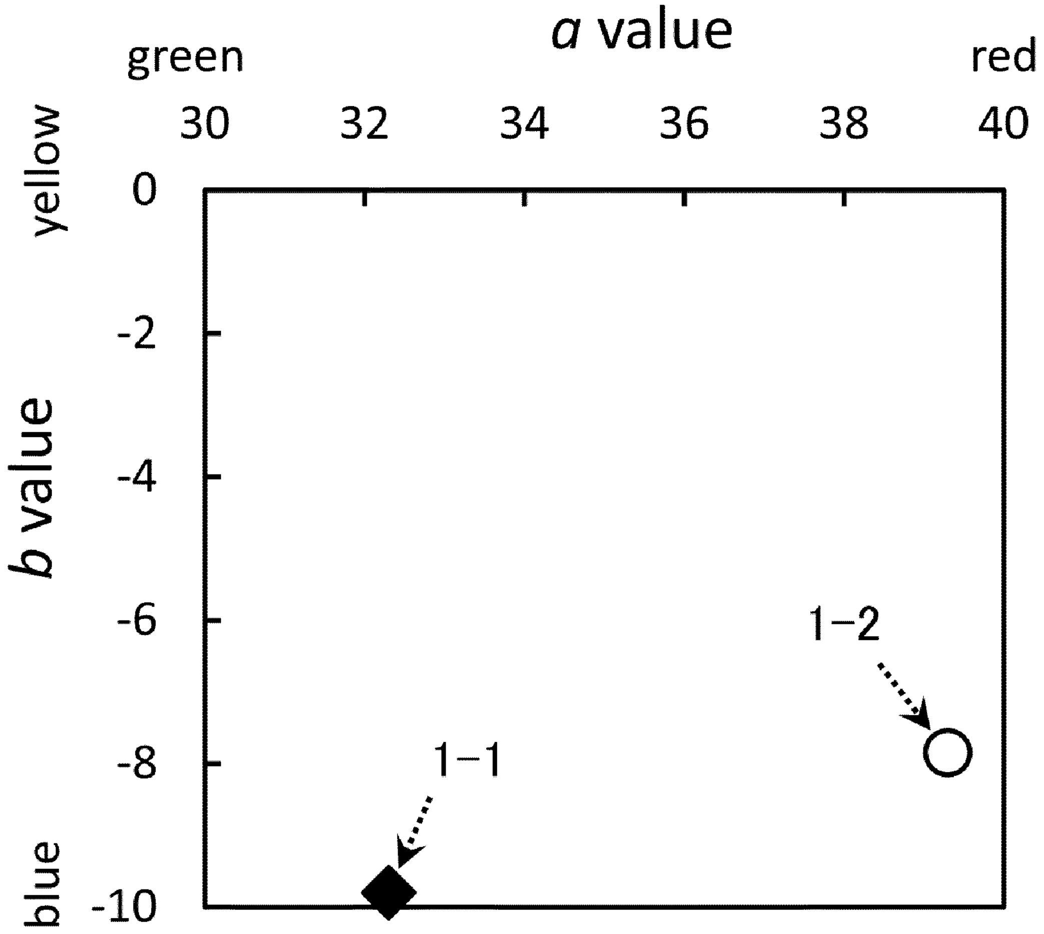


Sample 1-1: Produced under the presence of citric acid
Sample 1-2: Produced under the presence of ascorbic acid

FIG. 4

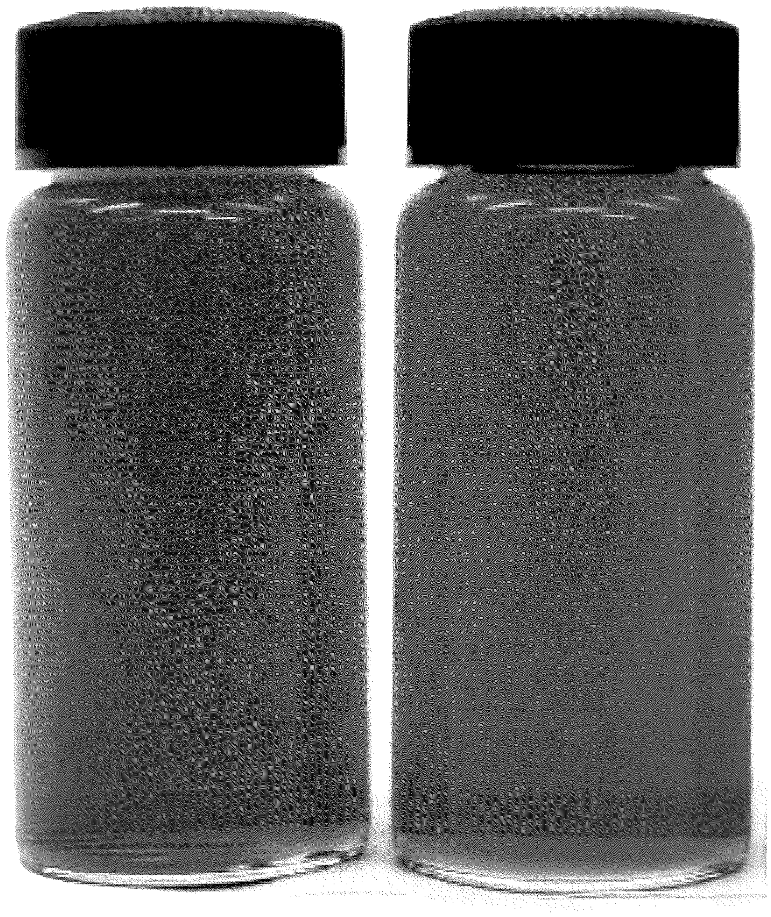

Sample No. 1−1 1−2

Citric acid Ascorbic acid

FIG. 5

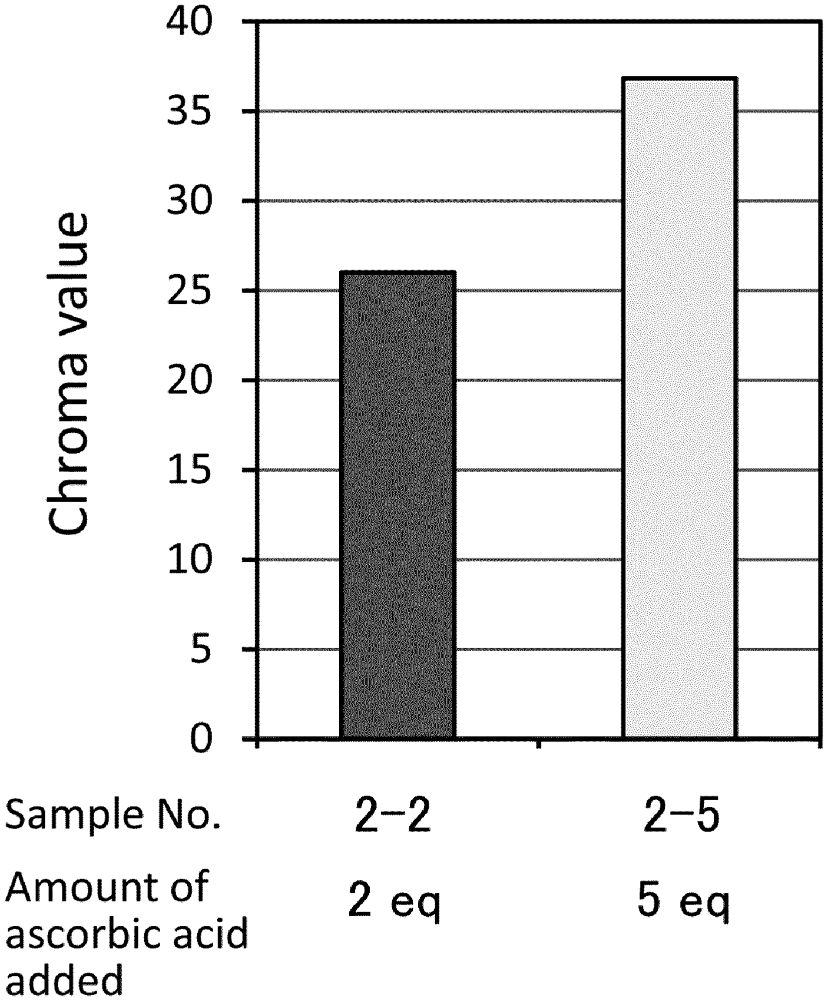


(eq: molar ratio with respect to GP)

FIG. 6

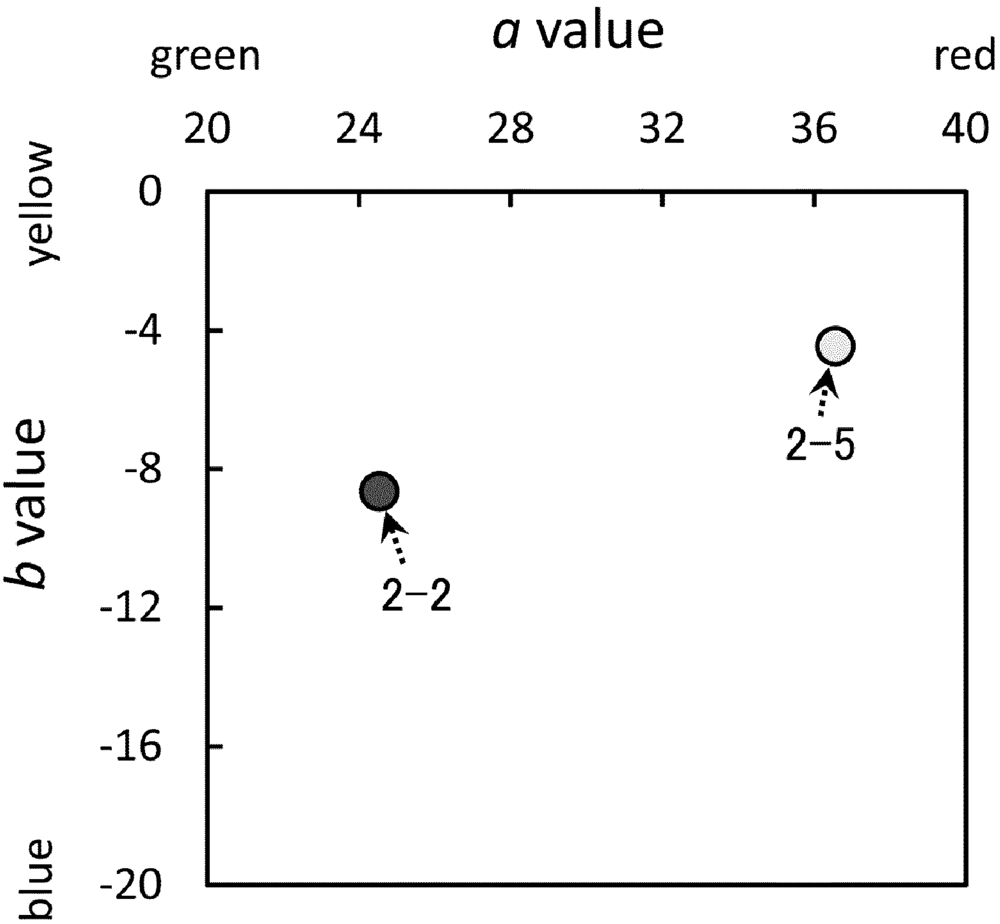


Sample 2-2: 2 eq of ascorbic acid added
Sample 2-5: 5 eq of ascorbic acid added

(eq: molar ratio with respect to GP)

*FIG. 7*

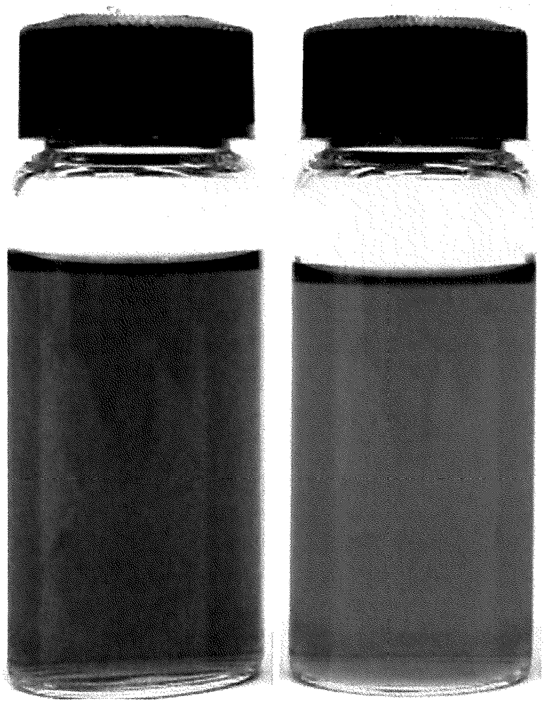

| Sample No. | 2-2 | 2-5 |
|---|---|---|
| Amount of ascorbic acid added | 2 eq | 5 eq |

(eq: molar ratio with respect to GP)

FIG. 8

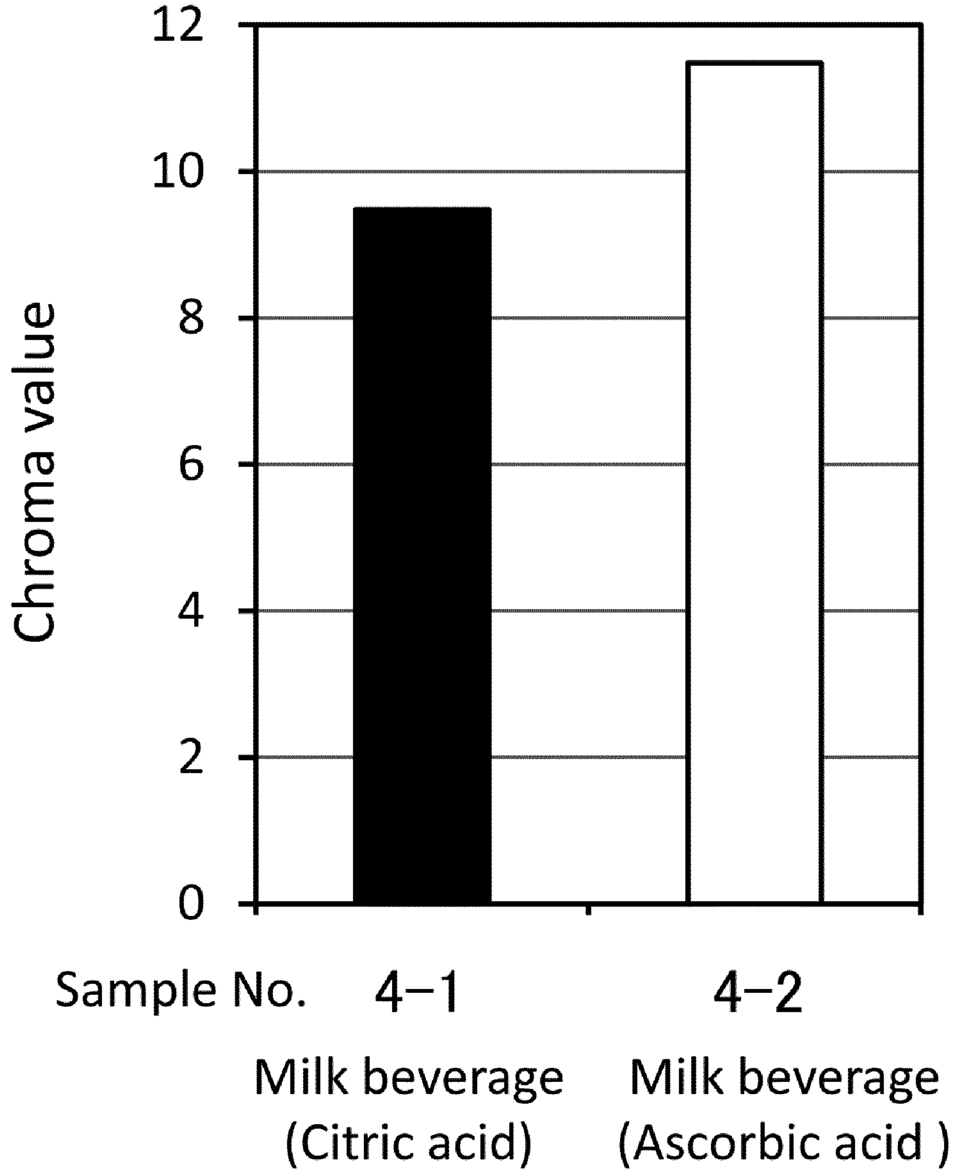
12
10
8
6
4
2
0
Chroma value
Sample No.
4−1
4−2
Milk beverage
(Citric acid)
Milk beverage
(Ascorbic acid )

FIG. 9

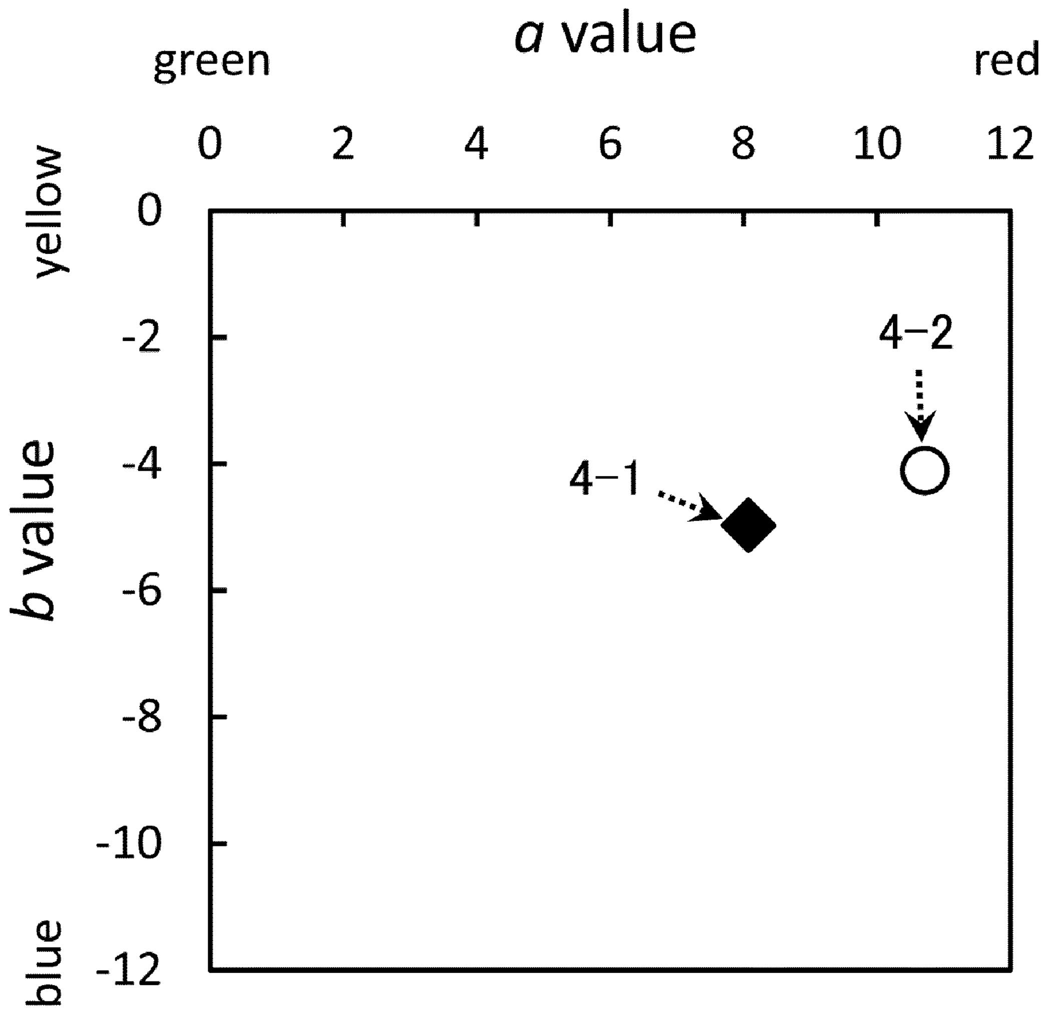


Sample 4-1: Milk beverage (citric acid)
Sample 4-2: Milk beverage (ascorbic acid)

FIG. 10

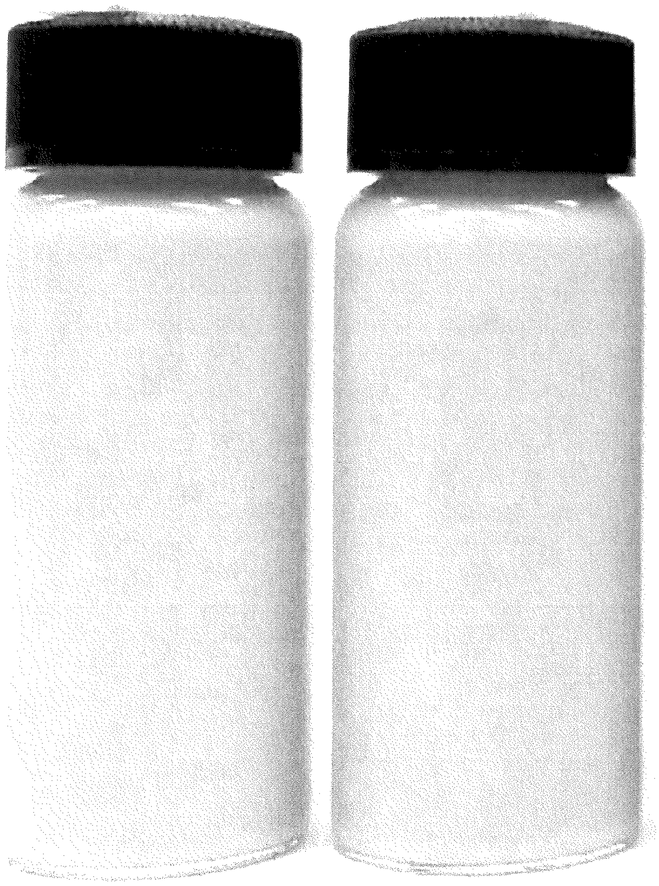

Sample No. 4−1 4−2

Milk beverage (Citric acid) Milk beverage (Ascorbic acid)

FIG. 11

Control

Processed fish meat product
(Uncolored)

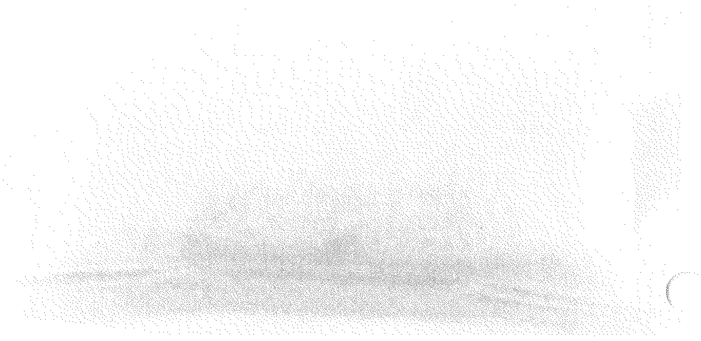

Sample 5-1

Processed fish meat product
(Citric acid)

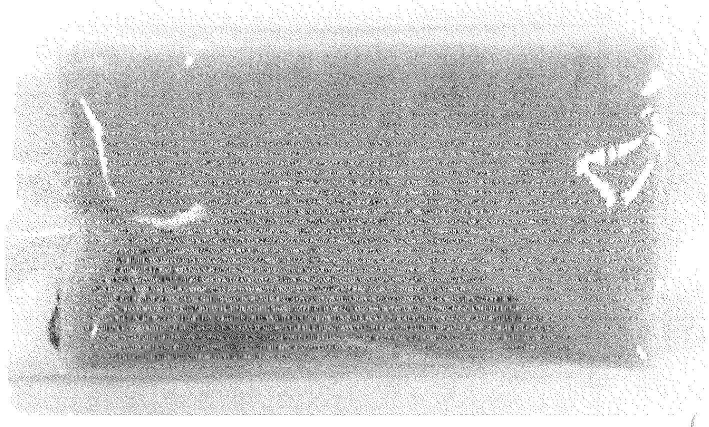

Sample 5-2

Processed fish meat product
(Ascorbic acid)

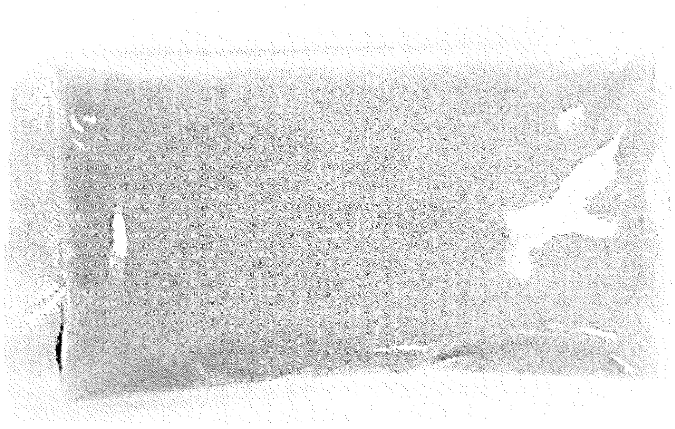

*FIG. 12*

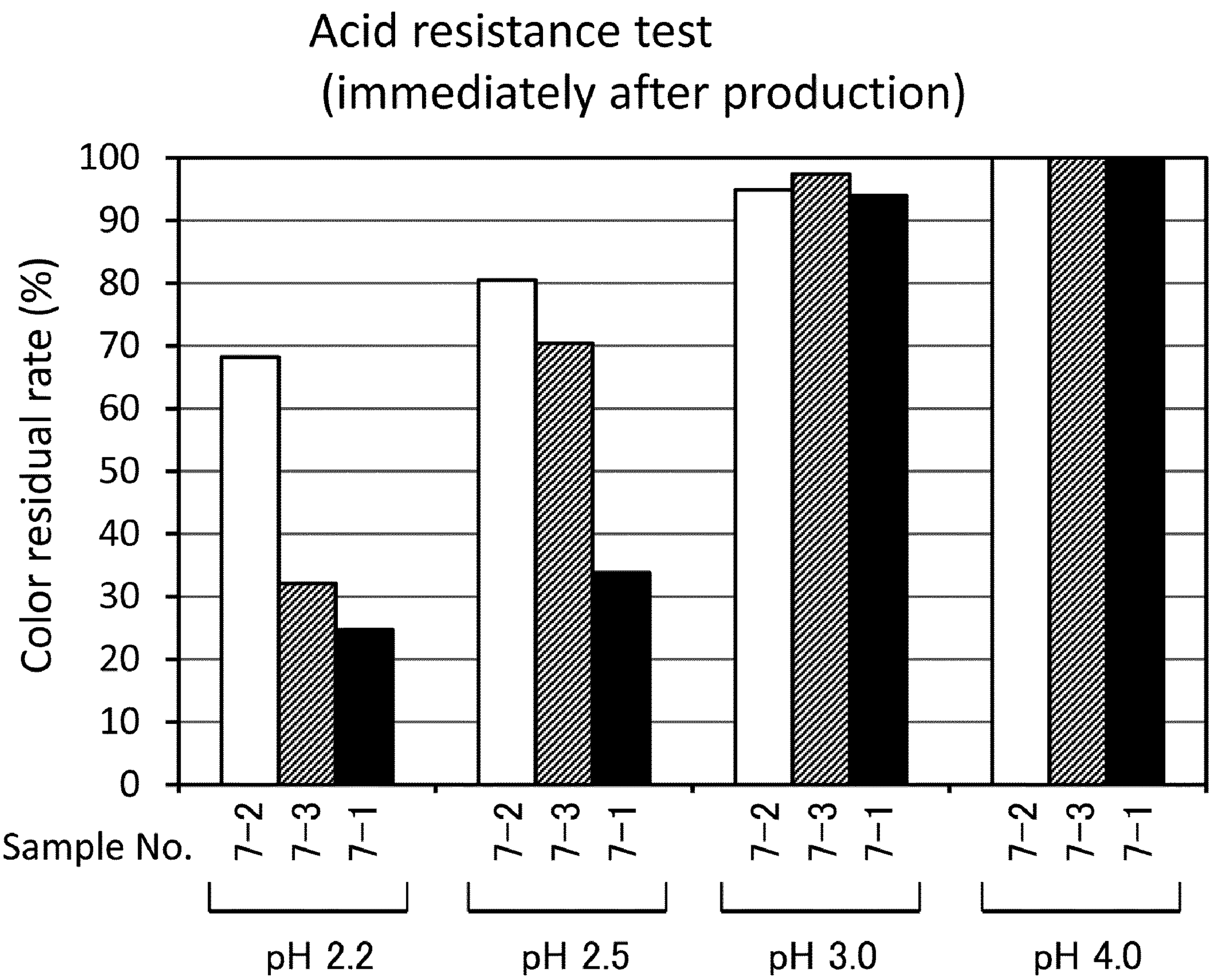


Sample 7-2: Produced under the presence of ascorbic acid
Sample 7-3: Produced under the presence of taurine
Sample 7-1: Produced under the presence of citric acid

FIG. 13

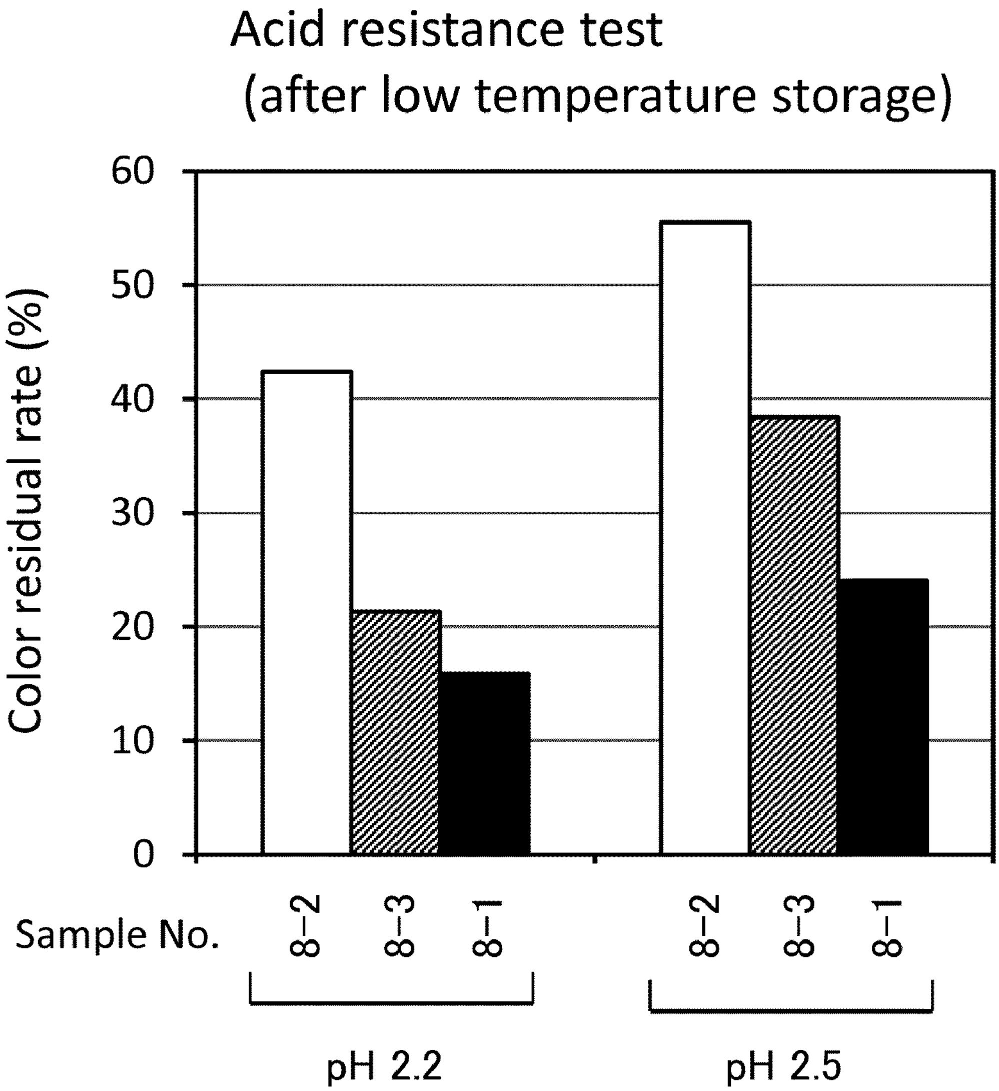


Sample 8-2: Produced under the presence of ascorbic acid
Sample 8-3: Produced under the presence of taurine
Sample 8-1: Produced under the presence of citric acid

*FIG. 14*

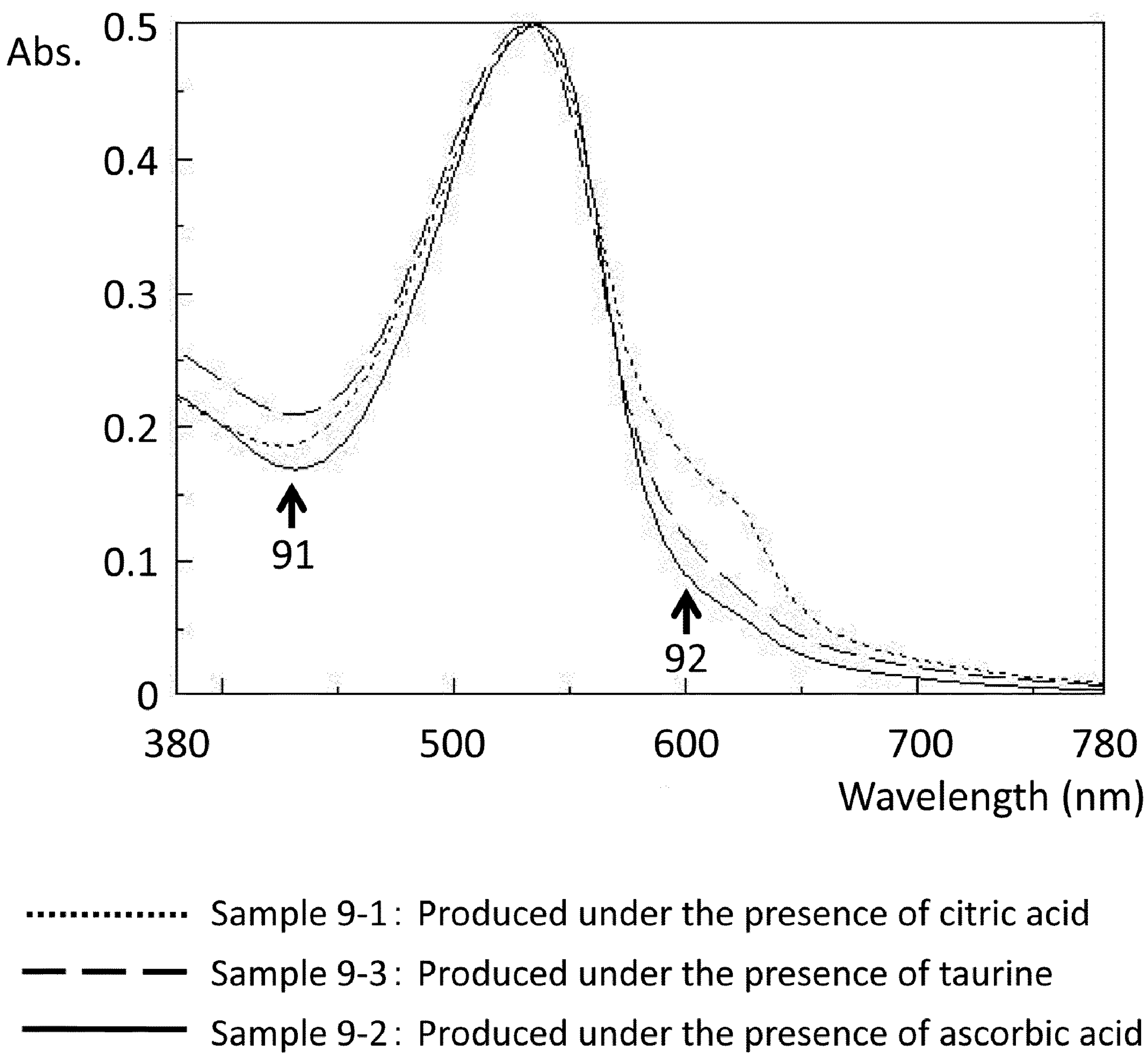
Abs.
0.5
0.4
0.3
0.2
0.1
0
91
92
380
500
600
700
780
Wavelength (nm)
Sample 9-1 : Produced under the presence of citric acid
Sample 9-3 : Produced under the presence of taurine
Sample 9-2 : Produced under the presence of ascorbic acid

FIG. 15

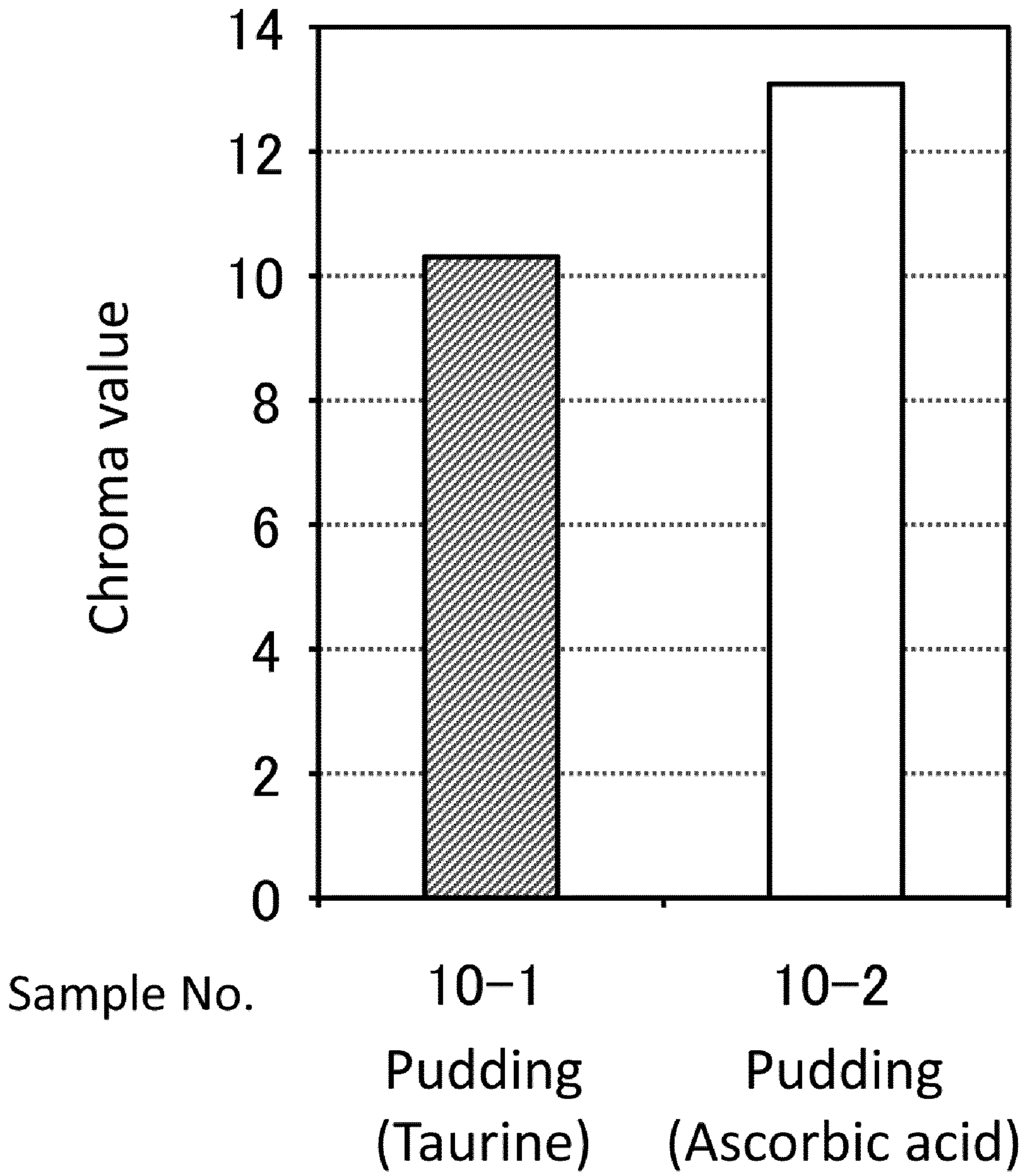
Chroma value
14
12
10
8
6
4
2
0
Sample No.
10−1
Pudding
(Taurine)
10−2
Pudding
(Ascorbic acid)

FIG. 16

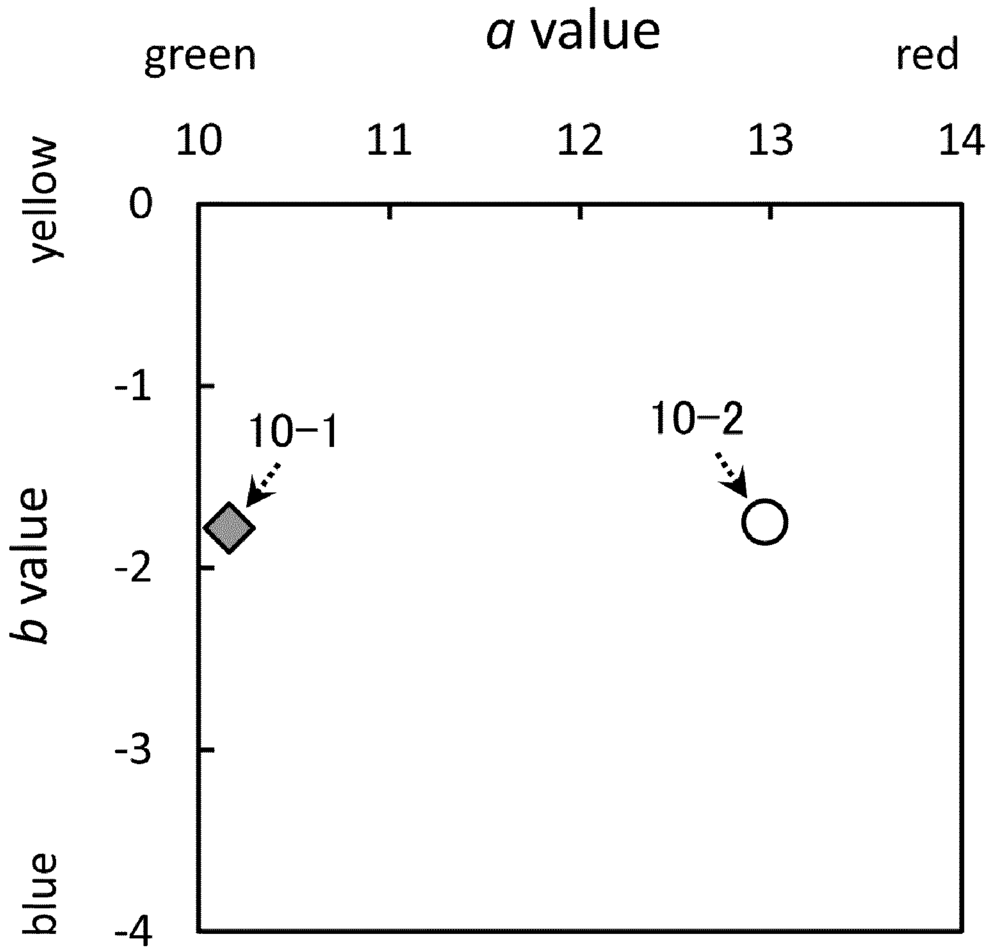


Sample 10-1: Pudding (taurine)
Sample 10-2: Pudding (ascorbic acid)

FIG. 17

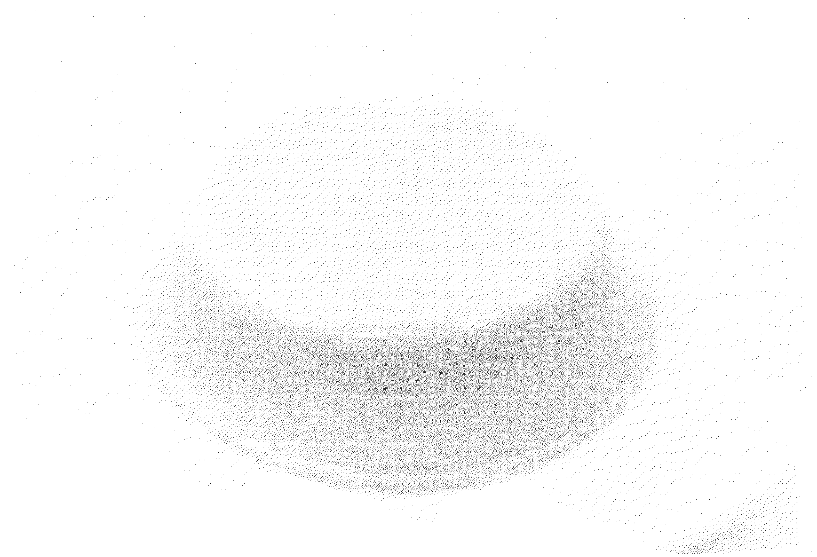

Sample 10-1
Pudding
(Taurine)

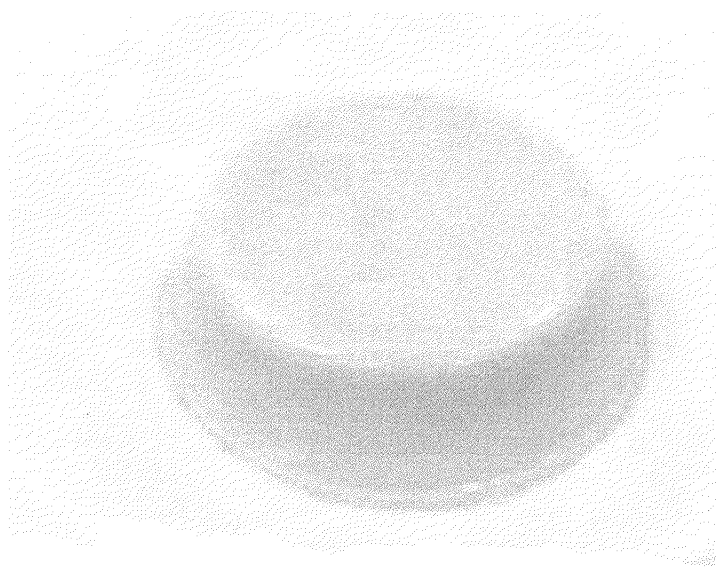

Sample 10-2
Pudding
(Ascorbic acid)

FIG. 18

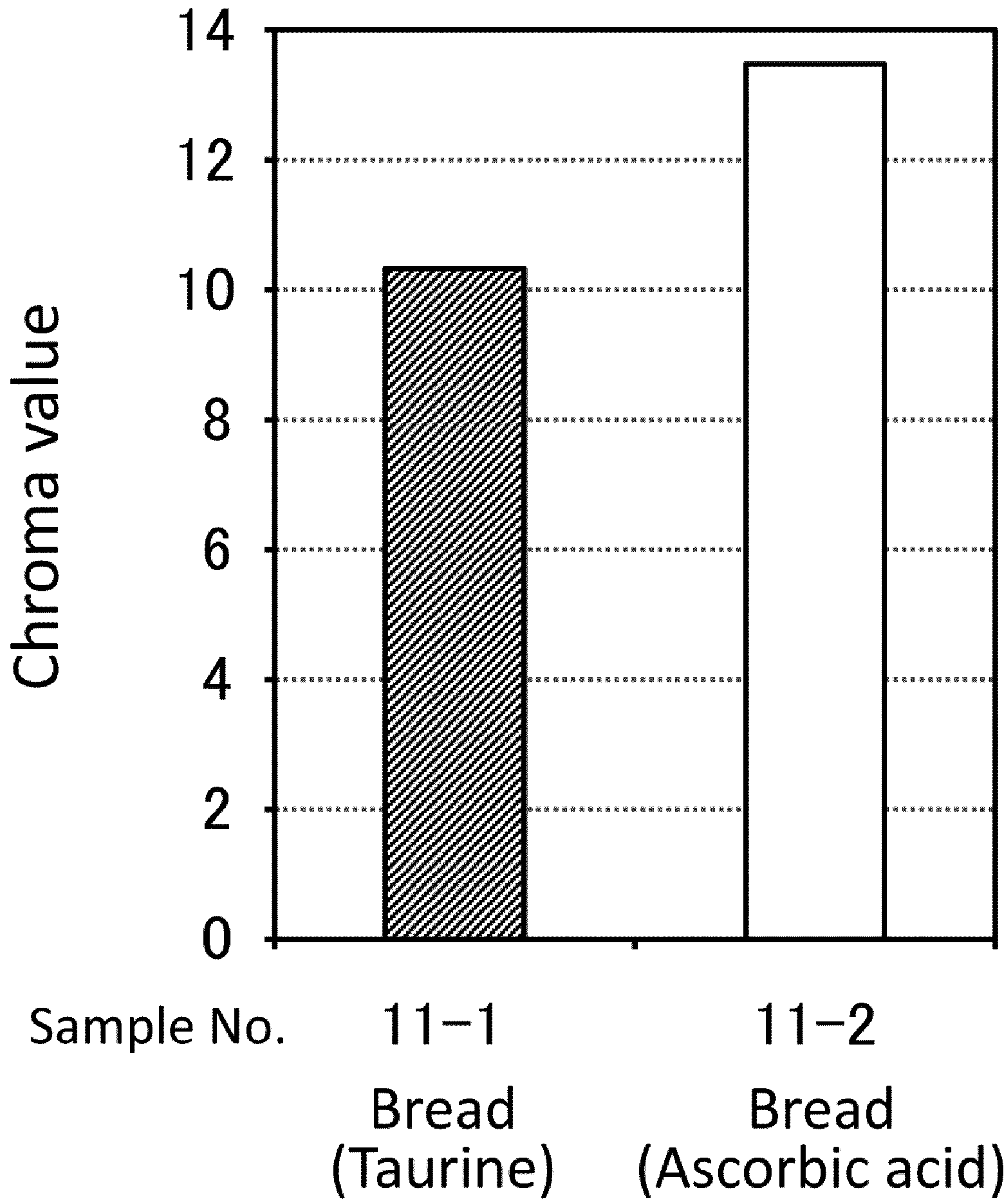
14
12
10
8
6
4
2
0
Chroma value
Sample No.
11−1
Bread
(Taurine)
11−2
Bread
(Ascorbic acid)

FIG. 19

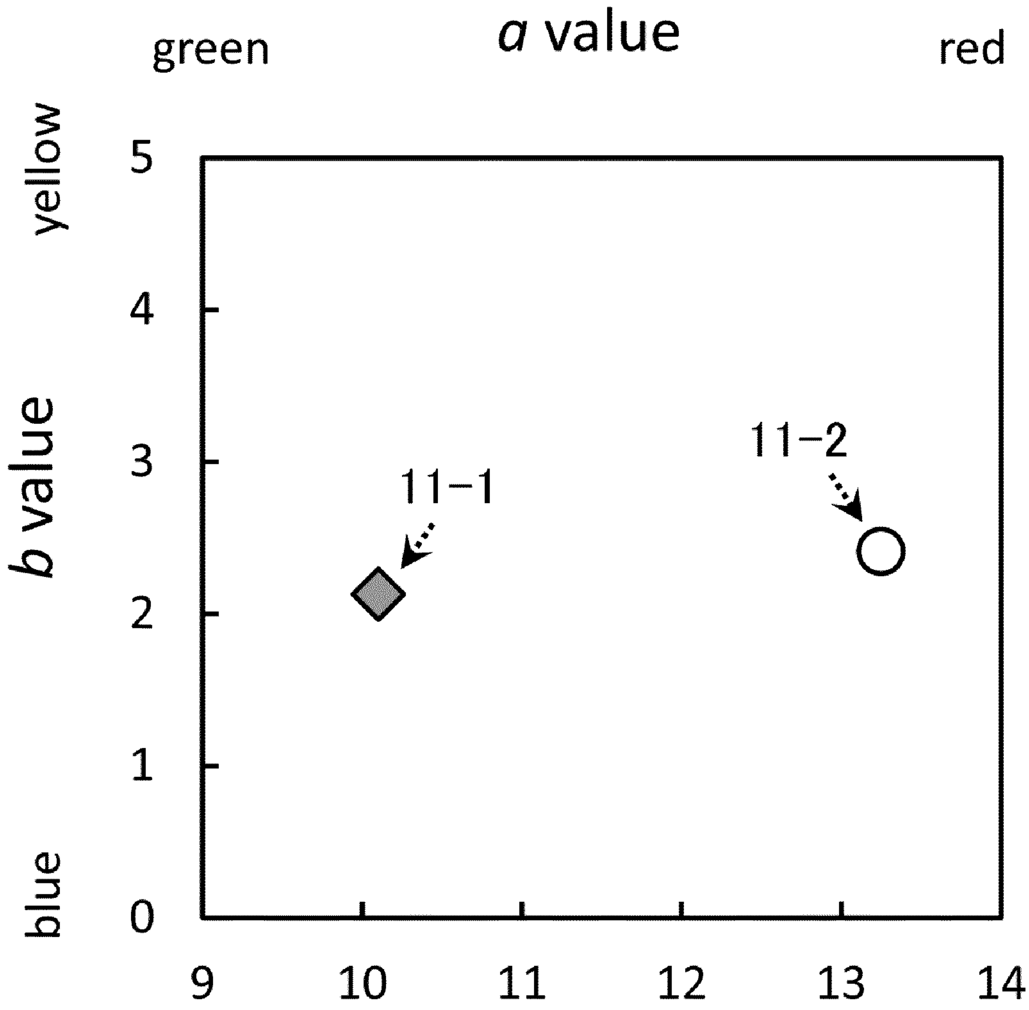


Sample 11-1: Bread (taurine)
Sample 11-2: Bread (ascorbic acid)

*FIG. 20*

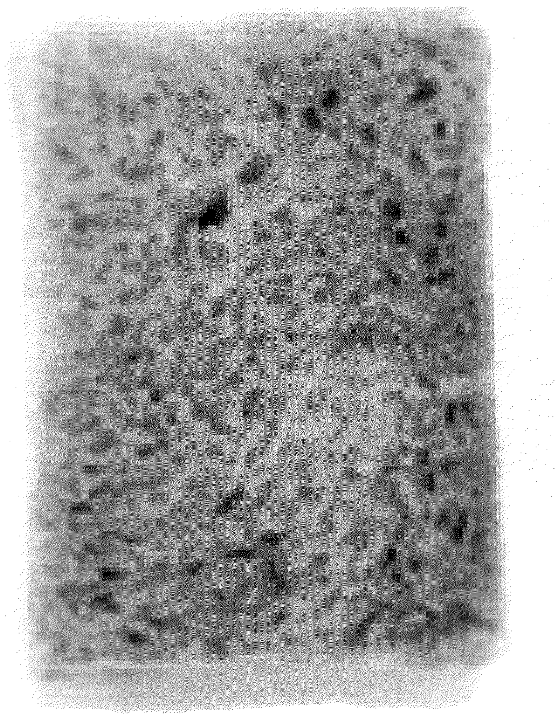

Sample 11-1
Bread
(Taurine)

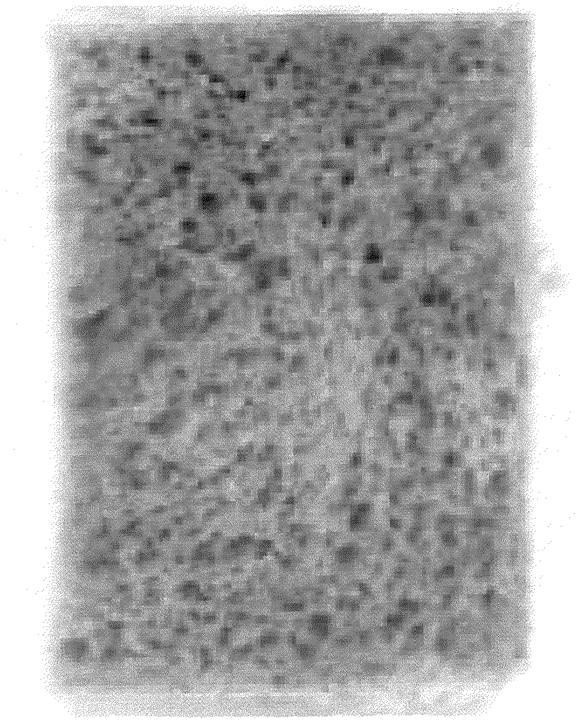

Sample 11-2
Bread
(Ascorbic acid)

FIG. 21

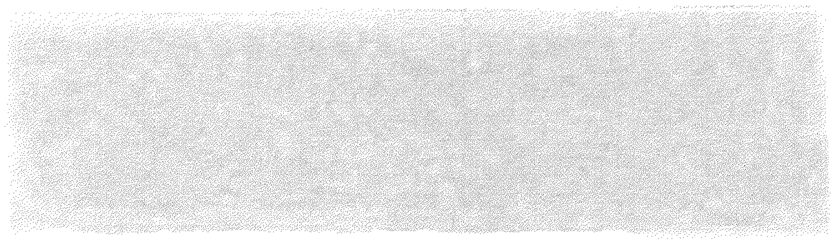

Sample 12-1
Chewing gum
(Taurine)

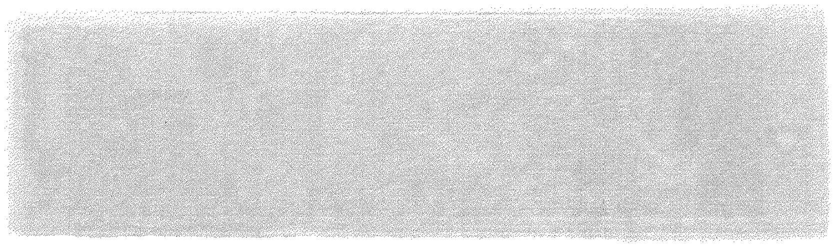

Sample 12-2
Chewing gum
(Ascorbic acid)

FIG. 22

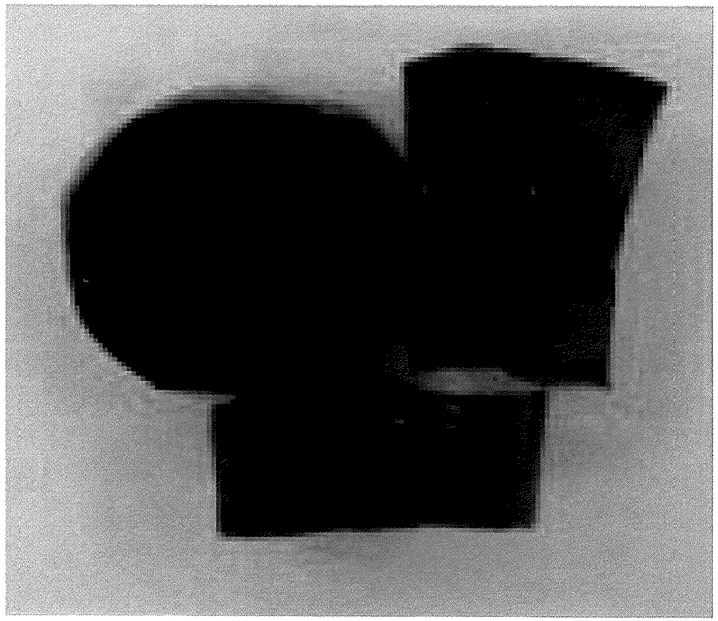

Sample 13-1

Pickles

(Taurine)

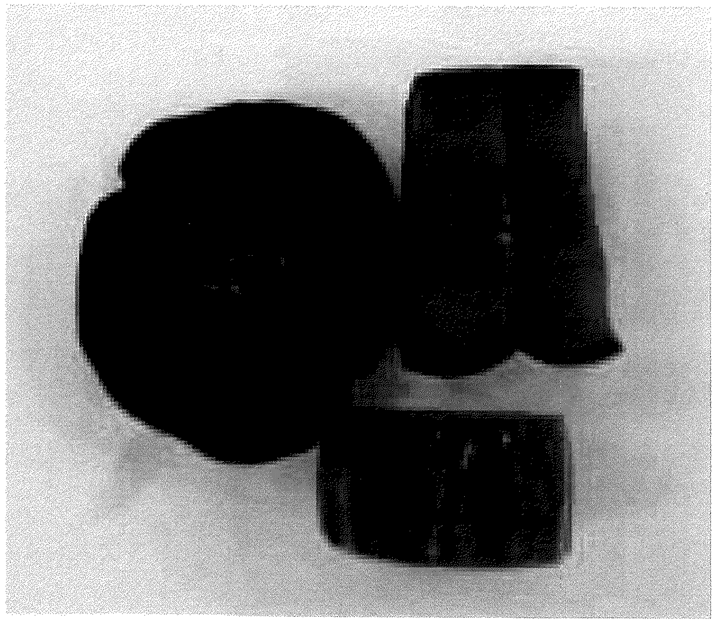

Sample 13-2

Pickles

(Ascorbic acid)

FIG. 23

Immediately after production

After light irradiation

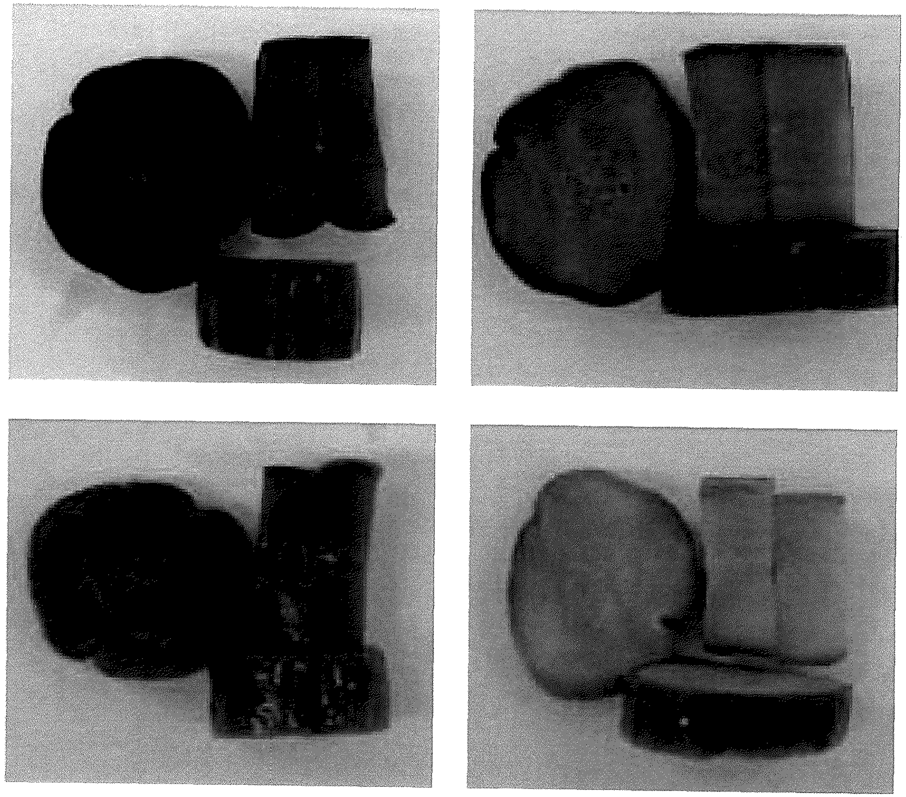

Sample 13-2 Pickles (Ascorbic acid)

Sample 13-3 Pickles (Anthocyanin pigments)

*FIG. 24*

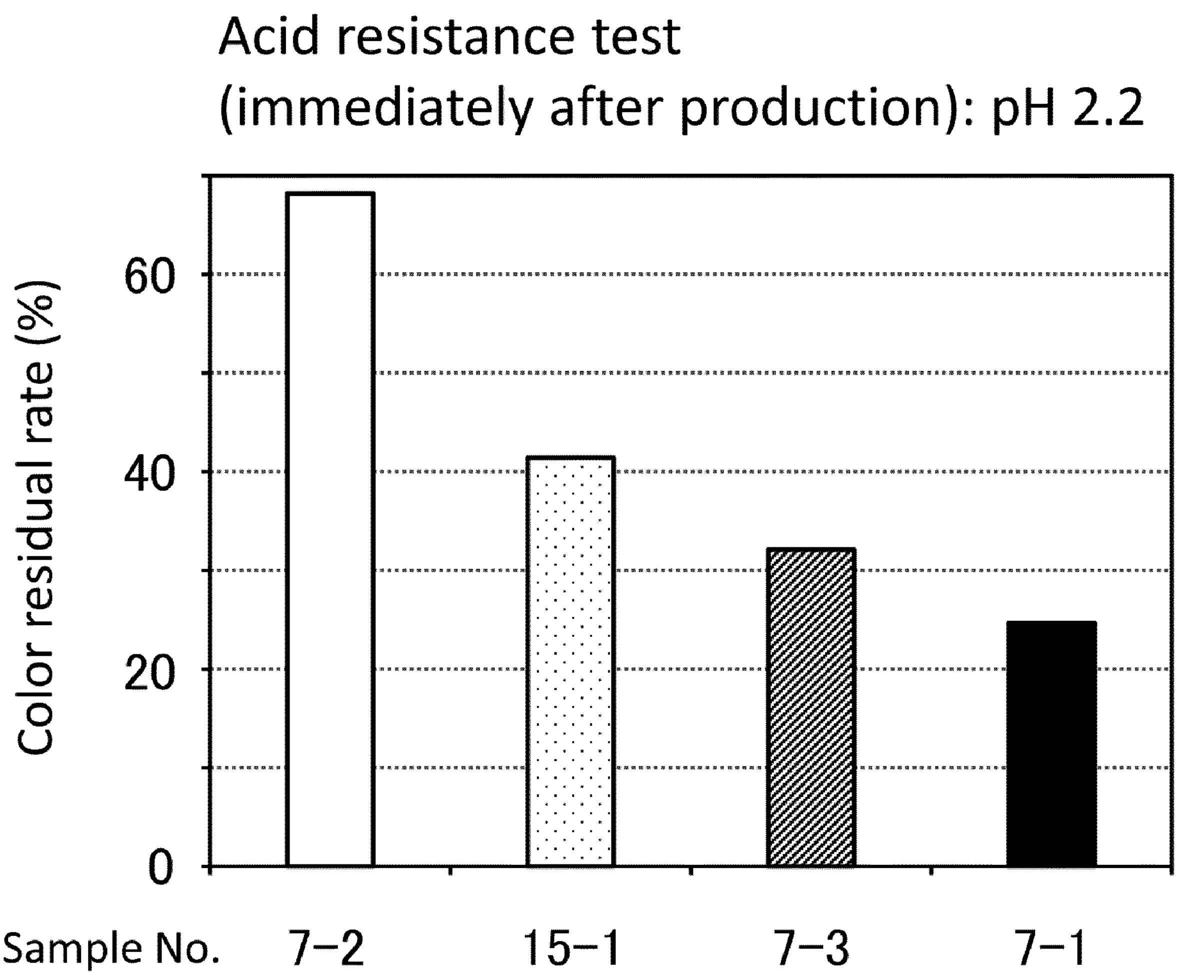


Sample 7-2: Ascorbic acid (8 eq)
Sample 15-1: Ascorbic acid (6.5 eq)
Sample 7-3: Taurine
Sample 7-1: Citric acid

*FIG. 25*

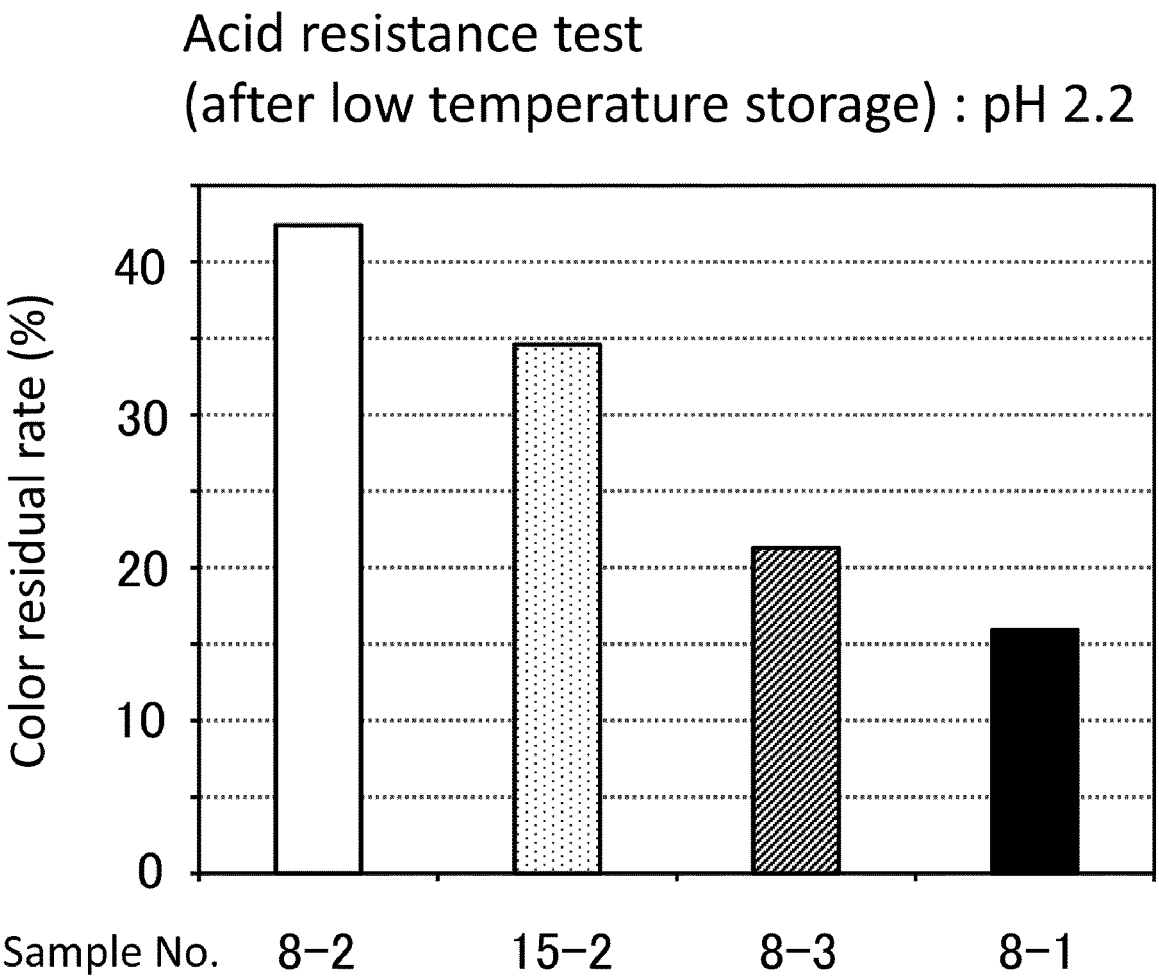


Sample 8-2: Ascorbic acid (8 eq)
Sample 15-2: Ascorbic acid (6.5 eq)
Sample 8-3: Taurine
Sample 8-1: Citric acid

## INTERNATIONAL SEARCH REPORT

International application No.
PCT/JP2017/022274

A. CLASSIFICATION OF SUBJECT MATTER
*C09B61/00*(2006.01)i, *A23K10/30*(2016.01)i, *A23K20/121*(2016.01)i, *A23K20/179*(2016.01)i, *A23L5/41*(2016.01)i, *A61K8/49*(2006.01)i, *A61K8/97*(2017.01)i, *A61Q1/02*(2006.01)i

According to International Patent Classification (IPC) or to both national classification and IPC

B. FIELDS SEARCHED

Minimum documentation searched (classification system followed by classification symbols)
C09B61/00, A23K10/30, A23K20/121, A23K20/179, A23L5/41, A61K8/49, A61K8/97, A61Q1/02

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2017 |
| Kokai Jitsuyo Shinan Koho | 1971-2017 | Toroku Jitsuyo Shinan Koho | 1994-2017 |

Electronic data base consulted during the international search (name of data base and, where practicable, search terms used)
CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| X | JP 03-277663 A (Taito Co., Ltd.), 09 December 1991 (09.12.1991), claims; page 2, upper left column, line 1 to page 3, upper left column, line 9; examples (Family: none) | 1-15 |
| A | WO 2011/132334 A1 (Mitsui Sugar Co., Ltd.), 27 October 2011 (27.10.2011), claim 1 & JP 2012-36334 A & JP 2011-225718 A & JP 4526600 B1 & CN 102300477 A & TW 201136534 A & KR 10-1054624 B1 | 1-15 |
| A | JP 05-059296 A (Taito Co., Ltd.), 09 March 1993 (09.03.1993), claim 1 (Family: none) | 1-15 |

☒ Further documents are listed in the continuation of Box C.

☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 05 September 2017 (05.09.17) | 19 September 2017 (19.09.17) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (January 2015)

INTERNATIONAL SEARCH REPORT

International application No.
PCT/JP2017/022274

C (Continuation). DOCUMENTS CONSIDERED TO BE RELEVANT

| Category* | Citation of document, with indication, where appropriate, of the relevant passages | Relevant to claim No. |
|---|---|---|
| A | JP 54-086668 A (Taito Co., Ltd.), 10 July 1979 (10.07.1979), claims & US 4247698 A & WO 1979/000394 A1 & DE 2857280 C & DE 2857280 T | 1-15 |
| A | JP 2011-217728 A (Riken Vitamin Co., Ltd.), 04 November 2011 (04.11.2011), claim 2 (Family: none) | 1-15 |
| A | JP 2012-116925 A (Mitsui Sugar Co., Ltd.), 21 June 2012 (21.06.2012), claim 1 (Family: none) | 1-15 |

Form PCT/ISA/210 (continuation of second sheet) (January 2015)

**INTERNATIONAL SEARCH REPORT**

International application No.
PCT/JP2017/022274

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

(i) Number of inventions involved in claims: two

(ii) Number of claim classified into each invention

Invention 1: claims 1-7 and 10-15

Invention 2: claims 8-10 and 12-15

(iii) Reason for that unity of invention is not satisfied:

There is found no common technical matter between the invention described in claim 1 which relates to a method for producing an iridoid compound-derived red dye composition and the invention described in claim 8 which relates to an iridoid compound-derived red dye composition.

1. ☐ As all required additional search fees were timely paid by the applicant, this international search report covers all searchable claims.

2. ☒ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP S555778 B
- JP 5753373 B
- JP 4605824 B
- JP 2802451 B
- JP 2011217728 A
- JP 2016121254 A
- JP 2017088924 A